# EUROPEAN PATENT SPECIFICATION

(11) **EP 2 370 091 B1**
(45) Date of publication and mention of the grant of the patent: **12.04.2017**
(21) Application number: 09831269.7
(22) Date of filing: 07.12.2009
(51) Int. Cl.: A61K 38/16, A61K 38/17, A61K 31/7105, A61P 35/00, C12N 15/113

(54) **COMPOSITIONS AND METHODS RELATING TO MIR-31**
ZUSAMMENSETZUNGEN UND VERFAHREN IM ZUSAMMENHANG MIT MIR-31
COMPOSITIONS ET PROCÉDÉS CONCERNANT LE MIR-31

(30) Priority: 05.12.2008 US 120322 P
(43) Date of publication of application: 05.10.2011
(73) Proprietor: Whitehead Institute for Biomedical Research, Cambridge, MA 02142 (US)
(72) Inventor: WEINBERG, Robert, A., Brookline, MA 02446 (US); VALASTYAN, Scott, A., Cambridge, MA 02142 (US)
(74) Representative: V.O.
(86) International application number: PCT/US2009/067015
(87) International publication number: WO 2010/065961

(56) References cited:
- WO-A1-2005/113770
- WO-A2-00/22130
- WO-A2-99/34004
- WO-A2-2006/061430
- WO-A2-2006/091776
- WO-A2-2007/081740
- WO-A2-2008/036776
- US-A1- 2006 247 193
- US-A1- 2008 014 579
- US-A1- 2008 050 744
- SAHAI E. AND MARSHALL C.J.: "RHO-GTPases and Cancer", NATURE REVIEWS. CANCER, NATUR PUBLISHING GROUP, LONDON, GB, vol. 2, no. 2, 1 February 2002 (2002-02-01), pages 133-142, XP009040996, ISSN: 1474-175X, DOI: 10.1038/NRC725
- SCOTT VALASTYAN ET AL.: "A pleiotropically acting microRNA, miR-31, inhibits breast cancer metastasis", CELL, vol. 137, no. 6, 12 June 2009 (2009-06-12) , pages 1032-1046, XP002658971, CELL PRESS, US ISSN: 0092-8674, DOI: 10.1016/J.CELL.2009.03.047 [retrieved on 2009-06-11] -& SCOTT VALASTYAN ET AL.: "Supplemental Data: A Pleiotropically Acting MicroRNA,miR-31, Inhibits BreastCancer Metastasis", Cell, 12 June 2009 (2009-06-12), XP002689568, Retrieved from the Internet: URL:http://www.sciencedirect.com/science/M iamiMultiMediaURL/1-s2.0-S0092867409003900 /1-s2.0-S0092867409003900-mmc1.pdf/272196/ FULL/S0092867409003900/029af534cbda5711a96 bc901b7e713c8/mmc1.pdf [retrieved on 2012-12-19]
- Y.-H. TSAI ET AL.: "The M Type K15 Protein of Kaposi's Sarcoma-Associated Herpesvirus regulates microRNA expression via its SH2-Binding Motif to induce cell migration and invasion", JOURNAL OF VIROLOGY, vol. 83, no. 2, 29 October 2008 (2008-10-29), pages 622-632, XP055048397, ISSN: 0022-538X, DOI: 10.1128/JVI.00869-08 -& Y.-H. TSAI ET AL.: "Supplementary data - KSHV K15M protein", Journal of Virology, 29 October 2008 (2008-10-29), XP002689569, Retrieved from the Internet: URL:http://infobio.ym.edu.tw/_media/downlo ads/k15msuppl.pdf [retrieved on 2012-12-19]
- L.-X. YAN ET AL.: "MicroRNA miR-21 overexpression in human breast cancer is associated with advanced clinical stage, lymph node metastasis and patient poor prognosis", RNA, vol. 14, no. 11, 23 September 2008 (2008-09-23), pages 2348-2360, XP055038607, ISSN: 1355-8382, DOI: 10.1261/rna.1034808 -& L.-X. YAN ET AL.: "Supplemental Research Data: MicroRNA miR-21 overexpression in human breast cancer is associated with advanced clinical stage, lymph node metastasis and patient poor prognosis", RNA, 23 September 2008 (2008-09-23), XP002689570, Retrieved from the Internet: URL:http://rnajournal.cshlp.org/content/14 /11/2348/suppl/DC1 [retrieved on 2012-12-19]
- SLABY O ET AL.: "Altered expression of miR-21, miR-31, miR-143 and miR-145 is related to clinicopathologic features of colorectal cancer", ONCOLOGY (BASEL), vol. 72, no. 5-6, 2007, pages 397-402, XP009111358, ISSN: 0030-2414
- WONG TS. ET AL: 'Mature miR-184 as potential oncogenic microRNA of squamous cell carcinoma of tongue' CLIN. CANCER RES. vol. 14, no. 9, May 2008, pages 2588 - 2592, XP002545704

## Description

### Related Applications

This application claims priority to, and the benefit of, U.S. Provisional Application No. 61/120,322, filed December 5, 2008.

### Background of the Invention

Metastases account for 90% of human cancer deaths (Gupta and Massagué, 2006). The invasion-metastasis cascade, which leads to these growths, is a complex, multistep process involving the escape of neoplastic cells from a primary tumor (local invasion), intravasation into the systemic circulation, survival during transit through the vasculature, extravasation into the parenchyma of distant tissues, the establishment of micrometastases, and ultimately the outgrowth of macroscopic secondary tumors (colonization) (Fidler, 2003). Understanding of the molecular circuitry that governs metastatic dissemination remains incomplete. There is a need for new approaches to identify agents that would reduce the incidence of metastasis. There is also a need for new methods to help assess the likelihood of metastatic dissemination and/or of metastatic recurrence following therapy.

### Summary of the Invention

The present invention relates at least in part to microRNA-31 (miR-31), targets of miR-31, the role of miR-31 in inhibiting tumor metastasis, and the role of miR-31 target genes in promoting tumor metastasis.

The invention provides a method of inhibiting tumor metastasis in a subject comprising inhibiting expression or activity of at least one target of miR-31 in the tumor. In some embodiments, the method comprises inhibiting expression of at least one miR-31 target gene. In some embodiments the method comprises inhibiting activity of at least one protein encoded by a miR-31 target gene. In some embodiments, the method comprises administering an RNAi agent to the subject. In some embodiments the method comprises administering a miRNA, miRNA precursor, agent that mimics a miRNA, or siRNA to the subject. In some embodiments, the method comprises administering a nucleic acid construct that encodes a precursor to a miRNA or siRNA to the subject. In some embodiments, the method comprises administering mature miR-31, an agent that mimics miR-31, or a nucleic acid construct encoding miR-31 or encoding a precursor of miR-31 to the subject.

The invention provides method of inhibiting metastasis of a tumor cell comprising inhibiting expression or activity of at least one target of miR-31 in the tumor cell. In some embodiments, the tumor cell is in a subject. In some embodiments, the method comprises inhibiting expression of at least one miR-31 target gene. In some embodiments the method comprises inhibiting activity of at least one protein encoded by a miR-31 target gene. In some embodiments, the method comprises administering an RNAi agent to the cell or subject. In some embodiments, the method comprises administering a miRNA, agent that mimics a miRNA, shRNA, or siRNA to the cell or subject. In some embodiments, the method comprises expressing a miRNA or shRNA in the tumor cell. In some embodiments, the method comprises expressing miR-31 in the tumor cell.

The invention further provides a composition for inhibiting tumor metastasis comprising an agent that inhibits expression or activity of at least one target of miR-31 in a cell. In some embodiments, the composition comprises an siRNA, miRNA, miRNA precursor, or an agent that mimics a miRNA (e.g, an agent that mimics miR-31). In some embodiments, the agent inhibits expression of a miR-31 target gene. In some embodiments, the agent inhibits activity of a protein encoded by a miR-31 target gene.

The invention further provides method for assessing the likelihood that a tumor in a subject will metastasize, the method comprising (a) determining the level of a miR-31 gene product in a biological sample obtained from the tumor; and (b) comparing the level with a control level, wherein if the level determined in (a) is less than the control level, the tumor is considered to have increased likelihood of metastasizing. In some embodiments, the biological sample comprises tumor tissue. In some embodiments, the tumor is a breast cancer. In some embodiments, the miR-31 gene product is a miR-31 RNA or a miR-31 precursor RNA (e.g., pre-miR-31). In some embodiments the miR-31 gene product is mature miR-31. In some embodiments, determining the level of the miR-31 gene product in the biological sample comprises performing a hybridization based assay, polymerase chain reaction assay, or sequencing.

The invention further provides a method of assessing the likelihood of development of a tumor metastasis in a subject, comprising the steps of: (a) determining the level of a miR-31 gene product in a biological sample obtained from a subject; and (b) comparing the level determined in (a) with an appropriate control, wherein if the level determined in (a) is less than the control level of the miR-31 gene product, then the subject has an increased likelihood of developing a metastasis. In some embodiments, the biological sample comprises tumor tissue. In some embodiments a method of assessing the likelihood of development of a tumor metastasis in a subject, comprises the steps of: (a) obtaining a biological sample from the subject; (b) determining the level of a miR-31 gene product in the biological sample; and (c) comparing the level determined in (b) with an appropriate control, wherein if the level determined in (b) is less than the level of the miR-31 gene product in a control sample, then the subject has an increased likelihood of developing a metastasis. In some embodiments, the tumor is a breast cancer. In some embodiments, the miR-31 gene product is a miR-31 RNA or a miR-31 precursor RNA (e.g., pre-miR-31). In some embodiments, the miR-31 gene product is a mature miR-31 RNA. In some embodiments, determining the level of the miR-31 gene product in the biological sample and/or in a control sample comprises performing a hybridization based assay, polymerase chain reaction assay, or sequencing.

The invention further provides a method for assessing the likelihood that a tumor in a subject will metastasize or has metastasized, the method comprising (a) determining the level of an expression product of a miR-31 target gene in a biological sample obtained from the tumor; and (b) comparing the level with a control level, wherein if the level determined in (a) is greater than the control level, the tumor is considered to have increased likelihood of metastasizing. In some embodiments, the biological sample comprises tumor tissue. In some embodiments a control sample or control level is obtained from non-tumor tissue, which is optionally non-tumor tissue obtained from the same subject (e.g., adjacent to the site of the tumor). In some embodiments, a control or reference level is obtained from historical data, e.g., measurements made on a plurality of samples of previously collected non-tumor tissue. In some embodiments, the tumor is a breast cancer. In some embodiments, the expression product is an mRNA or a polypeptide. In some embodiments, determining the level of the expression product comprises performing a hybridization based assay, polymerase chain reaction assay, sequencing, ELISA assay, Western blot, mass spectrometry, or immunohistochemistry. In some embodiments, the method comprises measuring the level of expression products of at least two miR-31 target genes in the biological sample, wherein if the level of expression of products of at least two miR-31 target genes is increased relative to control levels, the tumor is considered to have increased likelihood of metastazing or having metastasized.

The invention further provides a method for providing prognostic information relating to the likelihood that a tumor in a subject will metastasize, the method comprising (a) determining the level of a miR-31 gene product in a biological sample obtained from the tumor; and (b) comparing the level with a control level, wherein (i) if the level determined in (a) is less than the control level, the tumor is considered to have a poor prognosis; or (ii) if the level determined in (a) is greater than the control level, the tumor is considered to have a good prognosis. In some embodiments, the biological sample comprises tumor tissue. In some embodiments, the tumor is a breast cancer. In some embodiments, the miR-31 gene product is a miR-31 RNA or a miR-31 precursor RNA. In some embodiments the miR-31 gene product is a mature miR-31 RNA. In some embodiments, determining the level of the miR-31 gene product in the biological sample comprises performing a hybridization based assay, polymerase chain reaction assay, or sequencing.

The invention further provides a method for testing the ability of a compound to inhibit the survival and/or proliferation of a cell, comprising (a) contacting one or more test cells with a compound, wherein the one or more test cells has reduced or absent miR-31 expression or activity as compared to a control cell, and (b) detecting the level of inhibition of the survival and/or proliferation of the one or more test cells by the compound. In some embodiments, the cell is a cancer cell. In some embodiments, the cell is a breast cancer cell. In some embodiments, the method comprises: (a) contacting one or more test cells and one or more control cells with the compound, wherein the one or more test cells has reduced or absent miR-31expression or activity as compared with the one or more control cells; and (b) detecting the level of inhibition of the survival and/or proliferation of the one or more test cells and control cells by the compound; and (c) identifying the compound as a candidate anti-tumor agent (e.g., an agent useful for inhibiting tumor metastasis) if the compound has a greater inhibitory effect on the survival and/or proliferation of the test cells than the control cells. In some embodiments, the test cells and the control cells are genetically matched. In some embodiments, the test cells have a mutation or deletion in the miR-31 gene or express a miRNA sponge that suppresses miR-31 activity. In some embodiments, the method further comprises administering the compound to a subject. In some embodiments, the subject is a non-human animal and the method optionally further comprises introducing one or more cancer cells into the subject or inducing cancer in the subject. In some embodiments, the subject has cancer. In some embodiments, the method further comprises assessing the ability of the compound to inhibit metastasis in the subject.

Certain aspects of the invention are described herein mainly in regard to breast cancer cells and breast cancer. However, the invention is not so limited but rather encompasses embodiments in which products and processes described herein may be applied in the context of other cancers, e.g., any cancer arising from cells that express miR-31. One of skill in the art will recognize that details of the invention may be modified according to the particular cancer type or cancer cell type of interest.

The practice of the present invention will typically employ, unless otherwise indicated, conventional techniques of molecular biology, cell culture, recombinant nucleic acid (e.g., DNA) technology, immunology, nucleic acid and polypeptide synthesis, detection, manipulation, and quantification, and RNA interference that are within the skill of the art. See, e.g., Ausubel, F., et al., (eds.), Current Protocols in Molecular Biology, Current Protocols in Immunology, Current Protocols in Protein Science, and Current Protocols in Cell Biology, all John Wiley & Sons, N.Y., edition as of December 2008; Sambrook, Russell, and Sambrook, Molecular Cloning: A Laboratory Manual, 3rd ed., Cold Spring Harbor Laboratory Press, Cold Spring Harbor, 2001; Harlow, E. and Lane, D., Antibodies - A Laboratory Manual, Cold Spring Harbor Laboratory Press, Cold Spring Harbor, 1988. Further information on cancer may be found in Cancer: Principles and Practice of Oncology (V.T. De Vita et al., eds., J.B. Lippincott Company, 7th ed., 2004 or 8th ed., 2008) and Weinberg, RA, The Biology of Cancer, Garland Science, 2006. In the event of a conflict or inconsistency with the specification, the specification shall control. The Applicants reserve the right to amend the specification to correct obvious errors. None of the content of the incorporated references shall limit the invention.

### Brief Description of the Drawings

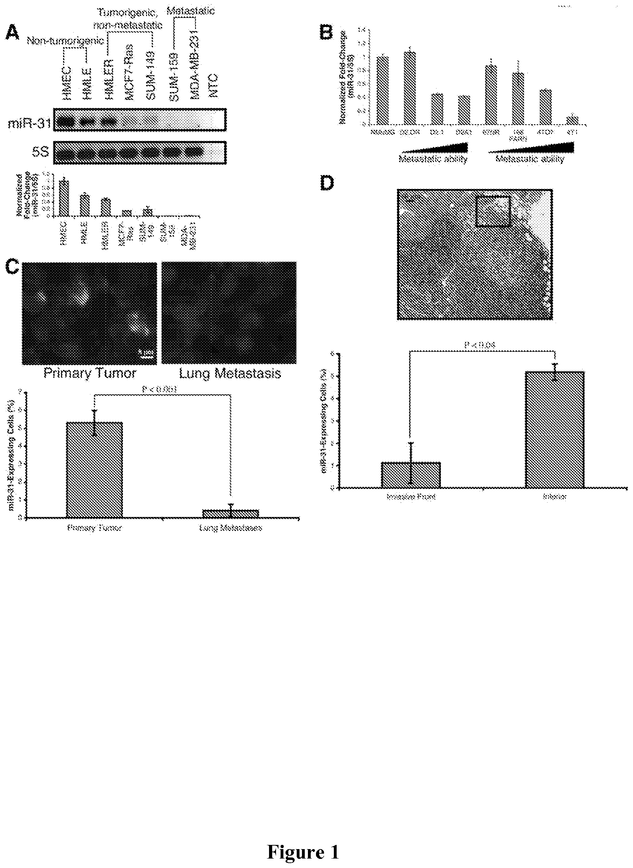

**Figure 1****. miR-31 Levels Correlate Inversely With Metastatic Ability in Breast Cell Lines.** (A) RT-PCR for miR-31 in seven human breast cell lines. 5S rRNA was a loading control. NTC: no template control. n = 3. (B) miR-31 RT-PCR in eight murine mammary cell lines. 5S rRNA was a loading control. n = 3. (C) *In situ* hybridization for miR-31 (green) in animal-matched 4T1 cell primary mammary tumors and lung metastases; DAPI counterstain (blue). n = 4. (D) Hematoxylin and eosin (H&E) stain of a 4T1 cell primary mammary tumor (top); box: invasive front. miR-31 *in situ* hybridization in 4T1 cells located near the invasive front or the interior of the primary tumors (bottom). n = 3.

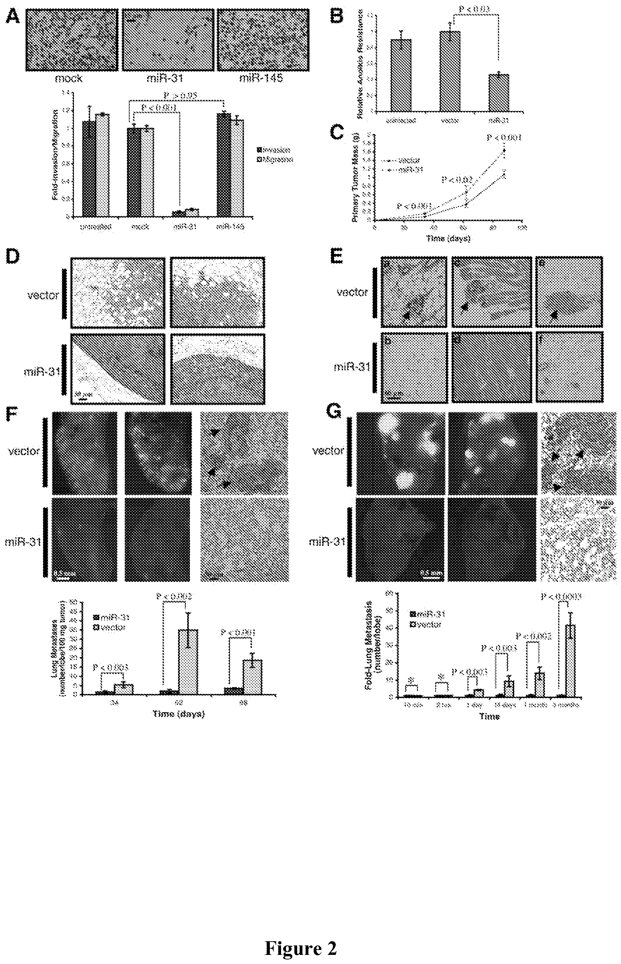

**Figure 2****. miR-31 Expression Inhibits Metastasis.** (A) Invasion and motility assays after transfection of MDA-MB-231 (231) cells with the indicated constructs. n = 3. (B) Anoikis assays using 231 cells infected as indicated. n = 3. (C) Primary tumor growth upon orthotopic injection of 1.0 x 10⁶ GFP-labeled 231 cells infected as indicated. The experiment was terminated after 13 weeks due to primary tumor burden. n = 5 per group per timepoint. (D) H&E stain of 231 primary tumors 62 days after orthotopic injection. (E) H&E stain of tissue adjacent to the indicated 231 primary mammary tumors 62 days after injection. Arrows: disseminated tumor cells in normal fat (a, b), muscle (c, d), and subcutis (e, f). (F) Images of murine lungs to visualize GFP-labeled 231 cells 62 days after orthotopic implantation (left). H&E stain of lungs from animals bearing the indicated tumors (right); arrows: metastatic foci. n = 5. (G) Images of murine lungs to detect GFP-labeled 231 cells 88 days after tail vein injection (left). H&E stain of lungs (right); arrows: metastatic foci. Asterisks: P >0.66. n = 5, except for 10 min and two hrs (n = 4).

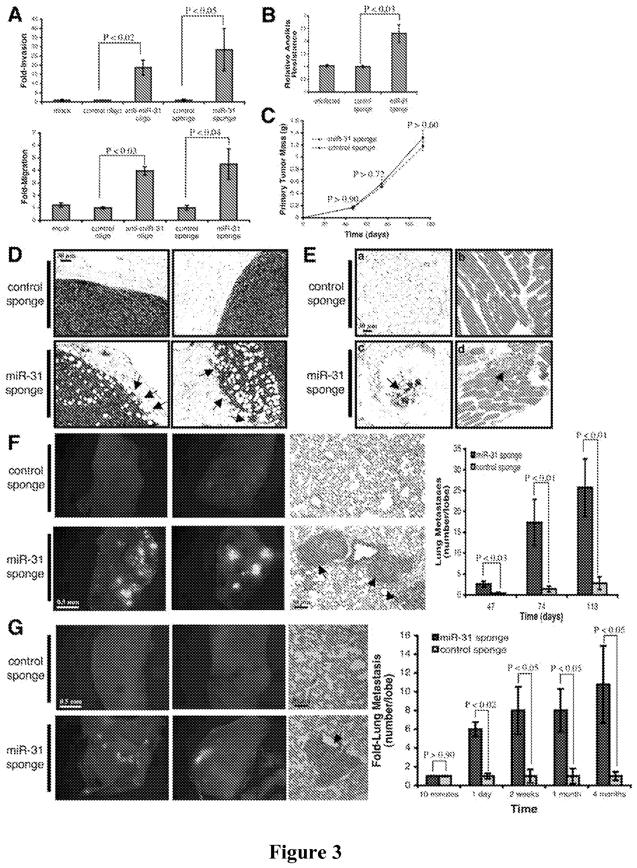

**Figure 3****. Inhibition of miR-31 Promotes Metastasis.** (A) Invasion and motility assays using MCF7-Ras cells transfected with the indicated transient miR-31 inhibitors. n = 3. (B) Anoikis assays with MCF7-Ras cells stably expressing the indicated sponge. n = 3. (C) Primary tumor growth upon orthotopic implantation of 5.0 x 10⁵ GFP-labeled MCF7-Ras cells infected as indicated. The experiment was terminated after 16 weeks due to primary tumor burden. n = 5 per group per timepoint. (D) H&E stain of MCF7-Ras primary tumors 47 days after orthotopic injection. Arrows: regions of poor encapsulation. (E) H&E stain of tissue adjacent to the indicated MCF7-Ras primary tumors 47 days post-injection. Arrows: disseminated tumor cells in normal fat (a, c) and muscle (b, d). (F) Images of murine lungs to visualize GFP-labeled MCF7-Ras cells 113 days after orthotopic injection (left). H&E stain of lungs from animals bearing the indicated tumors (middle); arrows: metastatic foci. n = 5. (G) Images of murine lungs to detect GFP-labeled MCF7-Ras cells 122 days after tail vein injection (left). H&E stain of lungs (middle); arrow: metastasis. n = 4, except for one day (n = 3).

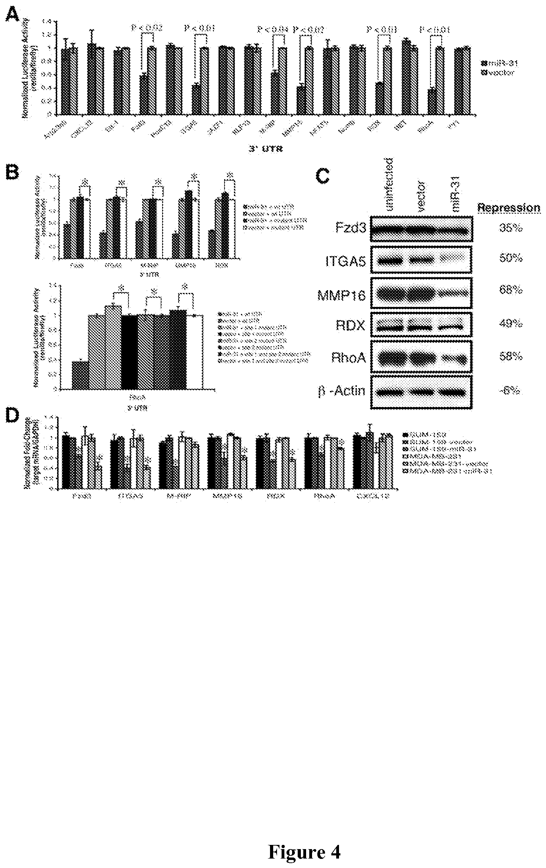

**Figure 4****. miR-31 Directly Regulates a Cohort of Pro-Metastatic Genes.** (A) Luciferase activity in 231 cells infected with miR-31 or control vector after transfection of the indicated 3' UTR-driven reporter constructs. n = 3. (B) Luciferase activity in the indicated 231 cells upon transfection of miR-31 site mutant 3' UTR-driven reporter constructs. wt: wild type; site 1: the miR-31 motif at nt 145-151 of the RhoA 3' UTR; site 2: the motif spanning nt 303-309. Asterisks: P >0.80 relative to mutant-UTR + vector controls. n = 3. (C) Immunoblots for endogenous Fzd3, ITGA5, MMP16, RDX, and RhoA in the indicated 231 cells. β-actin was a loading control. Repression: protein levels in miR-31-expressing cells relative to vector controls. (D) RT-PCR for endogenous CXCL12, Fzd3, ITGA5, M-RIP, MMP16, RDX, and RhoA. GAPDH was a loading control. Asterisks: P <0.03 relative to vector controls. n = 3.

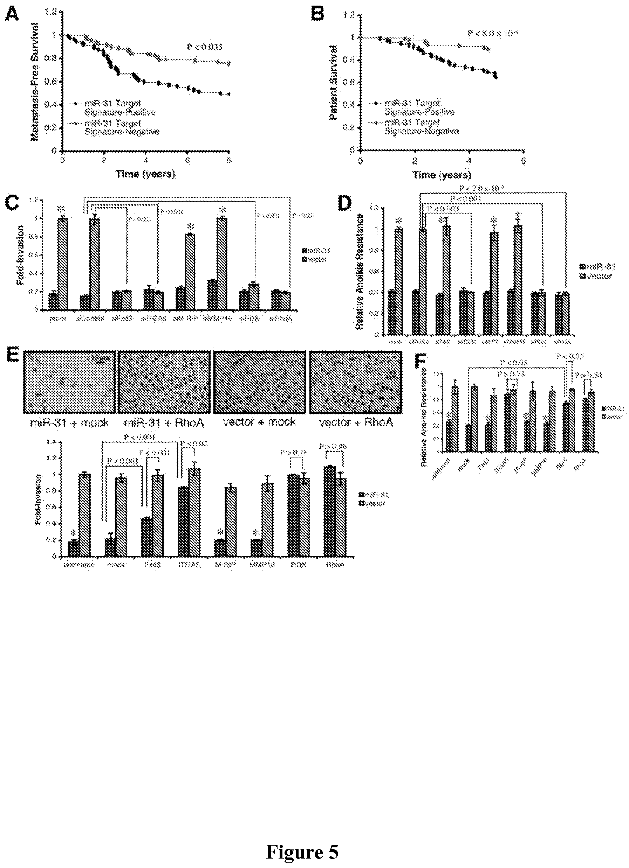

**Figure 5****. Repression of Fzd3, ITGA5, RDX, and RhoA Underlies miR-31-Dependent *Phenotypes in vitro.*** (A) Kaplan-Meier curves for 295 human primary breast tumors depicting metastasis-free survival, stratified based on expression of the six-gene miR-31 target signature. P-value based on a logrank test. (B) Kaplan-Meier five-year survival curves for 295 breast cancer patients, stratified based on miR-31 target signature expression in their primary tumors. P-value based on a logrank test. (C) Invasion assays with miR-31-expressing or control 231 cells transfected as indicated. Asterisks: P >0.19 relative to vector + siControl cells. n = 3. (D) Anoikis assays using 231 cells transfected with the indicated siRNAs. Asterisks: P >0.80 relative to vector + siControl cells. n = 3. (E) Invasion assays using the indicated 231 cells transfected with miRNA-resistant expression constructs. Asterisks: P >0.61 relative to miR-31 + mock cells. n = 3. (F) Anoikis assays with the indicated 231 cells transfected as noted. Asterisks: P >0.11 relative to miR-31 + mock cells. n = 3.

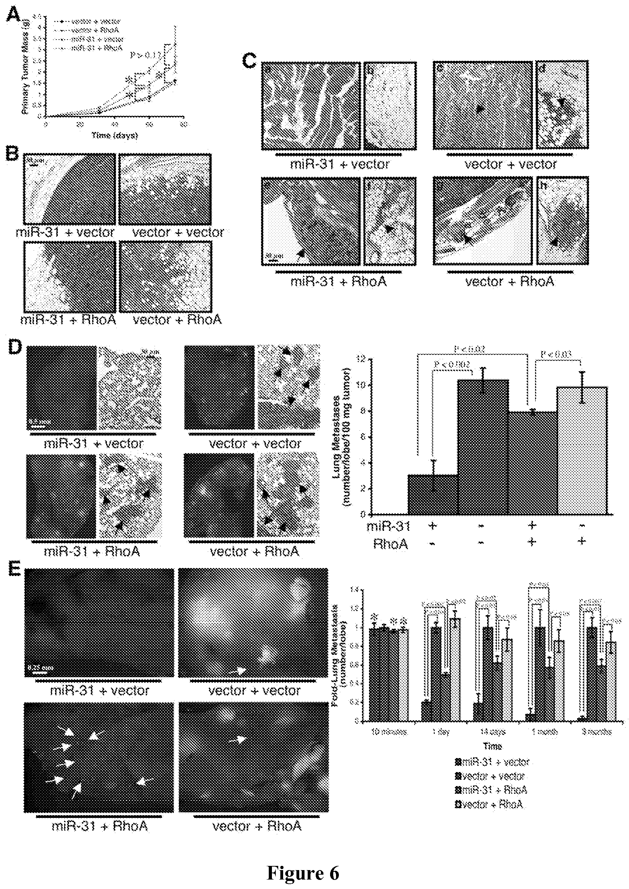

**Figure 6****. Re-Expression of RhoA Partially Reverses miR-31-Imposed Metastasis** ***Defects in vivo.*** (A) Primary tumor growth upon orthotopic injection of 5.0 x 10⁵ GFP-labeled 231 cells. The experiment was terminated after 11 weeks due to primary tumor burden. Asterisks: P <0.02. n = 5 per group per timepoint. (B) H&E stain of 231 primary tumors 60 days after orthotopic injection. (C) H&E stain of tissue adjacent to the indicated 231 primary mammary tumors 60 days after injection. Arrows: disseminated tumor cells in normal muscle (a, c, e, g) and fat (b, d, f, h). (D) Images of murine lungs to visualize GFP-labeled 231 cells 60 days after orthotopic injection (left). H&E stain of lungs from animals bearing the indicated tumors (right); arrows: metastatic foci. n = 5. (E) Images of murine lungs to detect GFP-labeled 231 cells 86 days after tail vein injection (left); arrows: micrometastatic lesions. Asterisks: P >0.87 relative to vector + vector controls. n = 4, except for 2 weeks (n = 3).

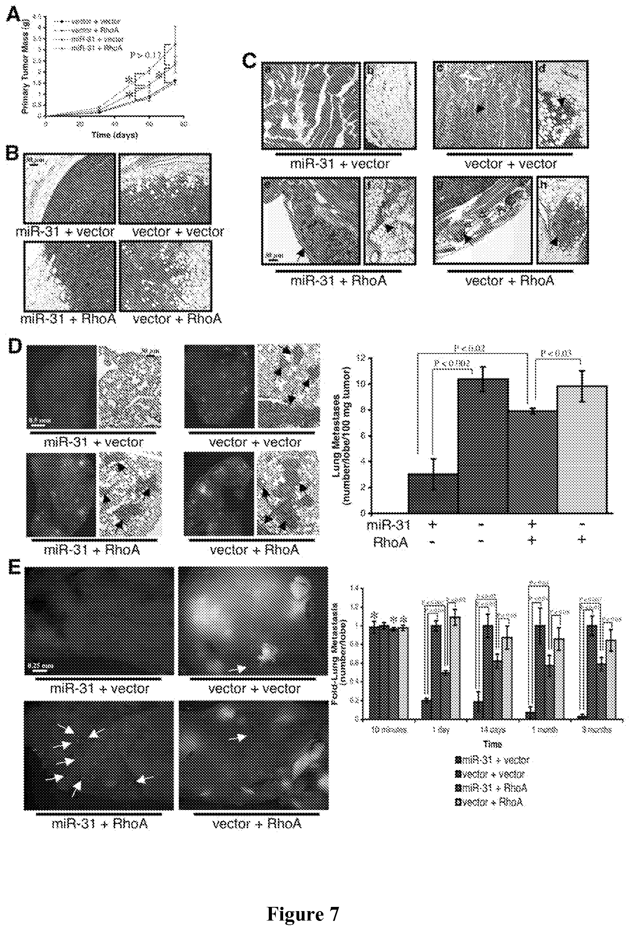

**Figure 7****. miR-31 Levels Correlate Inversely With Metastasis in Human Breast Tumors.** (A) miR-31 RT-PCR in 54 primary breast tumors. Normal: tissue from non-diseased individuals; metastasis-positive and -free: tumors of the indicated distant metastasis outcome. 5S rRNA was a loading control. n = 4 (normal); n = 14 (metastasis-positive); n = 40 (metastasis-free). (B) Kaplan-Meier distant metastasis-free survival curves for 54 breast cancer patients, stratified based on miR-31 levels in their primary tumors. P-value based on a chi-square test. (C) *In situ* hybridization for miR-31 (green) in patient-matched primary breast tumors and distant metastases (patient 1 = lung; 2 = pleura); DAPI counterstain (blue). n = 8 fields. (D) Immunohistochemical detection of ITGA5, RDX, and RhoA in patient-matched primary breast tumors and distant metastases (patient 1 = lung; 2 = pleura). n = 8 fields.

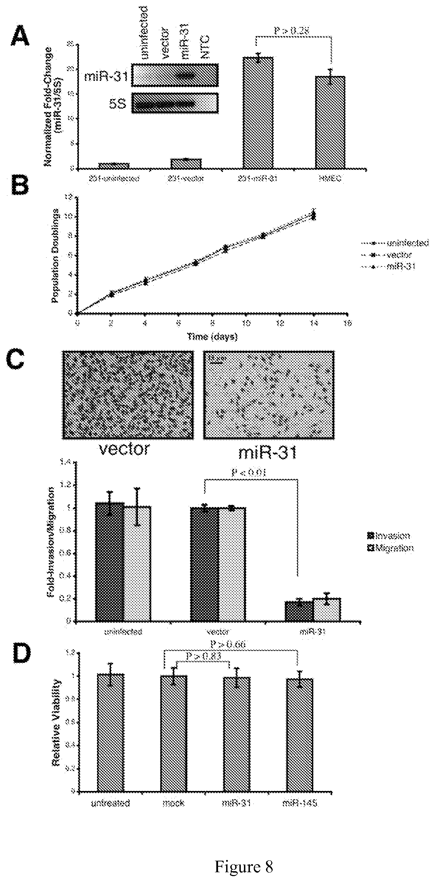

**Figure 8****. miR-31 Does Not Affect MDA-MB-231 Cell Viability and** ***Proliferation in vitro**.*(A) RT-PCR for miR-31 in MDA-MB-231 (231) cells infected with human miR-31 or control vector, as well as endogenous miR-31 levels in normal human mammary epithelial cells (HMECs). 5S rRNA was a loading control. NTC: no template control. n = 3. (B) *In vitro* growth curves of 231 cells expressing miR-31 or control vector. Triplicate wells from each cohort were counted for 14 days, as indicated. n = 3. (C) Invasion and motility assays using 231 cells infected with miR-31 or vector control. n = 3. (D) Trypan blue dye exclusion assay using 231 cells transfected with the indicated expression constructs for 24 hrs. n = 3.

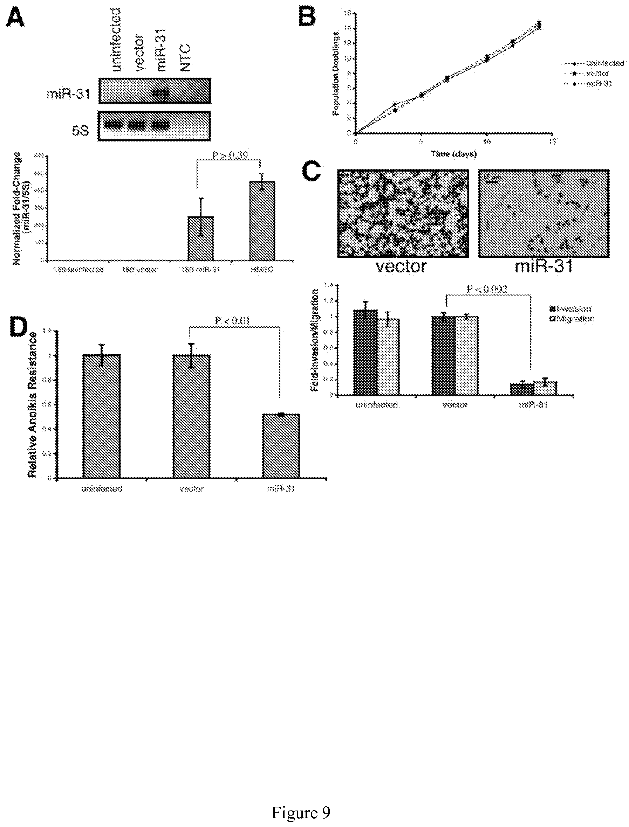

**Figure 9****. miR-31 Inhibits SUM-159 Invasion, Motility, and Anoikis** ***Resistance in vitro.*** (A) RT-PCR for miR-31 in SUM-159 (159) cells infected with miR-31 or control vector, as well as endogenous miR-31 in normal HMECs. 5S rRNA was a loading control. n = 3. (B) *In vitro* growth curves of 159 cells infected with miR-31 or vector control. Triplicate wells from each cohort were counted for 14 days, as indicated. n = 3. (C) Invasion and motility assays utilizing 159 cells expressing miR-31 or control vector. n = 3. (D) Anoikis assays using 159 cells infected with miR-31 or vector control. Cells were cultured in anchorage-independence for 24 hrs, then cell viability was assessed by trypan blue stain. n = 3.

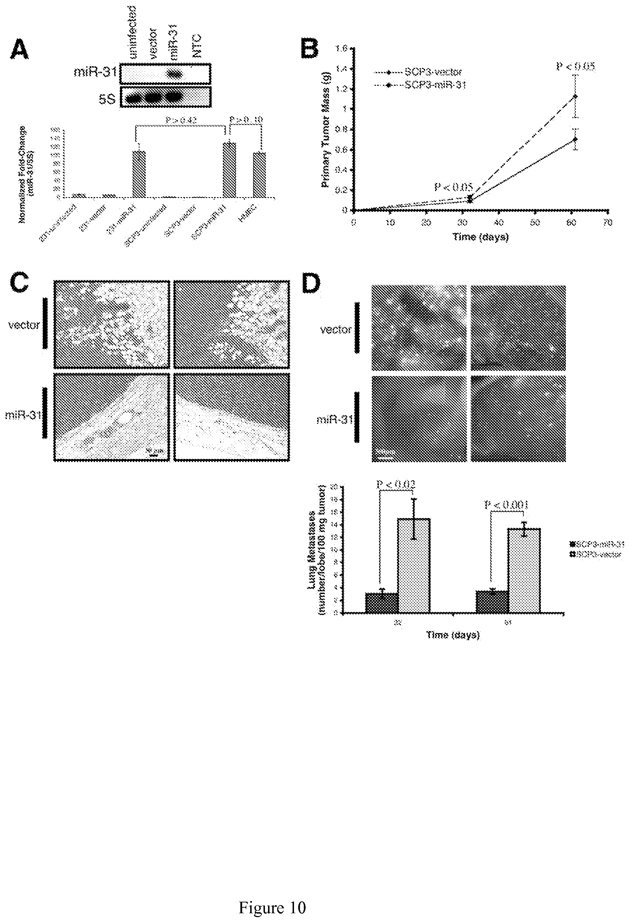

**Figure 10****. miR-31 Promotes Primary Tumor Growth and Inhibits Both Local Invasion and Lung Metastasis in a Single-Cell-Derived Population of MDA-MB-231 Cells.** (A) RT-PCR for miR-31 in SCP3 cells overexpressing miR-31 or control vector, the parental population of 231 cells infected as indicated, and endogenous miR-31 levels in normal HMECs. 5S rRNA was a loading control. n = 3. (B) Primary tumor growth kinetics upon orthotopic injection of 1.0 x 10⁶ GFP-labeled SCP3 cells infected as indicated. n = 5 per group per timepoint. (C) Hematoxylin and eosin (H&E) stain of the indicated SCP3 primary tumors 32 days after orthotopic injection. (D) Fluorescent images of murine lungs to visualize the indicated GFP-labeled SCP3 cells 61 days after orthotopic implantation (top panels). n = 5 per cohort per timepoint.
F**igure 11. miR-31 Promotes Primary Tumor Growth and Inhibits Local Invasion in SUM-159 Cells.** (A) Primary tumor burden 32 days after orthotopic injection of 1.0 x 10⁶ SUM-159 cells infected as indicated. n = 5 per group per timepoint. (B) H&E stain of the indicated SUM-159 primary tumors 32 days after orthotopic implantation.

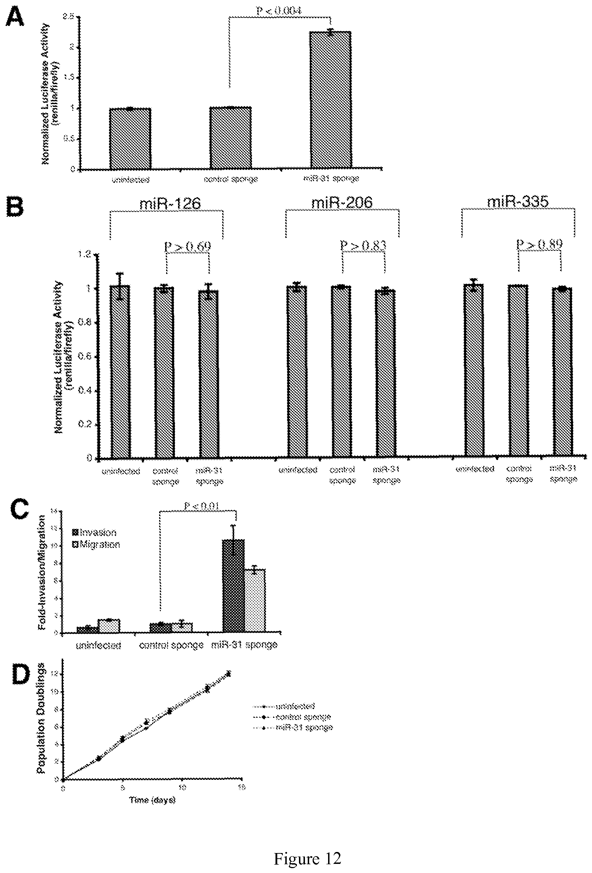

**Figure 12****. Modified miRNA Sponges Stably and Specifically Inhibit miR-31 in MCF7-Ras Cells.** (A) Luciferase assays upon stable infection of MCF7-Ras cells with a modified miR-31 sponge or control sponge. Cells were co-transfected with a *Renilla* luciferase reporter containing a miR-31 motif in its 3' UTR and a control firefly luciferase reporter. 24 hrs after transfection with the reporter genes, cell lysates were harvested and luciferase activity was quantitated. n = 3. (B) Luciferase assays using MCF7-Ras cells stably infected with a miR-31 or control sponge. Cells were co-transfected with a *Renilla* luciferase reporter containing either a miR-126, miR-206, or miR-335 motif in its 3' UTR and a control firefly luciferase reporter. 24 hrs after transfection with the reporters, lysates were harvested and luciferase activity quantified. n = 3. (C) Invasion and motility assays using MCF7-Ras cells stably expressing a miR-31 sponge or control sponge. n = 3. (D) *In vitro* growth curves of MCF7-Ras cells infected with a miR-31 sponge or control sponge. Triplicate wells from each cohort were counted for 14 days, as indicated. n = 3.

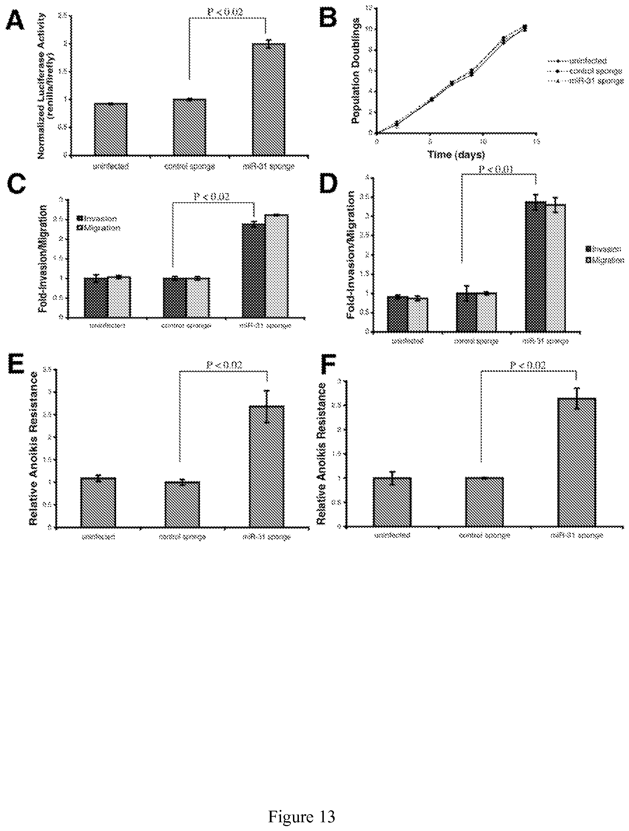

**Figure 13****. miR-31 Inhibits HME and SUM-149 Invasion, Motility, and Anoikis Resistance** ***in vitro.*** (A) Luciferase assays upon infection of HME cells with a miR-31 or control sponge. Cells were co-transfected with a *Renilla* luciferase reporter containing a miR-31 motif in its 3' UTR and a control firefly luciferase reporter. 24 hrs after transfection with the reporters, cell lysates were harvested and luciferase activity was quantitated. n = 3. (B) *In vitro* growth curves of HME cells infected with a miR-31 or control sponge. Triplicate wells from each cohort were counted for 14 days, as indicated. n = 3. (C) Invasion and motility assays using HME cells infected with the indicated sponges. n = 3. (D) Invasion and motility assays utilizing SUM-149 cells infected with a miR-31 or control sponge. n = 3. (E) Anoikis assays with HME cells infected as indicated. Cells were cultured in anchorage-independence for 24 hrs, then cell viability was assessed by trypan blue stain. n = 3. (F) Anoikis assays using SUM-149 cells infected as indicated. Cells were cultured in anchorage-independence for 24 hrs, then cell viability was assessed via trypan blue stain. n = 3.

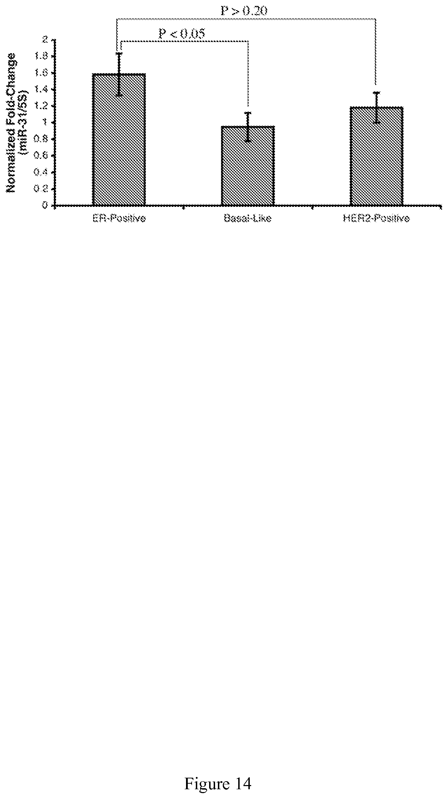

**Figure 14****. miR-31 Levels in Primary Human Breast Tumors Partially Correlate With Molecular Subtype.** miR-31 levels in the indicated Grade II and Grade III primary breast tumors, as assessed by RT-PCR. 5S rRNA was a loading control. n = 13 (ER⁺); n = 19 (basal-like); n = 16 (HER2⁺).

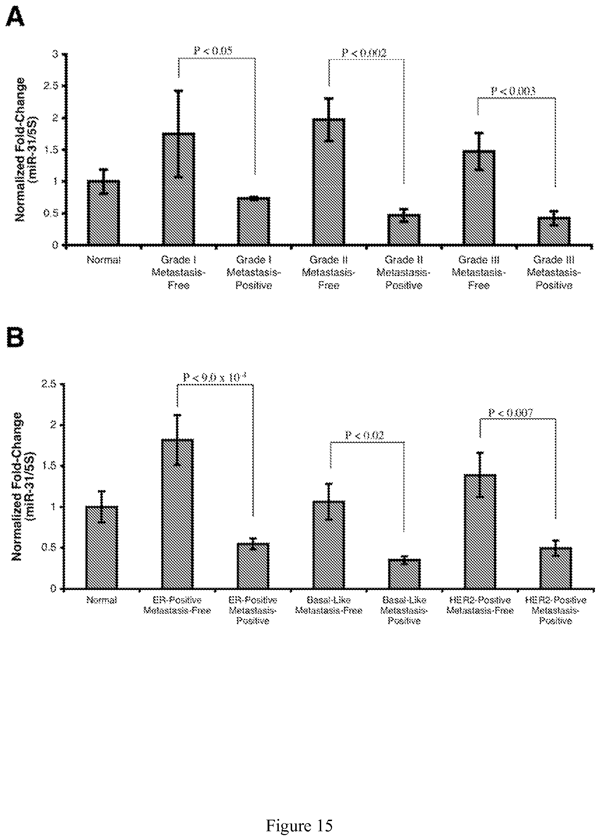

**Figure 15****. miR-31 Expression in Primary Human Breast Carcinoma**s **Correlates With Metastatic Recurrence Independent of Tumor Grade and Subtype.** (A) miR-31 levels in primary breast tumors of the indicated grade and metastasis status, as assessed by RT-PCR. 5S rRNA was a loading control. Normal: RNA derived from tissue of non-diseased individuals; metastasis-positive and -free: RNA from primary tumors of the indicated distant metastasis outcome. n = 4 (normal); n = 6 (Grade I, metastasis-free); n = 1 (Grade I, metastasis-positive); n = 9 (Grade II, metastasis-free); n = 5 (Grade II, metastasis-positive); n = 25 (Grade III, metastasis-free); n = 9 (Grade III, metastasis-positive). (B) RT-PCR for miR-31 in primary breast tumors of the indicated molecular subtype and metastasis status. 5S rRNA was a loading control. Normal: RNA derived from tissue of non-diseased individuals; metastasis-positive and -free: RNA from primary tumors of the indicated distant metastasis outcome. n = 4 (normal); n = 16 (ER-positive, metastasis-free); n = 4 (ER-positive, metastasis-positive); n = 10 (basal-like, metastasis-free); n = 5 (basal-like, metastasis-positive); n = 13 (HER2-positive, metastasis-free); n = 6 (HER2-positive, metastasis-positive).

### Detailed Description of Certain Embodiments of the Invention

### I. Compositions and Methods Relating to miR-31 and its Role in Metastasis

MicroRNAs (miRNAs) constitute an evolutionarily conserved class of small RNAs ranging from about 20 to 25 nucleotides (nt), e.g., 21-22 nt, in length that play a variety of important regulatory roles in animals and plants by targeting mRNAs for cleavage or translational repression in a sequence-specific manner. In mammalian cells, miRNAs effect gene silencing post-transcriptionally via translational inhibition (e.g., via sequence-specific interactions with the 3' untranslated regions (UTRs) of cognate mRNA targets) and/or mRNA degradation (Bartel, D., MicroRNAs: genomics, biogenesis, mechanism, and function. Cell, 116(2):281-97, 2004). miRNA biogenesis involves nuclear cleavage of primary miRNA transcripts, called pri-miRNAs, resulting in an approximately 60-70 nt stem loop intermediate, known as the miRNA precursor, or pre-miRNA, which is transported to the cytoplasm where it undergoes further processing by Dicer, leaving an siRNA-like imperfect duplex that comprises the mature miRNA and a similar-sized fragment (miRNA*) derived from the other arm of the pre-miRNA. MicroRNAs become incorporated into a ribonucleoprotein complex termed the RNA-induced silencing complex (RISC), wherein they direct downregulation of gene expression by mRNA cleavage and/or translational repression. In general, a miRNA can specify cleavage if the mRNA has sufficient complementarity to the miRNA and repress productive translation if the mRNA has a suitable constellation of miRNA complementary sites, which are often located in the 3' UTR.

miRNAs can modulate a wide variety of biological processes and are known to regulate genes relevant to a number of human diseases. A central role for miRNAs in the establishment and progression of human tumors has begun to emerge. More than 50% of miRNA-encoding loci reside in chromosomal regions altered during tumorigenesis (Calin et al., 2004), and expression profiling reveals characteristic miRNA signatures for many tumor types - including breast neoplasias - that predict disease status and clinical outcome (Calin and Croce, 2006). In addition, miRNAs have been identified that function as classical oncogenes or tumor suppressor genes (Ventura and Jacks, 2009), as well as a limited number that act at late stages of tumor progression (Ma et al., 2007; Tavazoie et al., 2008; Huang et al., 2008; Asangani et al., 2008; Zhu et al., 2008; Lujambio et al., 2008). The extent to which miRNAs specifically affect metastasis remains unclear, as all the miRNAs reported to affect metastasis also exert potentially confounding influences on primary tumor development, apoptosis, and/or cell proliferation (Voorhoeve et al., 2006; Sathyan et al., 2007; Ma et al., 2007; Si et al., 2007; Tavazoie et al., 2008; Kondo et al., 2008; Lujambio et al., 2008). Moreover, a role for miRNAs in steps of the invasion-metastasis cascade subsequent to local invasion has not been described.

A limited number of miRNAs with pro- (miR-10b, -21, and -373/520c) or anti-metastatic (miR-34b/c, -126, -148a, -206, and -335) functions have been identified. However, the contributions of miR-10b, miR-21, and miR-373/520c specifically to metastasis-promotion are not easily discerned due to their mitogenic and/or anti-apoptotic roles (Voorhoeve et al., 2006; Ma et al., 2007; Si et al., 2007). Similarly, the anti-metastatic miRNAs miR-34b/c, miR-126, and miR-148a impair primary tumor growth (Lujambio et al., 2008; Tavazoie et al., 2008), while miR-206 and miR-335 inhibit proliferation or promote apoptosis (Sathyan et al., 2007; Kondo et al., 2008), again obscuring their precise roles in metastasis.

The pleiotropic nature of gene regulation exhibited by miRNAs led the inventors to hypothesize that certain miRNAs might be endowed with a capacity to function as crucial modulators of tumor metastasis. As described further in the Examples, the inventors identified an anti-metastatic human miRNA, miR-31, that acts at multiple steps of the invasion-metastasis cascade via repression of a cohort of pro-metastatic targets. The inventors observed that miR-31 downregulation or deletion of the miR-31 genomic locus in human breast cancers was detected in genome-wide studies (Calin et al., 2004; Zhang et al., 2006; Yan et al., 2008). Examination of expression profiles of clinical breast tumors revealed reduced miR-31 in luminal B (relative to luminal A), basal-like, and HER2⁺ tumors (see Mattie et al., 2006; Blenkiron et al., 2007, for these studies) - patterns of reduction that correlate with aggressive disease (Sørlie et al., 2001). In contrast, another profiling study found elevated miR-31 in human breast tumors (Volinia et al., 2006). None of these studies stratified patients by metastasis status. Furthermore, miR-31 was but one of numerous genes that were differentially expressed. In addition, miR-31 was reported to be upregulated in colorectal cancer tumors (Slaby et al. Oncology 2007 72:397-402), but downregulated in breast cancer (Yan et al. RNA 2008 14:2348-2360). miR-31 was previously reported to induce cell motility in a K15M-induced cell migration assay (Tsai et al. Journal of Virology 2008 83:622-632). WO2008/036776 describes microarray gene expression analyses that were performed to identify genes that are mis-regulated by inhibition of hsa-miR-31 expression. Based on the analysis it was concluded that hsa-miR-31 directly or indirectly affects primarily cellular development-related genes and thus primarily affects functional pathways related to cellular development.

The present invention is based at least in part on the discovery that miR-31 is a pleiotropically acting miRNA that is necessary and sufficient to inhibit breast cancer metastasis. As described in Example 9, miR-31 expression in primary breast tumors correlated inversely with metastatic recurrence. The inventors showed that miR-31 normally acts to inhibit expression of certain target genes, e.g., Fzd3, ITGA5, M-RIP, MMP16, RDX, and RhoA. Accordingly, loss of miR-31 results in upregulation of miR-31 target genes, and such upregulation is associated with increased metastasis. Based at least in part on these discoveries, the invention provides compositions and methods for diagnosis and prognosis, e.g., to assess or predict likelihood that a tumor has or will metastasize and/or to assess the tumor aggressiveness or likelihood of recurrence. The invention further provides compositions and methods for research or therapy (e.g., to reduce likelihood of metastasis and/or to inhibit growth of metastases and/or to inhibit one or more aspects considered characteristic of a metastatic phenotype such as invasiveness or motility). In some embodiments the compositions and methods mimic the effects of miR-31, e.g., they inhibit one or more miR-31 target gene(s). Such agents could be, e.g., oligonucleotides (e.g., RNAs) identical or sufficiently similar to miR-31 to inhibit one or more miR-31 target genes or siRNA targeted to a miR-31 target gene. In some embodiments the compositions are pharmaceutical compositions. In some embodiments the compositions or agents contained in compositions are isolated (e.g., separated from at least some other components with which they are found in nature or synthetically produced).

Agents (also termed "compounds" herein) of use in various embodiments of the present invention can be, e.g., peptides, polypeptides, nucleic acids, oligonucleotides, small molecules (e.g., organic compounds having a molecular weight equal to or less than 1.5 kD, e.g., less than 1 kD, and usually multiple carbon-carbon bonds), antibodies, sugars, lipids, etc. It will be understood that an agent may comprise two or more of the foregoing types of materials, which may be covalently linked. In some embodiments, a "small molecule" refers to an organic compound having multiple carbon-carbon bonds and a molecular weight of less than 2000 daltons, e.g., between 50 and 1,500 daltons. Typically such compounds comprise one or more functional groups that mediate structural interactions with proteins, e.g., hydrogen bonding, and typically include at least an amine, carbonyl, hydroxyl or carboxyl group, and in some embodiments at least two of the functional chemical groups. A small molecule may comprise cyclic carbon or heterocyclic structures and/or aromatic or polyaromatic structures substituted with one or more chemical functional groups and/or heteroatoms. With regard to nucleic acids, e.g., oligonucleotides (which typically refers to short nucleic acids, e.g., 50 nucleotides in length or less), it is contemplated to use single-stranded, double-stranded (ds), blunt-ended, double-stranded with overhangs, in various embodiments of the invention. The full spectrum of modifications (e.g., nucleoside and/or backbone modifications), non-standard nucleotides, delivery vehicles and systems, etc., known in the art as being useful in the context of siRNA or antisense-based molecules for research or therapeutic purposes is contemplated for use in various embodiments of the instant invention. Polypeptides of use herein may contain amino acids such as those that are naturally found in proteins (e.g., the 20 "standard" amino acids), amino acids that are not naturally found in proteins, and/or amino acid analogs that are not amino acids. One or more of the amino acids in a polypeptide may be modified, for example, by the addition of a chemical entity such as a carbohydrate group, a phosphate group, a fatty acid group, etc. Nucleic acids and polypeptides can be produced using any suitable method known in the art. In some embodiments a nucleic acid or polypeptide is chemically synthesized. In some embodiments a nucleic acid is produced using enzymatic synthesis, e.g., by in vitro transcription or amplification reaction such as PCR. In some embodiments a polypeptide is produced using enzymatic synthesis, e.g., by in vitro translation. In some embodiments a nucleic acid or polypeptide is synthesized by a cell or multicellular organism and isolated therefrom. The cell or organism may be genetically modified to express the nucleic acid. The term "antibody" encompasses immunoglobulins and derivatives thereof containing an immunoglobulin domain capable of binding to an antigen. An antibody can originate from any mammalian or avian species, e.g., human, rodent (e.g., mouse, rabbit) goat, chicken, etc., or can be generated using, e.g., phage display. The antibody may be a member of any immunoglobulin class, e.g., IgG , IgM, IgA, IgD, IgE, or subclasses thereof such as IgG1, IgG2, etc. In various embodiments of the invention "antibody" refers to an antibody fragment such as an Fab', F(ab')2, scFv (single-chain variable) or other fragment that retains an antigen binding site, or a recombinantly produced scFv fragment, including recombinantly produced fragments. An antibody can be monovalent, bivalent or multivalent in various embodiments. The antibody may be a chimeric or "humanized" antibody, which can be generated using methods known in the art. An antibody may be polyclonal or monoclonal, though for purposes of the present invention monoclonal antibodies are generally preferred. Methods for producing antibodies that specifically bind to virtually any molecule of interest are known in the art. For example, monoclonal or polyclonal antibodies can be purified from blood or ascites fluid of an animal that produces the antibody (e.g., following natural exposure to or immunization with the molecule or an antigenic fragment thereof), can be produced using recombinant techniques in cell culture or transgenic organisms, or can be made at least in part by chemical synthesis. A compound or agent of use in the present invention (e.g., for research, testing, prognosis, and/or therapy) may be provided as part of a composition, which may be composed of one or more than one molecular entity. The composition may, but need not contain, e.g., an ion, salt, aqueous or non-aqueous diluent or carrier, buffer, enzyme inhibitor, preservative, etc.

In some aspects, the invention relates to or makes use of genetically modified cells e.g., cells that have been genetically modified to have reduced miR-31 expression or activity. A "genetically modified" or "engineered" cell refers to a cell into which a nucleic acid has been introduced by a process involving the hand of man (or a descendant of such a cell that has inherited at least a portion of the nucleic acid). The nucleic acid may for example contain a sequence that is not naturally found in the cell, it may contain native sequences (i.e., sequences naturally found in the cell) but in a non-naturally occurring arrangement (e.g., a coding region linked to a promoter from a different gene), or altered versions of native sequences, etc. The process of transferring the nucleic acid into the cell can be achieved by any suitable technique and will often involve use of a vector. Suitable techniques include calcium phosphate or lipid-mediated transfection, electroporation, and transduction or infection using a viral vector. In some embodiments the nucleic acid or a portion thereof is integrated into the genome of the cell and/or is otherwise stably heritable. The nucleic acid may have subsequently been removed or excised from the genome, provided that such removal or excision results in a detectable alteration in the cell relative to an unmodified but otherwise equivalent cell. In some aspects, the invention relates to or makes use of genetically modified multi-cellular organisms. An organism at least some of whose cells are genetically engineered or that is derived from such a cell is considered a genetically engineered organism. As used herein, a "vector" may comprise any of a variety of nucleic acid molecules into which a desired nucleic acid may be inserted, e.g., by restriction digestion followed by ligation. A vector can be used for transport of such nucleic acid between different environments, e.g., to introduce the nucleic acid into a cell of interest and, optionally, to direct expression in such cell. Vectors are often composed of DNA although RNA vectors are also known. Vectors include, but are not limited to, plasmids and virus genomes or portions thereof. Vectors may contain one or more nucleic acids encoding a marker suitable for use in the identifying and/or selecting cells that have or have not been transformed or transfected with the vector. Markers include, for example, proteins that increase or decrease either resistance or sensitivity to antibiotics or other compounds, enzymes whose activities are detectable by standard assays known in the art (e.g., β-galactosidase or alkaline phosphatase), and proteins or RNAs that detectably affect the phenotype of transformed or transfected cells (e.g., fluorescent proteins). An expression vector is one into which a desired nucleic acid may be inserted such that it is operably linked to regulatory elements (also termed "regulatory sequences", "expression control elements", or "expression control sequences") and may be expressed as an RNA transcript (e.g., an mRNA that can be translated into protein or a noncoding RNA such as an shRNA or miRNA precursor). Regulatory elements may be contained in the vector or may be part of the inserted nucleic acid or inserted prior to or following insertion of the nucleic acid whose expression is desired. As used herein, a nucleic acid and regulatory element(s) are said to be "operably linked" when they are covalently linked so as to place the expression or transcription of the nucleic acid under the influence or control of the regulatory element(s). For example, a promoter region would be operably linked to a nucleic acid if the promoter region were capable of effecting transcription of that nucleic acid. One of skill in the art will be aware that the precise nature of the regulatory sequences needed for gene expression may vary between species or cell types, but can in general include, as necessary, 5' non-transcribed and/or 5' untranslated sequences that may be involved with the initiation of transcription and translation respectively, such as a TATA box, cap sequence, CAAT sequence, and the like. Other regulatory elements include IRES sequences. Such 5' non-transcribed regulatory sequences will include a promoter region that includes a promoter sequence for transcriptional control of the operably linked gene. Regulatory sequences may also include enhancer sequences or upstream activator sequences. Vectors may optionally include 5' leader or signal sequences. Vectors may optionally include cleavage and/or polyadenylations signals and/or a 3' untranslated regions. The choice and design of an appropriate vector and regulatory element(s) is within the ability and discretion of one of ordinary skill in the art. For example, one of skill in the art will select an appropriate promoter (or other expression control sequences) for expression in a desired species (e.g., a mammalian species) or cell type. One of skill in the art is aware of regulatable (e.g., inducible or repressible) expression systems such as the Tet system and others that can be regulated by small molecules and the like, as well as tissue-specific and cell type specific regulatory elements. In some embodiments, a virus vector is selected from the group consisting of adenoviruses, adeno-associated viruses, poxviruses including vaccinia viruses and attenuated poxviruses, retroviruses (e.g., lentiviruses), Semliki Forest virus, Sindbis virus, etc. Optionally the virus is replication-defective. In some embodiments a replication-deficient retrovirus (i.e., a virus capable of directing synthesis of one or more desired transcripts, but incapable of manufacturing an infectious particle) is used. Various techniques may be employed for introducing nucleic acid molecules into cells. Such techniques include transfection of nucleic acid molecule-calcium phosphate precipitates, transfection of nucleic acid molecules associated with DEAE, transfection or infection with a virus that contains the nucleic acid molecule of interest, liposome-mediated transfection, nanoparticle-mediated transfection, and the like.

In some aspects, methods of the invention involve a subject. A "subject" may be a human or a non-human animal. The subject may be suffering from a disease (e.g., cancer) warranting medical and/or surgical attention or in need of prognostic information or may be at increased risk of developing a disease relative to an average member of the population. In some embodiments a subject is a female. A non-human subject may be a non-human mammal, e.g., a rodent such as a mouse, rat, hamster, rabbit, or guinea pig; a dog, a cat, a bovine or ovine, a non-human primate, etc. In some embodiments, the subject may serve as an animal model for cancer, e.g., breast cancer. For example, the subject may be a genetically engineered non-human mammal, e.g., a mouse, that has a predisposition to develop tumors. The mammal may overexpress an oncogene (e.g., as a transgene) or underexpress a tumor suppressor gene (e.g., the animal may have a mutation or deletion in the tumor suppressor gene).

In certain aspects, the invention provides compositions and methods for diagnosis and prognosis, e.g., for assessing or predicting the likelihood of tumor metastasis. In some embodiments the methods comprise assessing expression of a miR-31 gene product (e.g., assessing the level of mature miR-31 RNA or a miR-31 precursor RNA that is transcribed from the miR-31 gene, e.g., the pri-miRNA or pre-miRNA). In some embodiments the methods comprise assessing the level or activity of an expression product of a gene that is a target of regulation by miR-31. "Expression products" can refer to RNA transcribed from a gene and/or polypeptides obtained by translation of mRNA transcribed from a gene (including post-translationally processed forms thereof). The afore-mentioned methods may involve assessing level or activity in a biological sample obtained from a tumor. The sample may comprise cells obtained, for example, from a tissue biopsy, fine needle aspiration biopsy, surgical specimen, brushing, etc. In some embodiments the cells may be expanded in culture after being obtained from the tumor. In some embodiments the biological sample may be further processed (e.g., appropriately treated, stained, etc.) to render it suitable for use in subsequent assay procedures. Such processed material still falls within the scope of the terms "biological sample" or "sample." In some embodiments the methods are used in conjunction with measuring expression of one or more other microRNAs (e.g., one or more miRNAs with pro- or anti-metastatic properties) or with other diagnostic/prognostic approaches, e.g., assessing estrogen or progesterone receptor status, presence of mutations in oncogenes or tumor suppressor genes, etc. The methods may be used, e.g., to recommend or make treatment decisions, e.g., to select particular approaches (e.g., surgery, chemotherapy, radiation, etc.) to treating the tumor or preventing recurrence. In some embodiments, the invention involves comparison with an appropriate control. An appropriate control level may be, e.g., a level in nonmetastatic tumor, a level in normal tissues or cells (e.g., cells or tissues of the same type or from the same organ as that from which the tumor originated), etc. A control or reference level may be determined concurrently with assessing the biological sample of interest, or may be a historical control or reference level (e.g. an average measurement obtained from a plurality of samples collected prior to performing the method). The invention also relates to kits comprising agents and reagents suitable for use in the diagnostic, prognostic and therapeutic methods of the invention, optionally including instructions for use. Any of the kits can comprise instructions for using the contents of the kit to perform a method of the invention and, optionally, reagents useful to perform the method (e.g., enzymes, buffers) or suitable controls.

In some embodiments, inventive methods relate to a miR-31 target signature. Such a signature may be used in the diagnostic and prognostic methods of the present invention. In some embodiments, inventive methods relate to one or more miR-31 target genes. Embodiments of the invention relate to assessing expression or activity of one or more such genes in cells in culture. Embodiments of the invention relate to assessing expression or activity of one or more such genes in cells in vivo. Embodiments of the invention relate to modulating (e.g., increasing or inhibiting) expression or activity of one or more such genes in cells in culture. Embodiments of the invention relate to modulating (e.g., increasing or inhibiting) expression or activity of one or more such genes in cells in vivo. In some embodiments the cells are in an animal subject, e.g., a rodent, non-human primate, or human being. In some embodiments the rodent or non-human primate, or other non-human animal is an animal model useful for assessing tumor formation, development, and/or efficacy of therapeutic agents. In some embodiments the animal is immunocompromised. In some embodiments the cells are tumorigenic cells. In some embodiments the cells are non-tumorigenic cells. In some embodiments the cells are immortalized. In some embodiments the cells are genetically modified. For example, the cells may express one or more oncogenes, tumor suppressor genes, etc. Embodiments of the invention relate to any of the afore-mentioned genes or any subset thereof.

In various embodiments of the invention the tumor is a carcinoma, e.g., a breast carcinoma. In some embodiments the tumor is an adenocarcinoma. In some embodiments the tumor is a sarcoma. In some embodiments the tumor affects an organ or organ system selected from breast, lymph node, prostate, kidney, bladder, lung, liver, gastrointestinal tract, colon, testis, stomach, pancreas, thyroid, skin, ovary, uterus, cervix, skin, nerve, bone, and nervous system (e.g., brain). A variety of different tumor types may originate in certain of these organs, and the term "tumor" is intended to encompass all such tumor types. "Cancer", as used herein should be understood to encompass any tumor type that has the potential to metastasize, e.g., any malignant tumor (e.g., a carcinoma or sarcoma in various embodiments). In some embodiments the tumor originates from an epithelial cell, e.g., a breast epithelial cell.

The present application contemplates use of inventions, techniques, methods, and materials described in USSN 10/990,993 (US Pub. No. 20050193432), PCT/US2008/009639 filed August 11, 2008, and/or PCT/US08/85040 filed November 26, 2008, in the context of the present invention. For example, such inventions, techniques, methods, and/or materials may be useful in further analysis relating to role of miR-31 and/or miR-31 target genes in tumor metastasis and/or for screening and assessing therapeutic agents and drug candidates that relate to miR-31 and/or miR-31 target genes, etc. For example, the ability of an agent to inhibit a miR-31 target gene and/or the ability of agent to reduce the likelihood or magnitude of tumor growth or metastasis can be evaluated.

The sequence and genomic location of miR-31, e.g., human miR-31, are known in the art and are available in public databases such as miRBase (www.mirbase.org) miRBase: tools for microRNA genomics (Griffiths-Jones S, et al., NAR 2008 36(Database Issue):D154-D158; Griffiths-Jones S, et al., NAR 2006 34(Database Issue):D140-D144; Griffiths-Jones S. NAR 2004 32(Database Issue):D109-D111) and GenBank. The mature human miR-31 most commonly observed in cloning studies reportedly has the following sequence: AGGCAAGAUGCUGGCAUAGCU (SEQ ID NO: 1; miRBase accession number MIMAT0000089) and is located within a stem-loop predicted to have the following sequence:
GGAGAGGAGGCAAGAUGCUGGCAUAGCUGUUGAACUGGGAACCUGCUAUGCC AACAUAUUGCCAUCUUUCC (SEQ ID NO: 2; miRBase accession number MIMAT0004504; GenBank accession number NR_029505).

Some sequence at the 5' and 3' ends may not be included in the intermediate precursor miRNA produced by Drosha cleavage. It will be appreciated that sequence differences (polymorphisms) may exist among different individuals. For example, a sequence may differ at between 1 and 5 positions as compared with SEQ ID NO: 2. Furthermore, a mature miR-31 may have or lack additional nucleotide(s), e.g., 1 or 2 nts, at the 5' and/or 3' end. The invention encompasses use of miR-31 or miR-31 precursor from non-human animals in certain embodiments. For example, mouse miR-31 can be used, e.g., in embodiments that relate to models for tumor metastasis and drug discovery. Mature mouse miR-31 have the following sequence: AGGCAAGAUGCUGGCAUAGCUG (SEQ ID NO: 3; miRBase accession number MIMAT0000538) and is located in a stem-loop of the following sequence:
UGCUCCUGUAACUCGGAACUGGAGAGGAGGCAAGAUGCUGGCAUAGCUGUUG AACUGAGAACCUGCUAUGCCAACAUAUUGCCAUCUUUCCUGUCUGACAGCAG CU (SEQ ID NO: 4; miRBase accession number MI0000579). One of skill in the art can readily design probes to detect and/or amplify miR-31 or its stem-loop precursor or a portion thereof. One of skill in the art can also readily clone or synthesize DNA sequences encoding miR-31 or its stem-loop precursor, optionally together with flanking sequence(s), e.g., up to about 100-150 nt of flanking sequence. Such DNA can be inserted into a suitable vector and expressed in a cell of interest.

### II. Prognostic Methods

The invention provides a variety of methods that provide information, e.g., prognostic information relating to tumors, e.g., breast tumors. The methods are, in some embodiments, based at least in part on assessing (e.g., detecting, quantifying, etc.) the expression level or activity of miR-31 or a miR-31 precursor (or, in some embodiments, assessing the expression level or activity of one or more miR-31 target genes). Such methods are referred to herein as "miR-31-based prognostic methods or assays". In some embodiments, the level of miR-31 is used to discriminate between tumors at high risk of metastasis and those with a low risk. For example, in some embodiments, if a tumor has a significantly reduced level of miR-31 relative to a reference level the tumor is classified as having a high risk of metastasizing, while if the tumor does not have a significantly reduced level of miR-31 relative to a reference level, the tumor is classified as having a low risk. A reference level may be determined in a variety of ways. In some embodiments a reference level is an absolute level, while in other embodiments a reference level is a relative level. In some embodiments, tumors are classified as miR-31-positive or -negative based on comparison with a tumor cohort. For example, as described in Example 9, tumors may be considered miR-31-positive or -negative if the normalized expression of miR-31 resides in the top or bottom 30% of tumors in a cohort, respectively. Tumors that are miR-31 positive are at low risk of metastasis while tumors that are miR-31 negative are at high risk of metastasis. In other embodiments, tumors may be considered miR-31-positive or -negative if the normalized expression of miR-31 resides in the top or bottom 25% of tumors in a cohort, respectively. In other embodiments, tumors may be considered miR-31-positive or - negative if the normalized expression of miR-31 resides in the top or bottom 35% of tumors in a cohort, respectively. It will be understood that other values may be used to define miR-31 positive or negative tumors. In some embodiments one or more samples are obtained from a tumor, and one or more samples are obtained from nearby patient-matched normal (non-tumor) tissue composed of similar cell types. The relative level of miR-31 in the patient-matched non-tumor samples versus tumor samples is determined. In some embodiments, if the relative level (ratio) of miR-31 in the non-tumor samples versus the tumor samples is greater than a predetermined value (indicating that cells of the tumor have reduced or absent miR-31), the tumor is classified as high risk. In some embodiments the predetermined value is, e.g., at least 1.5, 2.0, 2.5, 3.0, etc. In some embodiments the predetermined value is between about 1.5 and about 10. Various risk categories may be defined. For example, tumors may be classified as at low, intermediate, or high risk of metastasis. A variety of statistical methods may be used to correlate the risk of metastasis with the relative or absolute level of miR-31 expression or loss of expression.

The invention provides a method assessing the likelihood of development of a tumor metastasis in a subject, comprising the steps of: (a) determining the level of a miR-31 gene product in a biological sample obtained from the subject; (b) comparing the level determined in (a) with an appropriate control level, wherein if the level determined in (a) is lower than the control level of the miR-31 gene product, then the subject has an increased likelihood of developing a metastasis (e.g., as compared with the likelihood if the level determined in (a) is not lower than the control level). A miR-31 gene product can be any transcript transcribed from the miR-31 gene or a processed form thereof. For example, a miR-31 gene product can be a mature miR-31 RNA or a miR-31 precursor RNA. In some embodiments, the biological sample is obtained from a tumor. In some embodiments the control level is determined using a sample of non-tumor tissue obtained from the subject. The invention further provides a method for assessing the likelihood that a tumor in a subject will metastasize or has metastasized, the method comprising (a) determining the level of an expression product of one or more miR-31 target gene(s) in a biological sample obtained from the tumor; and (b) comparing the level with a control level, wherein if the level determined in (a) is greater than the control level, the tumor is considered to have increased likelihood of metastasizing or having metastasized (e.g., as compared with the likelihood if the level determined in (a) is not greater than the control level). In some embodiments, expression levels of at least two miR-31 target genes are assessed. As used herein, a "miR-31 target gene" is a gene whose expression is directly regulated by miR-31, i.e., miR-31 directs cleavage and/or translational repression of the mRNA of the gene so that the expression level of the gene is higher in the absence of miR-31 than if miR-31 is present. In some embodiments the miR-31 target genes are selected from RhoA, RDX, and ITGA5. It will be appreciated that the prognostic methods could be expressed in terms of identifying a tumor having decreased risk of metastasizing or having metastasized. For example, a tumor that does not have reduced or absent levels of a miR-31 gene product as compared with a control has decreased likelihood of metastasizing as compared with the likelihood that would exist if the tumor does have reduced or absent levels of a miR-31 gene product. A tumor that does not have increased levels of expression of miR-31 target gene(s) as compared with a control has decreased likelihood of metastasizing as compared with the likelihood that would exist if the tumor does have increased levels of expression of miR-31 target gene(s),

The invention further provides a method for providing prognostic information relating to the likelihood that a tumor in a subject will metastasize, the method comprising (a) determining the level of a miR-31 gene product in a biological sample obtained from the tumor; and (b) comparing the level with a control level, wherein (i) if the level determined in (a) is less than the control level, the tumor is considered to have a poor prognosis; or (ii) if the level determined in (a) is greater than the control level, the tumor is considered to have a good prognosis. It is noted that references herein to "prognosis of a tumor", e.g., that a tumor has a good prognosis or a poor prognosis, and similar expressions, should be understood as meaning that a subject having such tumor has a good prognosis, or poor prognosis, respectively. In some embodiments, a prognostic method of the invention comprises providing a subject in need of treatment for cancer, e.g., breast cancer. In some embodiments, a prognostic method of the invention comprises diagnosing a subject in need of treatment for cancer, e.g., breast cancer.

A biological sample used in the miR-31 based prognostic methods will typically comprise or be derived from cells isolated from a subject. The cells will typically comprise tumor cells, e.g., breast tumor cells. Samples can be, e.g., surgical samples, tissue biopsy samples, fine needle aspiration biopsy samples, core needle samples. The sample may be obtained using methods known in the art. A sample can be subjected to one or more processing steps. In some embodiments the sample is frozen and/or fixed. In some embodiments the sample is sectioned and/or embedded, e.g., in paraffin. In some embodiments, tumor cells, e.g., epithelial tumor cells, are separated from at least some surrounding stromal tissue (e.g., stromal cells and/or extracellular matrix). Cells of interest can be isolated using, e.g., tissue microdissection, e.g., laser capture microdissection.

In some embodiments, cells of the sample are lysed. Nucleic acids or polypeptides may be isolated. In some embodiments RNA, optionally isolated from a sample, is reverse transcribed and/or amplified. In some embodiments short RNA (e.g., less than about 200 nucleotides) is separated from longer nucleic acids. A wide variety of solution phase or solid phase methods are available for detection of RNA, e.g., miRNA such as miR-31. Suitable methods include e.g., hybridization-based approaches (e.g., nuclease protection assays, Northern blots, microarrays, in situ hybridization), amplification-based approaches (e.g., reverse transcription polymerase chain reaction (which can be a real-time PCR reaction), or sequencing (e.g., RNA-Seq, which uses high throughput sequencing techniques to quantify RNA transcripts (see, e.g., Wang, Z., et al. Nature Reviews Genetics 10, 57-63, 2009)). In some embodiments of interest a quantitative PCR (qPCR) assay is used. Other methods include electrochemical detection, bioluminescence-based methods, fluorescence-correlation spectroscopy, etc. Probes composed at least in part of locked nucleic acids (LNA) are of use. In some embodiments, miR-31 is detected in a formalin-fixed sample using in situ hybridization. Prior to incubation with the probe the tissue is treated with EDC, a water-soluble fixative that irreversibly links the RNA's 5' phosphate to protein side chains. See, e.g., Pena, J, et al., Nature Methods 6, 139-141, 2009. Kits for isolating, detecting, and/or quantitating miRNAs are commercially available, such as the miRvana™ kits from Ambion/Applied Biosystems; the miRtect-IT™ miRNA Labeling and Detection Kit from USB Corp. (Affymetrix), which is a method for the labeling and detection of mature miRNA and small RNAs from total RNA by splinted-ligation technology. The splinted-ligation technology is a nucleic acid hybridization assay that uses a miRNA-specific Bridge Oligonucleotide to form base pairs with the miRNA and a Detection Oligonucleotide. The captured miRNA is subsequently ligated to the Detection Oligonucleotide. See, e.g., Maroney, PA, et al, Nature Protocols, 13 (1) 279-287, 2008. It will be understood that suitable controls and normalization procedures can be used to accurately quantify miR-31 expression. For example, values can be normalized based on the expression of a small RNA such as the 5S RNA or a microRNA whose expression is not correlated with cancer and/or with metastasis can be used. Values can also be normalized to account for the fact that different samples may contain different proportions of a cell type of interest, e.g., cancer cells, versus non-cancer cells. For example, the percentage of stromal cells, e.g., fibroblasts, may be assessed by measuring expression of a stromal cell-specific marker, and the overall results adjusted to accurately reflect miR-31 expression specifically in the tumor cells. In certain embodiments of the invention the level of miR-31 expression is not measured as part of a gene expression profile in which expression of at least 10 different genes (e.g., at least 20, 30, 50, or 100 genes) is measured (e.g., using a microarray). In certain embodiments of the invention, e.g., if miR-31 expression is measured as part of a gene expression profile in which expression of at least 10 different genes is measured (e.g., using a microarray), the level of miR-31 expression is used as an indicator of the likelihood of metastasis or as a prognostic indicator that is distinct from its contribution to the overall gene expression profile. In certain embodiments, the inventive methods involve using miR-31 expression level as an indicator of tumor aggressiveness, likelihood of metastasis, or prognosis in a manner that, at least in part, does not depend on the contribution of miR-31 to a gene expression profile. In one embodiment, miR-31 level is assessed as part of a gene expression profile, but the result obtained for miR-31 is analyzed or used in a manner distinct from its use as a contributor to a classification based on the overall gene expression profile. Expression levels miR-31 target genes may be assessed using any suitable method. Either mRNA or protein level may be measured. Exemplary methods for measuring mRNA include hybridization based assay, polymerase chain reaction assay, sequencing, in situ hybridization, etc. Exemplary methods for measuring protein level include ELISA assays, Western blot, mass spectrometry, or immunohistochemistry.

Results of a miR-31-based prognostic assay may be useful for selecting a therapeutic regimen for a subject. For example, such results may be useful in determining whether a subject should receive, e.g., would likely benefit from, administration of one or more chemotherapeutic agents (chemotherapy), hormonal therapy, or anti-HER2 antibody such as trastuzumab. In some embodiments, "chemotherapeutic agent" refers to an anti-tumor agent (also called anti-neoplastic agent) that has cytotoxic or cytostatic properties and does not act primarily by interacting with (e.g., interfering with) a hormonal pathway that is specific or relatively specific to particular cell type(s). Exemplary chemotherapeutic agents include anti-metabolites, alkylating agents, microtubule stabilizers or inhibitors (e.g., taxanes), topoisomerase inhibitors, and DNA intercalators (e.g., anthracycline antibiotics). Such agents are usually administered systemically. Often, multiple agents are administered. Exemplary regimens for breast cancer include CMF (cyclophosphamide, methotrexate, and 5-FU), AC (doxorubicin and cyclophosphamide), and anthracycline-based regimens. "Hormonal therapy" refers to administration of an anti-tumor agent that acts primarily by interacting with (e.g., interfering with) a hormonal pathway that is specific or relatively specific to particular cell type(s). "Adjuvant therapy" refers to administration of one or more anti-tumor agents in connection with, e.g., following, local therapy such as surgery and/or radiation. Adjuvant therapy may be used when the cancer appears to be largely or completely eradicated, but there is risk of recurrence. Such therapy may help eliminate residual cells at the site of the primary tumor and/or eliminate any cells that have disseminated. "Neoadjuvant therapy" refers to adjuvant therapy administered prior to local therapy, e.g., to shrink a primary tumor.

In some embodiments, a prognostic method of the invention may be used to identify cancer patients that do not require adjuvant therapy, e.g., adjuvant hormonal therapy and/or adjuvant chemotherapy (e.g., patients that would very likely not experience clinically evident metastasis in the absence of such treatment). Since such treatment can cause significant side effects, it would be beneficial to avoid administering it to individuals who would not benefit from it. In some embodiments, a prognostic method of the invention may be used to identify cancer patients that are at high risk of recurrence and/or metastasis and may therefore benefit from adjuvant therapy. In some embodiments, a prognostic method of the invention may be used to identify cancer patients that might not be considered high-risk based on other prognostic indicators (and may therefore not receive adjuvant therapy) but that are in fact at high risk of recurrence and/or metastasis. Results of an inventive prognostic assay may be useful for selecting a particular type of chemotherapy regimen and/or for selecting the type of procedures to be used to monitor the subject for metastatic recurrence after therapy and/or the frequency with which such procedures are performed. For example, subjects classified as being at high risk of metastasis may be assessed more frequently than those classified as being at low risk. Thus any of the prognostic methods can further comprise using the information obtained from the assay to help in selecting a treatment or monitoring regimen for a subject suffering from or at risk of cancer or in providing an estimate of the risk of poor outcome (e.g., cancer related mortality or recurrence) without adjuvant therapy. In some embodiments, any of the foregoing methods can comprise making a treatment recommendation or administering a treatment based at least in part on the result of the assay, e.g., based in part on the level of the miR-31 gene product. In some embodiments, any of the foregoing methods can comprise (i) recommending that the subject not receive chemotherapy (e.g., adjuvant chemotherapy) if the tumor is considered to have a good prognosis; or (ii) recommending that the subject receive chemotherapy (e.g., adjuvant chemotherapy), or administering such chemotherapy, if the tumor is considered to have a poor prognosis.

The miR-31-based methods may be used to provide prognostic information for subjects with tumors that have one or more recognized clinicopathologic features and/or fall into a particular class based on gene expression profiles. As known in the art, breast cancers can be classified based on a number of different clinicopathologic features such as histologic subtype (e.g., ductal; lobular; mixed), histologic grade (grade 1, 2, 3); estrogen receptor (ER) and/or progesterone receptor (PR) status (positive (+) or negative (-)), HER2 (ERBB2) expression status, and lymph node involvement. For example, the following breast cancer subtypes can be defined based on expression of estrogen receptor (ER) or progesterone receptor (PR) and human epidermal growth factor receptor 2 (Her2), e.g., as assessed by immunohistochemistry (IHC): ER+, Her2+; ER+, Her2-; ER-, Her2+; and ER-, Her2-. The level of expression can be used to further divide these subtypes. Amplification of the HER2 locus can be assessed, e.g., using fluorescent in situ hybridization (FISH). Breast cancers can also be classified into molecular subtypes based on gene expression profiles, e.g., luminal A, luminal B, ERBB2-associated, basal-like and normal-like (see, e.g., Sørlie, T., et al., 2001 and Desmedt, C., et al., 2008).

It has been reported that while about half of all breast cancers are assigned histologic grade 1 or 3 status (with a low or high risk of recurrence, respectively), a substantial percentage of tumors (30%-60%) are classified as histologic grade 2, which is less informative for clinical decision making because of intermediate risk of recurrence (Sotiriou C, et al., J Natl Cancer Inst., 98(4):262-72, 2006). Improved prognostic methods could be of significant use in this setting. In some embodiments, an inventive method is applied to a tumor classified as histologic grade 2, e.g., to classify histologic grade 2 tumors into high and low recurrence risk groups. In some embodiments, an inventive method is applied to a tumor classified as histologic grade 2, e.g., to classify histologic grade 2 tumors into high and low recurrence risk groups. In some embodiments, an inventive method is applied to a tumor that is ER+, while in other embodiments an inventive method is applied to a tumor that is ER-. In some embodiments an inventive method is applied to a tumor that is HER2 positive, while in other embodiments an inventive method is applied to a tumor that is HER2 negative. In some embodiments of particular interest, an inventive method is applied to a tumor that is ER-/HER2-. In other embodiments of particular interest an inventive method is applied to a tumor that is negative for expression of the estrogen and progesterone receptors and HER2 ("triple-negative"). In some embodiments a subject does not have lymph node involvement, i.e., the subject is "lymph node-negative" (LNN). In some embodiments a subject has Stage I or Stage II breast cancer. In some embodiments a subject has been treated with tamoxifen or another anti-estrogen agent, e.g., another selective estrogen receptor modulator such as raloxifene or toremifene. In some embodiments, a subject falls within a predefined age group or range, e.g., 50 years old or less, 60 years old or less, between 40 and 60 years of age, etc. Any age group or range may be selected, even if not specifically mentioned here.

In some embodiments, a miR-31-based prognostic assay is used together with additional information, such as results of a second assay (or multiple assays) and/or clinicopathological information. In some embodiments, such information comprises, e.g., subject age, tumor size, nodal involvement, tumor histologic grade, ER/PR status, and/or HER2 status, etc.) In some embodiments, information from a miR-31-based assay is used together with a decision making or risk assessment tool such as the computer program Adjuvant! Online (https://www.adjuvantonline.com/index.jsp). The basic format of an early version of Adjuvant! was described in the article Ravdin, Siminoff, Davis, et al. JCO 19(4) 980-991, 2001. In some embodiments, the second assay is a gene expression profiling assay such as the MammaPrint® (Agendia BV, Amsterdam, the Netherlands), Oncotype DX™ (Genomic Health, Redwood City, CA), Celera Metastasis Score™ (Celera, Inc., Rockville, MD), Breast BioClassifier (ARUP, Salt Lake City, UT), Rotterdam signature 76-gene panel (Erasmus University Cancer Center, Rotterdam, The Netherlands), or Invasiveness Gene Signature (OncoMed Pharmaceuticals, Redwood City, CA), or NuvoSelect™ assay (Nuvera Biosciences, Woburn, MA), that classifies tumors (e.g., into high or low risk groups) based on expression level of multiple genes using, e.g., a microarray or multiplex RT-PCR assay. The phrase "used together" with regard to two or more assays means that the two or more assays are applied to a particular tumor. For example, a miR-31-based assay may be used together with a gene expression profiling assay in which expression level of at least 10 different genes ("classifier genes") is used to classify a tumor. It will be understood that such assays typically measure expression of control genes as well as classifier genes. In some embodiments a miR-31-based assay is used together with an H:I™ test (AvariaDX, Carlsbad, CA), in which the ratio of expression of HOXB13 and IL-17B genes is used to classify a tumor. In some embodiments, a miR-31-based prognostic assay is used together with an antibody-based assay, e.g., the ProEx™ Br (TriPath Oncology, Durham, NC), Mammostrat® (Applied Genomics, Inc., Huntsville, AL), or a FISH-based test such as the eXaagenBC™ (eXagen Diagnostics, Inc., Albuquerque, NM). A miR-31-based assay may be used together with a second assay in a number of ways. For example, if results of the two tests are discordant (i.e., one test predicts that the subject is at high risk while the other predicts that the subject is at low risk), the subject may be treated. In some embodiments, one can have increased confidence if results are in agreement. For example, if both tests indicate that the subject is at low risk, there can be increased confidence in a decision not to administer adjuvant chemotherapy.

The disclosure provides kits comprising reagents suitable for performing a miR-31-based prognostic assay of the present invention. Such kits may contain, e.g., (i) a probe or primer (optionally labeled and/or attached to a support) for detecting, reverse transcribing, and/or amplifying miR-31 or a miR-31 precursor; (ii) a probe or primer for detecting, reverse transcribing, and/or amplifying an mRNA transcribed from a miR-31 target gene; (iii) an antibody for detecting a protein encoded by a miR-31 target gene; (iv) one or more controls (e.g., a probe or primer that does not detect or amplify miR-31). Optionally the kit comprises written material, e.g., instructions, e.g., in a paper or electronic format (e.g., on a computer-readable medium). Instructions may comprise directions for performing the assay and/or for interpreting results, e.g., in regard to tumor classification, likelihood of metastasis, and/or prognosis. Such material could also be provided online. In some embodiments, the invention provides a system which is adapted or programmed to carry out a miR-31-based assay. The system may include one or more instruments (e.g., a PCR machine) and/or computer processors. The system may be programmed with parameters that have been selected or optimized for detection and/or quantification of miR-31, e.g., in tumor samples. The system may be adapted to perform the assay on multiple samples in parallel and/or may have appropriate software to provide an interpretation of the result. The system can comprise appropriate input and output devices, e.g., keyboard, display, etc.

In some embodiments, a miR-31 based assay is performed at one or more central testing facilities, which may be specially qualified to perform the assay and, optionally, provide an interpretation of the results. In the latter case, samples are sent to the laboratory and a result of the assay is provided. The disclosure provides a method comprising: providing to a testing facility (a) a sample obtained from a subject to a testing facility; and (b) instructions to perform a miR-31-based assay of the invention (and, optionally, instructions to perform one or more additional assays such as an ICH, FISH, gene expression profile, or other assay described herein. The disclosure also provides a method comprising: (a) providing a sample obtained from a subject to a testing facility; and (b) receiving results of a prognostic assay of the invention. The invention further provides a method comprising providing, e.g., electronically, a result of a prognostic assay of the invention. In some embodiments, the result provided comprises a measurement made on the sample, with or without associated prognostic information. The invention contemplates that the assay may be performed at a testing facility which is remote from the site where the sample is obtained from a subject. It is also contemplated that samples and/or results may be transmitted through one or more different entities, which may carry out one or more steps of an inventive method or transmit or receive results thereof. All such activities are within the scope of the invention.

### III. Modulating Level or Activity or miR-31 or miR-31 Target Genes

The invention provides a variety of methods that, in certain embodiments, involve modulating the level or activity of miR-31 and/or one or more miR-31 target genes. Modulating can comprise causing or facilitating a qualitative or quantitative change, alteration, or modification (e.g., an increase or decrease) in the amount or activity of miR-31 and/or one or more miR-31 target genes in a cell or organism. A variety of methods for modulating miRNA level or activity are of use in the present invention. Cells can be contacted in vitro with molecules that are taken up and modulate miRNA expression or activity, or such molecules can be administered to a subject. miRNA, miRNA precursors, or synthetic oligonucleotides that resemble miRNA or miRNA precursors can be introduced into cells and result in increased inhibition of miRNA target gene expression. For example, Pre-miR™ miRNA Precursor Molecules (Ambion) are small, chemically modified double-stranded RNA molecules designed to mimic endogenous mature miRNAs. Nucleic acids that provide a template for transcription of miRNA or miRNA precursors can be introduced into cells and stably or transiently expressed therein. Vectors for expressing miRNA precursors are well known in the art and commercially available, e.g., from Open Biosystems. Such vectors may contain a site for insertion of a miRNA sequence of interest to create a template for transcription of a miRNA precursor, miRNA flanking sequences, and a promoter to direct transcription of the miRNA precursor sequence. See, e.g., Dickens NG et al., Nature Genetics, 37(11):1289-1295, 2005.

miRNA can be inhibited by a variety of approaches. In some embodiments, miR-31 is inhibited by introducing an oligonucleotide that is complementary to miR-31 or to a miR-31 precursor into a cell (e.g., in vitro) or administering such an oligonucleotide to a subject, e.g., a human. The oligonucleotide, sometimes termed an "antagomir" need not be perfectly complementary to miR-31 or miR-31 precursor, e.g., it may have 1, 2, 3, 4, 5, or more mismatches and/or be at least 70%, at least 80%, or at least 90% complementary thereto. In some embodiments the oligonucleotide is between about 17 nt and about 50 nt in length, e.g., about 19-25 nt long. See, e.g., Krutzfeldt J, et al. Nature 438: 685-9, 2005. In some embodiments, such oligonucleotides are expressed in cells (Scherr, M., et al., Nucleic Acids Res., 35(22): e149, 2007). In other embodiments a miRNA is inhibited using a miRNA sponge approach, which comprises expressing an RNA comprising multiplebinding sites for the particular miRNA in a cell, or introducing a nucleic acid comprising a plurality of binding sites for the particular miRNA into a cell (see, e.g., Ebert, M.S., et al. MicroRNA sponges: competitive inhibitors of small RNAs in mammalian cells. Nat Methods 4, 721-726, and Examples 4 and 5).

An oligonucleotide may contain one or more non-standard nucleotides, modified nucleotides (e.g., having modified bases and/or sugars) or nucleotide analogs, and/or have a modified backbone. In some embodiments an oligonucleotide is attached to one or more non-nucleic acid moieties. In some embodiments, the oligonucleotide has one or more modifications as compared with standard RNA or DNA, e.g., to provide at least partial protection from RNase and/or pharmacologic properties such as enhanced tissue and/or cellular uptake, increased half-life, etc. In some embodiments the oligonucleotide differs from standard RNA or DNA by having partial or complete 2'-O-methylation or 2'-O-methoxyethyl modification of sugar, phosphorothioate backbone, and/or a cholesterol-moiety at the 3'-end. In some embodiments, a nucleic acid comprising a morpholino oligonucleotide or a locked nucleic acid is used. US Patent Application Publications 20080171715, 20070213292 and PCT publications WO/2006/137941 WO/2008/025025 disclose a variety of compounds and methods of use to modulate miRNA. Certain agents that modulate miRNA level or activity are available from a variety of commercial suppliers (e.g., Thermo Scientific, Ambion).

In some embodiments a miRNA modulating agent is physically associated with a moiety that increases cell uptake, such as a cell-penetrating peptide. In some embodiments, a miR-31 modulating agent is physically associated with a targeting moiety, which moiety targets the agent to a cell type of interest. In some embodiments the targeting moiety binds to a molecule or portion thereof that is exposed at the surface of an epithelial cell, e.g., a breast epithelial cell. In some embodiments the targeting moiety binds to a molecule or portion thereof that is exposed at the surface of a cancer cell, e.g., a breast cancer cell. In some embodiments the targeting moiety binds to a tumor antigen. In some embodiments the targeting moiety binds to a receptor, e.g., a hormone receptor. A targeting moiety can be, e.g., an antibody, aptamer, peptide, sugar, small molecule, etc. Two or more moieties that are "physically associated" may be covalently or non-covalently bound to each other, either directly or via a third moiety. A variety of compositions and methods can be used to deliver agents to cells in vitro or in vivo. For example, agents can be incorporated into or attached to various types of particles such as liposomes, lipoplexes, or polymer-based particles, e.g., microparticles or nanoparticles composed at least in part of one or more biocompatible polymers or co-polymers comprising poly(lactide-glycolide), copolyoxalates, polycaprolactones, polyesteramides, polyorthoesters, polyhydroxybutyric acid, and/or polyanhydrides.

Expression of miR-31 target genes can be inhibited, e.g., using RNA silencing, also termed RNA interference (RNAi), which encompasses processes in which sequence-specific silencing of gene expression is effected by an RNA-induced silencing complex (RISC) that has a short RNA strand incorporated therein, which strand directs or "guides" sequence-specific degradation or translational repression of mRNA to which it has complementarity. The complementarity between the short RNA and mRNA need not be perfect (100%) but need only be sufficient to result in inhibition of gene expression. For example, the degree of complementarity and/or the characteristics of the structure formed by hybridization of the mRNA and the short RNA strand can be such that the strand can (i) guide cleavage of the mRNA in the RNA-induced silencing complex (RISC) and/or (ii) cause translational repression of the mRNA by RISC. The short RNA is often incorporated into RISC as part of a short double-stranded RNA (dsRNA). RNAi may be achieved artificially in mammalian cells in a variety of ways. For example, RNAi may be achieved by introducing an appropriate short double-stranded nucleic acid into the cells or expressing in the cells a nucleic acid that is processed intracellularly to yield such short dsRNA. The term "RNAi agent" encompasses nucleic acids that can be used to achieve RNA silencing in mammalian cells. Exemplary RNAi agents are a short hairpin RNA (shRNA), a short interfering RNA (siRNA), and a microRNA precursor. siRNAs typically comprise two separate nucleic acid strands that are hybridized to each other to form a duplex. They can be synthesized *in vitro,* e.g., using standard nucleic acid synthesis techniques. They can comprise a wide variety of modified nucleosides (e.g., comprising modified bases), modified backbones, etc. Any modification or analog recognized in the art as being useful for RNAi or other uses of oligonucleotides can be used. Some modifications result in increased stability, cell uptake, potency, etc. In certain embodiments the siRNA or shRNA comprises a duplex about 19 nucleotides in length, wherein one or both strands has a 3' overhang of 1-5 nucleotides in length (e.g., 2 nucleotides), which may be composed of deoxyribonucleotides. shRNA comprise a single nucleic acid strand that contains two complementary portions separated by a predominantly non-self-complementary region. The complementary portions hybridize to form a duplex structure and the non-self-complementary region forms a loop connecting the 3' end of one strand of the duplex and the 5' end of the other strand. shRNAs can undergo intracellular processing to generate siRNAs. In certain embodiments the term "RNAi agent" also encompasses vectors, e.g., expression vectors, that comprise templates for transcription of an siRNA (e.g., as two separate strands that can hybridize), shRNA, or microRNA precursor, and can be used to introduce such template into mammalian cells and result in transient or stable expression thereof. Other methods of inhibiting expression or activity of miR-31 target genes include, e.g., use of small molecules, antibodies, aptamers, dominant negative approaches, etc.

### IV. Drug Discovery and Characterization

The invention provides methods for identification, characterization, and/or evaluation of compounds, e.g., for treatment of cancer. Certain aspects of the invention relate to identifying compounds that selectively kill or inhibit proliferation of cells that have reduced or absent expression of miR-31 as compared, e.g., with their effect on cells that express miR-31, e.g., cells that do not have reduced or absent expression of miR-31. Such compounds may be of use as anti-tumor agents, e.g., to reduce the likelihood of metastasis and/or to inhibit the growth of metastases arising from cells having reduced or absent miR-31 expression.

Without wishing to be bound by theory, aberrantly reduced or absent expression or activity of a miRNA may result in increased vulnerability to certain compounds as a result of alterations in expression of one or more gene(s) that are normally regulated by the miRNA. Similarly, aberrantly increased expression or activity of a miRNA may result in increased vulnerability to certain compounds as a result of alterations in expression of one or more gene(s) that are normally regulated by the miRNA. Such vulnerabilities can be exploited to identify compounds that have selective activity towards cells that have altered miRNA expression. The invention encompasses applying this principle to discover compounds that have selective activity towards a cell that has altered, e.g., reduced, expression of a miRNA of interest, wherein the reduced expression confers on a cell an increased ability to cause or contribute to a disease or undesirable condition, e.g., cancer. For example, and without wishing to be bound by theory, reduced or absent expression of miR-31, in addition to conferring increased metastatic ability on a cell, may result in increased vulnerability to certain compounds as a result of alterations in expression of one or more gene(s) that are normally regulated by miR-31. Such vulnerabilities can be exploited to identify compounds that have selective activity towards cells that have reduced or absent miR-31 expression. The invention also encompasses applying this principle to discover compounds that have selective activity towards a cell that has increased expression of a miRNA of interest, wherein the increased expression confers on the cell an increased ability to cause or contribute to a disease or undesirable condition, e.g., cancer.

In one embodiment, the invention provides a method of identifying a potential anti-tumor compound comprising (a) providing a first cell that has reduced or absent miR-31 expression or activity as compared with a second cell; (b) contacting the first cell with a test compound; (c) identifying the test compound as a potential anti-tumor compound if the survival or proliferation of the first cell is reduced relative to a reference value. It will be understood that the first cell is often provided as a member of a population of cells, which may be substantially genetically identical. A first cell that has reduced or absent expression or activity of miR-31 as compared with a second cell (or can be induced to have reduced or absent miR-31 expression or activity) may be referred to herein as a "test cell", and the second cell may be referred to as a "control cell". In some embodiments expression or activity of miR-31 in control cells may be greater than that in the test cells by at least 1.2-fold, e.g., between about 1.5-fold and about 100-fold, 500-fold, 1000-fold or more. In some embodiments, miR-31 expression or activity in the test cell is reduced by at least 20%, 30%, 40%, 50%, 60%, 70%, 80%, 90%, 95%, 99% or more, as compared with a control cell. In some embodiments, the reference value is a value obtained using control cells that are exposed to the same compound (typically at the same or about the same concentration). In some embodiments, the cells that have reduced or absent miR-31 expression or activity are cancer cells (e.g., breast cancer cells), while the reference value is obtained using cells that are not cancer cells, e.g., normal breast epithelial cells). The invention further provides a method for testing the ability of a compound to inhibit the survival and/or proliferation of a cell, comprising (a) contacting one or more test cells with a compound, wherein the one or more test cells has reduced or absent miR-31 expression or activity as compared to a control cell, and (b) detecting the level of inhibition of the survival and/or proliferation of the one or more test cells by the compound. In various embodiments test and/or control cells can be primary cells, non-immortalized cell lines, immortalized cell lines, transformed immortalized cell lines, benign tumor-derived cells or cell lines, malignant tumor derived cells or cell lines (which may be derived from a primary tumor or from a metastasis), transgenic cell lines, etc. Optionally the method further comprises comparing the level of inhibition of the survival and/or proliferation of the one or more test cells by the compound with the level of inhibition of the survival and/or proliferation of one or more control cells by the compound, and may further comprise identifying a compound that has greater inhibitory effect on the test cell(s) than the control cell(s). Determining the level of inhibition of the survival and/or proliferation of one or more control cells by the compound can be done prior to, in parallel, or after determining the level of inhibition of the survival and/or proliferation of the one or more test cells.

Assays of biological activity of test compounds may be conducted in vitro or in vivo using cells identified or generated using any suitable method and in any suitable system for testing compound effect. In some embodiments, non-tumorigenic or tumorigenic cells that have been genetically modified or treated so that they have reduced or absent miR-31 expression or activity are used as test cells. For example, such cells could have a mutation or deletion in the miR-31 gene. Test cells in which other methods of reducing or eliminating miR-31 activity are used may be employed in the invention. For example, in some embodiments, test cells are non-tumorigenic or tumorigenic cells that have been genetically modified to stably express a nucleic acid comprising multiple miR-31 binding sites. Optionally, expression of such nucleic acid is under control of regulatable expression control element(s), e.g., an inducible promoter. In some embodiments cancer cells (primary cells or cell lines) that naturally (i.e., not due to deliberate genetic modification or other treatment) have reduced or absent expression of miR-31 are used as test cells. In some embodiments, the cancer cells have been experimentally transformed, e.g., rendered tumorigenic by engineering them to express one or more oncogenes and/or to have reduced or absent expression of one or more tumor suppressor genes are used. Cells that do not have reduced or absent miR-31 expression or activity may be used as control cells. The control cells may or may not be cancer cells. In some embodiments a control cell is derived from a cell, e.g., a cancer cell, that naturally has reduced or absent expression of miR-31, wherein the control cell is generated by introducing a vector that encodes a miR-31 precursor into the cell or otherwise genetically modifying the cell to express miR-31.

In some embodiments the test cells and control cells are genetically matched. "Genetically matched" includes cells or populations of cells that have largely identical genomes, e.g., their genomes are at least 95%, 98%, 99%, 99.5%, 99.9%, 99.99% identical, or more. If desired, a precise or approximate measurement of the level of identity could be established, e.g., by sequencing and comparing all or a significant portion (e.g., at least 1%, 5%, or 10%) of the genomes of the test and control cells. Typically, genetically matched cells are derived from the same subject or, in the case of certain species such as mice or rats, from different subjects belonging to a particular inbred strain. In some embodiments of the invention, genetically matched cells are derived from the same tissue sample. In some embodiments of the invention, test cells and control cells will have been derived from the same cell or the same initial population of genetically matched cells and will have undergone no more than 2, 5, 10, 15, 20, 25, 30, 35, 40, 50, 60, 70, 80, 90, or 100 rounds of cell division before being used in an inventive method. The invention provides genetically matched pairs of cell lines suitable for performing the inventive methods, wherein one cell line of the pair has reduced or absent miR-31 expression or activity (or can be induced to have reduced or absent miR-31 expression or activity) as compared with the other cell line. For example, in some embodiments either test cells or control cells have a specific genetic modification such as an engineered mutation in the miR-31 gene. Without wishing to be bound by any theory and without limiting the invention in any way, by using test and control cells that are genetically matched and differ primarily or essentially in that the test cells have reduced or absent miR-31 expression (or the equivalent) and the control cells have "normal" miR-31 expression, the invention allows identification of compounds that differentially affect the test cells versus the control cells (e.g., compounds that inhibit survival and/or proliferation of the test cells to a significantly greater extent than the extent to which they inhibit growth of the control cells) as a result of differences in the test cells and control cells that arise as a consequence of the difference in miR-31 expression level rather than because of other, possibly unknown, genetic or epigenetic differences in the test and control cells.

In some embodiments, test and control cells are breast cells, e.g., breast epithelial cells. Numerous breast cell lines are known and can be used in embodiments of the invention. Examples include 184A1, BT20, BT474, BT483, BT549, Hs578T, hTERT-HME1, MCF7, MCF10A, MDA-MB134, MDA-MB157, MDA-MB175, MDA-MB231, MDA-MB361, MDA-MB436, MDA-MB453, MDA-MB468, SKBR3, T47D, UACC812, UACC893, ZR75-1 and ZR75-30 (available from ATCC, Manassas, VA, USA). EFM19 and EFM192A (available from DSMZ, Braunschweig, Germany). HCC38, HCC70, HCC202, HCC712, HCC1007, HCC1143, HCC1395, HCC1419, HCC1428, HCC1500, HCC1569, HCC1599, HCC1806, HCC1937, HCC1954, HCC2157, HCC2185, HCC2218, HCC2688 and HCC3153 (available from the cell repository of the Hamon Center for Therapeutic Oncology Research, UT Southwestern Medical Center and/or from ATCC). SUM44PE, SUM52PE, SUM102PT, SUM149PT and SUM190PT (available from Asterand, Detroit, MI). Yet other cell lines are discussed in the Examples. In some embodiments, a cell line is experimentally generated, e.g., by introducing vectors expressing one or more immortalizing genes and/or oncogenes. For example, cells can be rendered tumorigenic by genetically engineering them to express hTERT, SV40 LT, and H-Ras.

One of skill in the art can select a tumorigenic or non-tumorigenic line as desired. One of skill in the art could also select a cell line based at least in part on characteristics that may be of interest, such as histologic subtype, molecular subtype, ER and/or PR receptor status, HER2 status, proliferation rate, etc. In other embodiments, test and control cells are non-breast cancer cells. Any type of cancer cell may be employed in various embodiments. In still other embodiments, test and control cells are not cancer cells.

In some embodiments, test cells and control cells are maintained in separate vessels (e.g., separate wells of a microwell plate) under substantially identical conditions to perform an assay. Assay systems comprising test cells, control cells, and one or more test compounds, e.g., 10, 100, 1000, 10,000, or more test compounds, wherein the cells and test compound(s) are arranged in one or more vessels in a manner suitable for assessing effect of the test compound(s) on the cells, are aspects of the invention. Typically the vessels contain a suitable tissue culture medium, and the test compounds are present in the tissue culture medium. One of skill in the art can select a medium appropriate for culturing a particular cell type. In some embodiments, a medium does not contain serum or tissue extracts, e.g., the medium is chemically defined, while in other embodiments such a component is present. In some embodiments a medium contains less than or equal to 0.01%, 0.05%, or 0.1% by weight or volume of serum or tissue extract. In some embodiments, cells are cultured on a plastic or glass surface. In some embodiments cells are cultured on or in a material comprising collagen, laminin, Matrigel®, or a synthetic material, e.g., a synthetic hydrogel, intended to provide an environment that resembles in at least some respects the extracellular environment found in many tissues. In some embodiments test and/or control cells are cultured with non-cancerous stromal cells. In some embodiments test and/or control cells are cultured with fibroblasts.

In some embodiments, an assay is performed ex vivo (e.g., in culture) is used to assess the effect of a test compound on cells. Such assays can measure, e.g., cell survival, proliferation, invasion, migration, apoptosis, anoikis, colony formation, etc. Suitable assays are known in the art. In some embodiments cells are cultured in a three-dimensional culture matrix or in soft agar. The test compound is typically added to the culture medium. In some embodiments, compounds are contacted with test cells (and optionally control cells) at a predetermined dose (e.g., predetermined concentration). In one embodiment the dose may be about up to 1 nM. In another embodiment the dose may be between about 1 nM and about 100 nM. In another embodiment the dose may be between about 100 nM and about 1 µM. In another embodiment the dose may be at or above 1 µM, e.g., up to about 10 µM or 100 µM. Following incubation for an appropriate time, optionally a predetermined time, the effect of compounds or composition on the growth and/or survival of the test cell is determined by an appropriate method known to one of ordinary skill in the art. Cells can be contacted with compounds for various periods of time. In certain embodiments cells are contacted for between 12 hours and 20 days, e.g., for between 1 and 10 days, for between 2 and 5 days, or any intervening range or particular value. Cells can be contacted transiently or continuously. If desired, a compound can be removed prior to assessing survival and/or proliferation (or other characteristics). As used herein, "suppress", "inhibit", or "reduce" and similar terms may, or may not, be complete. For example, cell proliferation may, or may not, be decreased to a state of complete arrest for an effect to be considered one of suppression, inhibition, or reduction of cell proliferation. In some embodiments, "suppress", "inhibit", or "reduce" comprises inhibiting the proliferation of a proliferating cell. In some embodiments, "suppress", "inhibit", or "reduce" may comprise maintaining an existing state. For example, inhibiting cell proliferation may refer to preventing proliferation of a non- proliferating cell (maintaining a non-proliferating state). Inhibiting cell survival may refer to killing a cell, or cells, such as by necrosis or apoptosis (e.g., anoikis), and the process of rendering a cell susceptible to death. The suppression, inhibition, or reduction may be at least about 5%, 10%, 15%, 20%, 25%, 30%, 35%, 40%, 45%, 50%, 55%, 60%, 65%, 70%, 75%, 80%, 85%, 90%, 95%, 99% of a reference level (e.g., a control level). In some embodiments, the level of modulation (e.g., suppression, inhibition, or reduction) compared with a control level is statistically significant. As used herein, "statistically significant" refers to a p-value of less than 0.05, e.g., a p-value of less than 0.025 or a p-value of less than 0.01, using an appropriate statistical test (e.g., an ANOVA, t-test, etc.). In certain embodiments, the survival and/or proliferation of the test cell and/or control cell is determined by an assay selected from: a cell counting assay, a cell membrane integrity assay, a cellular ATP-based viability assay, a mitochondrial reductase activity assay, a caspase activity assay, an Annexin V staining assay, a DNA content assay, a DNA degradation assay, and a nuclear fragmentation assay. Exemplary assays include BrdU, EdU, or H3-thymidine incorporation assays; DNA content assays using a nucleic acid dye, such as Hoechst Dye, DAPI, actinomycin D, 7- aminoactinomycin D or propidium iodide; cellular metabolism assays such as AlamarBlue, MTT, XTT, and CellTitre Glo®; cytoplasmic histone-associated DNA fragmentation assay; PARP cleavage assay; and a TUNEL assay.

In some embodiments, a mixture of cells that comprises at least two distinct populations of cells is used, wherein the populations differ with respect to expression or activity of miR-31. For example, in some embodiments cells of a first population have reduced or absent expression of miR-31 while cells of a second population do not. In some embodiments, a mixture of cells that comprises at least two distinct populations of cells is used, wherein the populations differ with respect to expression of one or more miR-31 target genes. In some embodiments, cells of a first population have increased expression of at least one (e.g., 2, 3, or more) miR-31 target gene relative to normal cells of the same cell type, while cells of a second population do not. Typically, each population of cells will have an identifying characteristic that is detectable and may be used to distinguish the cells from the other population(s) of cells in the mixture. In some embodiments, the identifying characteristic comprises a level of expression of a readily detectable protein. A readily detectable protein can be, e.g., a fluorescent protein (e.g., GFP) or an enzyme (e.g., luciferase, beta-glucuronidase) that acts on a substrate to render it detectable. Other identifying characteristics that enable detection and, optionally, quantification (e.g., by FACS or other suitable method) of each of the two or more populations of cells in the mixture can be used. Detectably labeled binding agents, e.g., antibodies, that bind to an epitope expressed on the surface of the cells, can be used. Such agents could be labeled with a radioactive moiety, metal atom or cluster, magnetic moiety, isotope, fluorescent moiety, quantum dot, etc. Compositions can comprise, e.g., between 1% and 99% cells of a first population and at least some cells of a second population (e.g., between 1% and 99%), are an aspect of the invention. In some embodiments the percentage of cells of the first population is between 10% and 90%, between 20% and 80%, between 30% and 70%, between 40% and 60%, e.g., about 50%. In some embodiments the two populations are cultured together in the presence of a test compound. The culture is assessed at various time points to determine whether the proportion of cells that have reduced or absent expression of miR-31 (or one or more miR-31 target genes) changes over time. If the proportion of cells that have reduced or absent expression of miR-31 decreases over time to a greater extent than in the absence of the test compound, the test compound may be selectively toxic to cells that have reduced or absent expression of miR-31.

In some embodiments of the methods, populations of test and/or control cells are contacted with different doses of a compound. A dose response curve can be generated, wherein the test dose response curve indicates the level of inhibition of the survival, proliferation (or any other relevant characteristic, e.g., metastasis-relevant characteristic) of test cells by the compound at a plurality of doses, and the control dose response curve indicates the level of inhibition of the survival, proliferation (or any other relevant characteristic) of control cells by the compound at a plurality of doses. In some embodiments, the method comprises determining an IC50 or EC50 value for a compound on test cells and/or control cells. In some embodiments, if the IC50 or EC50 value for the compound on test cells is statistically significantly less than the IC50 or EC50 value for the compound on control cells, the compound is identified as being selective for cells that have reduced or absent expression of miR-31. In some embodiments, if the IC50 or EC50 value for the compound on test cells is statistically significantly less than the IC50 or EC50 value for the compound on control cells, the compound is identified as being selective for metastatic cells and/or for cells that have a propensity to metastasize.

Cells that have reduced or absent expression or activity of miR-31 can also be used in methods that do not involve comparison of the effect of the compound on control cells that do not have reduced or absent expression of miR-31. For example, the invention provides a method of identifying a potential anti-tumor compound comprising (a) providing a first cell that has reduced or absent expression of miR-31 as compared with a normal cell; (b) contacting the first cell with a test compound; (c) identifying the test compound as a potential anti-tumor compound if the survival or proliferation of the first cell is reduced relative to a reference value. In some embodiments the reference value is a value obtained using cells that are not contacted with the compound.

In some embodiments, a test compound is tested in vivo, by administering the compound to a subject, e.g., a non-human subject. In some embodiments, the test compound may have first been identified using an in vitro assay such as those described above. In some embodiments the test compound is administered to a nonhuman subject to which a plurality of cancer cells has been administered (e.g., by implantation or injection), e.g., a number of cancer cells sufficient to induce the formation of one or more tumors, a tumor xenograft, etc. In some embodiments the cancer cells are of human origin, e.g., human breast cancer cells. In some embodiments, cancer cells are introduced into the circulation, e.g., by injection, and the size and/or number of tumor growths, e.g., in the lungs, liver, bone, etc. is monitored. In some embodiments, cancer cells are introduced locally, e.g., into an organ, e.g., orthotopically. In some embodiments cancer cells are introduced subcutanoeusly. In some embodiments, cancer cells are introduced into a mammary gland, e.g., a mammary fat pad. The cells can be introduced together with substances such as Matrigel™, collagen, or other materials, e.g., that mimic the stromal environment or extracellular matrix. In some embodiments, cancer cells are introduced together with non-cancerous cells, e.g., non-cancerous fibroblasts, e.g., non-cancerous mammary fibroblasts. The cell lines can be genetically modified to express a readily detectable protein, e.g., to facilitate detection and/or measurement of metastases. The nonhuman subject can be, e.g., a rodent such as a mouse or rat). Optionally the nonhuman subject is immunocompromised, e.g., a Nude, SCID, NOD-SCID, Ragl-/-, Rag2-/-, and/or athymic mouse or one that has been immunosuppressed through administration of an immunosuppressive drug. In some embodiments the test subject is a cancer-prone animal, e.g., an animal model harboring an activated oncogene and/or lacking a tumor suppressor gene, or an animal that has been exposed to a condition, compound, or stimulus that renders the animal prone to develop cancer, i.e., the animal has an increased likelihood of developing cancer relative to animals that are otherwise substantially identical but do not harbor the activated oncogene, do not lack the tumor suppressor gene, or have not been exposed to the condition, compound, or stimulus, respectively. Optionally a hormone, e.g., estrogen or an estrogen receptor agonist, is administered to the test subject. Any animal model for cancer known in the art can be used. Breast cancer models are of particular interest. Such models include, e.g., rodents, e.g., mice or rats, engineered to express mutant or wild type ERBB-2, TGF alpha, Wnt-1, c-Myc, IGF-I, IGF-II, mutant p53, SV-40 T antigen, and/or cyclin D, optionally under control of tissue-specific regulatory element(s). In some embodiments, an animal model such as those described in USSN 10/990,993 or in PCT/US2008/085040 (Pub. No. WO/2009/070767) is used. In some embodiments an animal model expresses a readily detectable protein in cells of the type from which the tumor is expected to arise, e.g., breast epithelial cells. Such protein may facilitate detection and/or measurement of metastases, e.g., using suitable imaging methods.

The test compound can be administered to the subject by any regimen known in the art. For example, the test compound can be administered prior to, concomitant with, and/or following the administration of cancer cells or exposure to a condition that promotes development of cancer. A test compound can also be administered regularly for example, 1, 2, 3, or more times a day, weekly, bi-weekly, or monthly, beginning before or after cancer cells have been administered. In other embodiments, the test compound is administered continuously to the subject (e.g., by release from an implant, pump, sustained release formulation, etc.). The dose of the test compound to be administered can depend on multiple factors, including the identity of the compound, weight of the subject, frequency of administration, etc. Determination of dosages is routine for one of ordinary skill in the art. Exemplary doses in certain embodiments are 0.1 µg to 10,000 mg, e.g., about 1 µg to 5000 mg, e.g., from about 10 µg to 100 mg once or more per day, week, month, or other time interval. In terms of body weight, exemplary dosages in certain embodiments are 0.01-200 mg/kg/day (e.g., 0.1-20 or 1-10 mg/kg/day, e.g., from about 1 to 5 mg/kg/day).

The size and/or number of primary tumors and/or metastases in the subject, and/or cell properties or processes such as invasiveness, motility, extravasation, survival at a distant site (relative to the site of introduction) etc., can be determined following administration of the tumor cells and the test compound. Such features can be determined non-invasively by any means known in the art. For example, tumor cells that are labeled (e.g., by expressing a fluorescent protein) can be monitored by various imaging techniques or instruments. Useful imaging methods include nuclear imaging, optical imaging (e.g., near-infrared (NIR) optical imaging), positron emission tomography (PET) (optionally using (18)F-fluorodeoxyglucose (FDG)), and magnetic resonance imaging (MRI). Labeled imaging agents can be administered, which may be physically associated with a targeting moiety that targets them selectively to introduced cells or their progeny. To determine whether a compound affects tumor size and/or metastasis, the size and/or number of tumors in the subject (or any cell property or process of interest) can be compared to a reference standard (e.g., a control value). A reference standard can be obtained using a control subject that has been given the same regimen of administration of cancer cells and, optionally, a mock compound administration. The mock administration can lack a test compound or contain a compound that is considered inactive. A reference standard can be a numerical value, values, or range of values representing the size and/or number of tumors or metastases (or any cell property or process of interest) expected in an untreated subject, which can be determined by observation of a representative sample of untreated subjects.

Any compound or combination thereof can be tested in the inventive methods. Compounds can be small organic molecules, inorganic molecules, nucleic acids, polypeptides, antibodies etc. In some embodiments, nucleic acids or polypeptides to be evaluated are expressed in a cell using recombinant nucleic acid technology, e.g., by introducing a vector encoding the nucleic acid or polypeptide into the cell. For example, cDNA libraries encoding a variety of proteins (which may have a naturally occurring or artificial sequence) may be evaluated. Compounds can be members of, e.g., chemical libraries, natural product libraries, combinatorial libraries, etc. Chemical libraries can comprise diverse chemical structures, some of which may be known compounds, analogs of known compounds, or analogs or compounds that have been identified as "hits" or "leads" in other drug discovery screens, while others are derived from natural products, and still others arise from non-directed synthetic organic chemistry. Compounds from such libraries are often arrayed in multi-well plates (e.g., 96, 384, or 1536 -well plates). They may be dissolved in a suitable solvent, e.g., DMSO. Natural product libraries can be prepared, e.g., from collections of microorganisms, animals, plants, or marine organisms which are used to create mixtures for screening by, e.g., (1) fermentation and extraction of broths from microorganisms, or (2) extraction of plants or marine organisms. Compound libraries are commercially available from a number of companies such as Sigma-Aldrich, TimTec (Newark, DE), and ChemBridge Corporation (San Diego, CA). Various government and non-profit research institutions have compound libraries that are available to the scientific community. For example, the Molecular Libraries Small Molecule Repository (MLSMR), a component of the National Institutes of Health (NIH) Molecular Libraries Program is designed to identify, acquire, maintain, and distribute a collection of >300,000 chemically diverse compounds with known and unknown biological activities for use, e.g., in high-throughput screening (HTS) assays (see https://mli.nih.gov/mli/). The NIH Clinical Collection (NCC) is a plated array of approximately 450 small molecules that have a history of use in human clinical trials. These compounds are highly drug-like with known safety profiles. In some embodiments the drug is one that has been approved by a government regulatory agency such as the US Food and Drug Administration or a compound that is in preclinical or clinical development, or under consideration for development, as a therapeutic agent. In some embodiments the drug has been approved for use in cancer, e.g., breast cancer, while in other embodiments it has not. The compound is typically one that is not present in standard cell culture medium, or if present is present in lower amounts than when used in the present invention.

Applicants reserve the right to exclude any particular compound, compounds, or compound class from the scope of "test compound" and/or from the compositions and methods of the invention. In some embodiments the "test compound" is not a compound found in, or known in the art as an ingredient of, tissue culture medium, e.g., a compound provided for purposes of culturing the cells. In some embodiments the test compound may be one found in, or known in the art as an ingredient of, tissue culture medium, but is used as a test compound at concentrations differing from those at which it is typically used as an ingredient of tissue culture medium. In some embodiments the compound is not a compound known in the art as being useful for treating cancer and/or for reducing side effects associated with chemotherapy.

Certain results of the compound identification and characterization methods disclosed herein may be of therapeutic interest, such as if the compound is a suppressor of metastasis (e.g., a suppressor of one or more steps in the invasion-metastasis cascade) and/or if the compound inhibits survival or proliferation of cells that have a propensity to metastasize. Other beneficial results may include: (a) ability, through administration of an agent identified according to the invention, to reduce the dose or duration of chemotherapy (or to avoid chemotherapy all together) without compromising outcome; (b) ability, through administration of an agent identified according to the invention, to use a chemotherapeutic agent that has reduced toxicity as compared with other agents, without compromising outcome; and (c) extension of survival of a test subject. Compounds with clinically beneficial results are potential chemotherapeutics, and may be formulated as such.

Compounds identified as having a useful effect can be selected and systematically altered, e.g., using rational design, to optimize binding affinity, avidity, specificity, or other parameters. For example, one can screen a first library of compounds using the methods described herein, identify one or more compounds that are "hits" or "leads" (by virtue of, for example, their ability to inhibit metastasis), and subject those hits to systematic structural alteration to create a second library of compounds structurally related to the hit or lead. The second library can then be screened using the methods described herein or other methods known in the art. A compound can be modified or selected to achieve (i) improved potency, (ii) decreased toxicity and/or decreased side effects; (iii) modified onset of therapeutic action and/or duration of effect; and/or (iv) modified pharmacokinetic parameters (absorption, distribution, metabolism and/or excretion).

In certain embodiments of the invention a compound identified or developed using the inventive methods displays selective activity (e.g., selective inhibition of survival and/or proliferation, selective toxicity) against test cells relative to its activity against control cells. For example, the IC50 of a compound may be about 2, 5, 10, 20, 50, 100, 250, 500, or 1000-fold lower for test cells versus control cells. In some embodiments, the IC50 of a compound may be about 2, 5, 10, 20, 50, 100, 250, 500, or 1000-fold lower for cells that have reduced or absent expression of miR-31 than for genetically matched cells that express miR-31 (e.g., at normal levels for cells of that type). In some embodiments, the IC50 of a compound may be about 2, 5, 10, 20, 50, 100, 250, 500, or 1000-fold lower for cancer cells than for non-cancer cells. In some embodiments, the IC50 of a compound may be about 2, 5, 10, 20, 50, 100, 250, 500, or 1000-fold lower for cancer cells that lack miR-31 expression than for non-cancer cells that lack miR-31 expression.

The invention encompasses identifying biological target(s) of compounds that are identified using the inventive methods, e.g., the compounds that selectively inhibit survival or proliferation of cancer cells that lack expression of miR-31. The term "biological target" is used herein consistent with its meaning in the art to refer to a biomolecule (e.g., a biomolecule present in the body of a subject) on which a biologically active agent exerts an effect, e.g., an effect leading to a therapeutic benefit such as inhibiting survival, proliferation, and/or metastasis of cancer cells. For example, a compound may act directly (by physical interaction) or indirectly on one or more cellular gene products. A biological target may be, e.g., cell surface or intracellular receptor, enzyme, channel, secreted protein, etc. Biological targets of compounds that are identified using the methods of the invention are candidates for further drug discovery efforts (such candidates are sometimes referred to as "drug discovery targets"). The invention thus provides methods for identifying candidate biomolecules towards which drug discovery efforts may be usefully directed. Compounds that are already known in the art to act on such biomolecules may be useful as agents that have selective activity against cancer cells that have reduced or absent miR-31 expression. Such agents may be tested using the methods described herein. Furthermore, standard screening methods known in the art can be used to identify additional compounds such as small molecules, proteins, aptamers, antibodies, nucleic acids (e.g., siRNAs), etc., that act on such biomolecules. A variety of approaches can be used to identify one or more biological target(s) of a compound of interest. In some embodiments, an RNAi library (e.g., a shRNA or siRNA library) or genetic suppressor element (GSE) library is used to inhibit individual genes in cells, e.g., cells that have reduced or absent expression of miR-31 and are susceptible to inhibition by the compound. In one embodiment, a cDNA library is used to overexpress each gene in cells, e.g., cancer cells that have reduced or absent expression of miR-31 and are susceptible to inhibition by the compound. If inhibition or overexpression of a particular gene modulates (e.g., abrogates, reduces, or increases, in various embodiments of the invention) sensitivity of the cell to the compound, the gene or product of such gene is a candidate for being a target of the compound and/or a target for drug discovery. One of skill in the art will select an appropriate assay or combination of assays.

In other embodiments, the compound is used to identify its molecular target. Any suitable method can be used for target identification. The compound may be labeled or modified to include a reactive group or tag. The reactive group or tag may be incorporated, e.g., at a site where such incorporation does not substantially affect activity of the compound. Cells are contacted with the compound and maintained under conditions wherein the compound interacts with its target(s). The cells may, but need not be, test cells, e.g., cells that have reduced or absent miR-31 expression or activity. They may, but need not be, of the same type as those used to initially identify the compound. Optionally the compound is crosslinked to the biomolecule(s) with which it physically interacts. The compound is then isolated (e.g., removed from some or all other cellular constituents; partially purified) together with the biomolecule(s) to which it is bound. If the compound is tagged, the tag may be used to isolate the compound. In another embodiment, affinity chromatography is used to identify the biological target(s) of a compound. The compound is attached to a support, e.g., chromatography resin or other support, thereby forming an affinity matrix. The affinity matrix is incubated with cell lysates (e.g., test cell lysate, control cell lysate) and then washed, and bound biomolecules, e.g., proteins, eluted, e.g., by boiling in SDS-containing buffer. The proteins retained by the affinity matrices may be separated, e.g., by SDS/PAGE and visualized, e.g., by silver staining or any appropriate method. They may then be isolated from the gel. The biomolecules are then identified, e.g., using peptide sequencing, mass spectrometry, etc. In some embodiments, biomolecules that are present selectively in test cells and/or that selectively bind to the compound in test cells versus control cells are of interest. In other embodiments, microarray analysis, serial analysis of gene expression (SAGE), or similar techniques is/are used to assess the effect of a compound identified using the inventive methods on gene expression in cells of a selected type, e.g., test cells, control cells, cancer cells, etc. Genes whose expression is modulated as a result of exposure to the compound are candidates for being targets of the compound and/or targets for drug discovery. In other embodiments of the invention, methods known in the art are used to determine the effect of a compound on protein levels, localization, and/or modification state (e.g., phosphorylation state), etc. Proteins whose levels, localization, and/or modification state are modulated as a result of exposure to the compound are candidates for being targets of the compound and/or targets for drug discovery. Compounds identified using the inventive methods are therefore useful as probes to identify genes and gene products that play significant roles in cancer biology, e.g., in metastasis and/or in the survival or proliferation of cells that have a propensity to metastasize. Such genes and gene products are useful, without limitation, as targets for drug discovery for purposes of identifying additional agents that selectively inhibit growth and/or proliferation of metastatic cells and/or cells with a propensity to metastasize.

In other embodiments, a screen is performed to identify genes whose inhibition results in altered, e.g., decreased, survival or proliferation of cells, e.g., cancer cells, that have reduced or absent miR-31 expression (or results in alteration in any other relevant characteristic, e.g., metastasis-relevant characteristic such as invasion ability), e.g., as compared with cells (e.g., genetically matched cells) that do not have reduced or absent miR-31 expression. The screen may be performed using, e.g., an siRNA or shRNA library. Such genes, e.g., genes that exhibit significant reduction in viability, e.g., "synthetic lethality", with miR-31 (i.e., loss of both miR-31 and the identified gene results in greatly reduced viability) are also targets for drug discovery.

As noted above, the methods are applicable to other miRNAs whose aberrant expression contributes to disease, e.g., cancer. For example, the invention provides a method of identifying a potential anti-tumor compound comprising (a) providing a first cell that has reduced or absent expression or activity of a miRNA as compared with a second cell, wherein reduced or absent expression or activity of the miRNA contributes to tumor development, progression, or metastasis; (b) contacting the first cell with a test compound; (c) identifying the test compound as a potential anti-tumor compound if the survival or proliferation of the first cell is reduced relative to a reference value. The reference value may be, e.g., a value obtained using cells that do not have reduced or absent expression or activity of the miRNA, e.g., non-tumor cells. In another embodiment, the invention provides a method of identifying a potential anti-tumor compound comprising (a) providing a first cell that has increased expression of a miRNA as compared with a second cell, wherein increased expression or activity of the miRNA contributes to tumor development, progression, or metastasis; (b) contacting the first cell with a test compound; (c) identifying the test compound as a potential anti-tumor compound if the survival or proliferation of the first cell is reduced relative to a reference value. The reference value may be, e.g., a value obtained using cells that do not have increased expression or activity of the miRNA, e.g., non-tumor cells.

### V. Cell-based Models, Animal Models, and Uses Thereof

As discussed above, the invention provides cells, e.g., cancer cells, that are genetically modified to have altered (e.g., increased or decreased) miR-31 expression and/or activity. Such cells (e.g., any of the cells described in Section IV and/or in the Examples) can be used for a variety of purposes in addition to or instead of drug discovery and/or characterization. For example, such cells can be used in *in vitro* or *in vivo* preclinical toxicology studies, e.g., to test whether a potential therapeutic agent (e.g., for a disease other than cancer) promotes metastases. *In vivo* studies will typically involve introducing the cells into a non-human animal. Such cells can be used in *in vitro* or *in vivo* toxicology studies of agents such as food ingredients or additives, pesticides, or any agent with which humans may come into contact, e.g., to test whether such agent promotes (or inhibits) metastases. Such cells can also be used to assess the effect of conditions or treatments such as radiation, diet, or any parameter of interest, on metastasis. In some embodiments, such cells are used to assess the effect of immune system status and/or immune system modulating agents (e.g., "cancer vaccines") on metastasis. In other aspects, such cells can be used in research aimed at gaining further understanding of the events involved in metastasis. For example, such cells can be used in *in vitro* or *in vivo* models to study cell invasion, motility, etc.

The invention further provides genetically engineered non-human animals in which at least one allele of the miR-31 gene is disabled or deleted, or in which miR-31 activity is stably inhibited by at least 20%, in at least some cells of the animal, e.g., in all or substantially all cells of the animal. For purposes of the invention, such animals are termed "miR-31-deficient animals". In some embodiments, a miR-31 gene is considered "disabled" if it is altered in such a way as to reduce miR-31 activity by at least 20%, e.g., by reducing expression of miR-31 and/or by altering the miR-31 sequence. In some embodiments, a disabled miR-31 gene results in reduction of miR-31 activity by at least 30%, 40%, 50%, 60%, 70%, 80%, 90%, 95%, or more. In some embodiments, miR-31 activity is reduced by a factor of between about 1.5 and about 20. In some embodiments, both alleles of the miR-31 gene are disabled or deleted in at least some cells of the animal, e.g., in all or substantially all cells of the animal. In some embodiments, miR-31 activity is stably inhibited by at least 30%, 40%, 50%, 60%, 70%, 80%, 90%, 95%, or more. In some embodiments, miR-31 activity is reduced by a factor of between about 1.5 and about 20. In some embodiments, the miR-31 gene is disabled or deleted, or miR-31 activity is stably inhibited, in breast cells, e.g., breast epithelial cells. In some embodiments, at least some (e.g., all or substantially all) cells of the animal contain a transgene that encodes a nucleic acid comprising one or more miR-31 binding sites, resulting in reduced miR-31 inhibition of miR-31 target genes. In some embodiments, the transgene comprises regulatable expression control element(s), e.g., a regulatable promoter, e.g., an inducible promoter. In some embodiments the transgene comprises tissue-specific expression control element(s), e.g., a promoter that is active primarily or exclusively in a subset of tissues, e.g., breast epithelial cells. In some embodiments, the promoter is an MMTV promoter or whey acidic protein (WAP) promoter. In some embodiments, the deletion or alteration can be induced at a post-embryonic or post-natal stage of development and/or in certain tissues or cell types. For example, a miR-31 gene could be replaced by a version in which at least part of the gene is flanked by sites for a recombinase, which may be expressed or induced in specific cells or tissues and/or at specific stages of development. For example, the Cre-Lox of Frt-Flp systems can be used. See, e.g., Wang, X., Cre transgenic mouse lines. Methods Mol Biol., 561:265-73, 2009. In some embodiments, a miR-31-deficient animal has one or more naturally occurring or genetically engineered mutations or modifications that increase the likelihood that the animal will develop a tumor, e.g., breast cancer. The invention contemplates deleting or disabling miR-31 in any animal model for breast cancer known in the art. Such animals are an aspect of the invention. They may be obtained, e.g., by breeding a miR-31 deficient animal, e.g., a miR-31 deficient animal that is not known to be cancer-prone, to a cancer-prone animal such as those mentioned above, or by genetic modification of such animal. In some embodiments, the animal is heterozygous for the disabled or deleted miR-31 gene and/or for the genetic alteration that renders the animal cancer prone. In some embodiments, the animal is homozygous for the disabled or deleted miR-31 gene and/or for the genetic alteration that renders the animal cancer prone. The invention contemplates deleting or disabling miR-31 in any animal model for breast cancer known in the art. In some embodiments, the non-human animal exhibits an increased propensity for tumor metastasis as compared with control animals, e.g., otherwise genetically identical animals that are not genetically engineered to have reduced or absent miR-31 expression or activity. For example, the animal develops more metastases, on average, and/or develops them with a decreased latency, as compared with control animals.

The non-human animals may be used, e.g., as animal models to test or characterize anti-neoplastic compounds, tumor diagnostic modalities, e.g., imaging techniques and reagents, etc. The non-human animals may be used for *in vivo* preclinical toxicology studies, e.g., to test whether a potential therapeutic agent (e.g., for a disease other than cancer) promotes metastases. Such animal can be used in *in vivo* toxicology studies of agents such as food ingredients or additives, pesticides, or any agent with which humans may come into contact, e.g., to test whether such agent promotes (or inhibits) metastases and/or to assess the effect of conditions or treatments such as radiation, diet, or any parameter of interest, on metastasis. In some embodiments, such animal are used to assess the effect of immune system status and/or immune system modulating agents (e.g., "cancer vaccines") on metastasis. In other aspects, such animals can be used in research aimed at gaining further understanding of the events involved in metastasis. In some embodiments, compounds are tested to determine whether they promote metastases. For example, test compounds may be administered to the animals and the incidence of metastasis determined, e.g., using in vivo imaging, e.g., bioluminescent imaging. Methods of testing or characterizing compounds, e.g. with regard to their effect on metastasis, using miR-31 deficient non-human animals are aspects of the invention. Methods of testing or characterizing tumor diagnostic modalities, e.g., with respect to their ability to detect and/or quantify metastases, are aspects of the invention.

### VI. Therapeutic Applications

The invention provides methods of treating a subject suffering from cancer or at risk of cancer or cancer recurrence. In some embodiments, the cancer is breast cancer. A subject at risk of cancer may be, e.g., a subject who has not been diagnosed with cancer but has an increased risk of developing cancer as compared with an age-matched control of the same sex. For example, the subject may have a risk at least 1.2 times that of an age and sex matched control. Determining whether a subject is considered "at risk" of cancer may be within the discretion of the skilled practitioner caring for the subject. Any suitable diagnostic test(s) and/or criteria can be used. For example, a subject may be considered "at risk" of developing cancer if (i) the subject has a mutation, genetic polymorphism, gene or protein expression profile, and/or presence of particular substances in the blood, associated with increased risk of developing or having cancer relative to other members of the general population not having such mutation or genetic polymorphism; (ii) the subject has one or more risk factors such as having a family history of cancer, having been exposed to a carcinogen or tumor-promoting agent or condition, e.g., asbestos, tobacco smoke, aflatoxin, radiation, chronic infection/inflammation, etc., advanced age; (iii) the subject has one or more symptoms of cancer, etc. As used herein, treatment or treating can include amelioration, cure, and/or maintenance of a cure (i.e., the prevention or delay of recurrence) of cancer. Treatment after a disorder has started aims to reduce, ameliorate or altogether eliminate the disorder, and/or at least some of its associated symptoms, to prevent it from becoming more severe, to slow the rate of progression, or to prevent the disorder from recurring once it has been initially eliminated. Treatment can be prophylactic, e.g., administered to a subject that has not been diagnosed with cancer, e.g., a subject with a significant risk of developing cancer. A subject at risk of cancer recurrence has been diagnosed with cancer and has been treated such that the cancer appears to be largely or completely eradicated. In some embodiments, a therapeutic method of the invention comprises providing a subject in need of treatment for cancer, e.g., breast cancer. In some embodiments, a therapeutic method of the invention comprises diagnosing a subject in need of treatment for cancer, e.g., breast cancer.

In some aspects the invention provides methods of inhibiting metastasis (e.g., reducing the likelihood that a subject will develop evident metastasis (e.g., within a defined time period such as 5 or 10 years following diagnosis) and/or slowing the growth and/or reducing the average number of metastases). The breast cancer may have any of the clinicopathological and/or molecular characteristics described above in various embodiments. In some embodiments, the methods comprise administering a compound identified as described in Section IV hereof to a subject suffering from or at risk of cancer, e.g., breast cancer. In some embodiments the methods comprise functionally increasing miR-31 activity or miR-31-like activity in at least some cells of interest. In some embodiments, the cells of interest comprise cells, e.g., cancer cells, that have reduced or absent expression of miR-31 as compared with normal cells of that cell type (e.g., as compared with cells from which the cancer cells arose). In some embodiments, cells that have reduced or absent expression of miR-31 comprise breast cancer epithelial cells. (It will be understood that the methods may also result in increased miR-31 activity or miR-31-like activity in at least some cells that are not cells of interest.) "miR-31-like activity" refers to ability to suppress (inhibit, reduce) expression or activity of at least some miR-31 target genes, or proteins encoded thereby, e.g., at least 2, 3, 4, or more miR-31 target genes or proteins encoded thereby. In some embodiments, the miR-31 target genes include at least two of the following genes: integrin-α5 (ITGA5), radixin (RDX), and RhoA. In some embodiments, the miR-31 target genes include all three of these genes. In some embodiments the methods comprise increasing the level of miR-31 in at least some cells of interest. For example, a nucleic acid having the sequence of miR-31 or a miR-31 precursor (e.g., pri-miR-31 or pre-miR-31) can be administered. In some embodiments, a vector that encodes a miR-31 precursor is administered systemically or locally. For example, expression of miR-31 may be re-established using a viral vector such as an AAV vector (see, e.g., Kota, J., et al. Cell., 137(6):1005-17, 2009).

One aspect of the invention is the recognition that suppression of three genes, i.e., ITGA5, RDX, and RhoA can account for the anti-metastatic activity of miR-31 in breast cancer cells. WO2005/113770 suggests the use of siRNAs for inhibiting RhoA for use in treating tumor metastasis. Without wishing to be bound by theory, concurrent inhibition/antagonism of at least two of these genes or their encoded proteins may be of particular benefit to inhibit metastasis. The disclosure thus describes methods of treating a subject suffering from or at risk of cancer, e.g., breast cancer, comprising inhibiting expression or activity of least two miR-31 target genes or their encoded proteins, wherein the genes are selected from ITGA5, RDX, and RhoA. In some embodiments the method comprises inhibiting expression or activity of all three of these genes or their encoded proteins. Any suitable method may be used to inhibit ITGA5, RDX, and/or RhoA, e.g., RNA silencing-based approaches, small molecules, antibodies, dominant negative approaches, etc. For example, RhoA may be inhibited using a small molecule (e.g., a statin), RDX may be inhibited using an siRNA, ITGA5 may be inhibited using a monoclonal antibody or small molecule such as JSM6427.

The invention provides pharmaceutical compositions, comprising a compound identified as described in Section IV hereof. The invention further provides pharmaceutical compositions comprising a compound that inhibits expression or activity of a miR-31 target gene. In some embodiments, a pharmaceutical composition comprises a first compound that inhibits expression or activity of a first miR-31 target gene or a protein encoded thereby and a second compound that inhibits expression or activity of a second miR-31 target gene or a protein encoded thereby. A pharmaceutical composition may comprise a variety of pharmaceutically acceptable carriers.

The compound may be delivered in an effective amount, by which is meant an amount sufficient to achieve a biological response of interest, e.g., reducing gene expression or protein activity by a certain amount, reducing one or more symptoms or manifestations of a disease or condition, e.g., reducing the likelihood of metastasis. Pharmaceutically acceptable carriers are well known in the art and include, for example, aqueous solutions such as water, 5% dextrose, or physiologically buffered saline or other solvents or vehicles such as glycols, glycerol, oils such as olive oil or injectable organic esters that are suitable for administration to a human or non-human subject. In some embodiments, a pharmaceutically acceptable carrier or composition is sterile. A pharmaceutical composition can comprise, in addition to the active agent, physiologically acceptable compounds that act, for example, as bulking agents, fillers, solubilizers, stabilizers, osmotic agents, uptake enhancers, etc. Physiologically acceptable compounds include, for example, carbohydrates, such as glucose, sucrose, lactose; dextrans; polyols such as mannitol; antioxidants, such as ascorbic acid or glutathione; preservatives; chelating agents; buffers; or other stabilizers or excipients. The choice of a pharmaceutically acceptable carrier(s) and/or physiologically acceptable compound(s) can depend for example, on the nature of the active agent, e.g., solubility, compatibility (meaning that the substances can be present together in the composition without interacting in a manner that would substantially reduce the pharmaceutical efficacy of the pharmaceutical composition under ordinary use situations) and/or route of administration of the composition. The pharmaceutical composition could be in the form of a liquid, gel, lotion, tablet, capsule, ointment, transdermal patch, etc. A pharmaceutical composition can be administered to a subject by various routes including, for example, parenteral administration. Exemplary routes of administration include intravenous administration; respiratory administration (e.g., by inhalation), nasal administration, intraperitoneal administration, oral administration, subcutaneous administration and topical administration. For oral administration, the compounds can be formulated with pharmaceutically acceptable carriers as tablets, pills, dragees, capsules, liquids, gels, syrups, slurries, suspensions, etc. In some embodiments a compound may be administered directly to a tissue e.g., a tissue in which cancer cells are or may be present or in which the cancer is likely to arise. Direct administration could be accomplished, e.g., by injection or by implanting a sustained release implant within the tissue. Of course a sustained release implant could be implanted at any suitable site. In some embodiments, a sustained release implant may be particularly suitable for prophylactic treatment of subjects at risk of developing a recurrent cancer. In some embodiments, a sustained release implant delivers therapeutic levels of the active agent for at least 30 days, e.g., at least 60 days, e.g., up to 3 months, 6 months, or more. One skilled in the art would select an effective dose and administration regimen taking into consideration factors such as the patient's weight and general health, the particular condition being treated, etc. Exemplary doses may be selected using in vitro studies, tested in animal models, and/or in human clinical trials as standard in the art.

In some embodiments, the pharmaceutical composition is delivered by means of a microparticle or nanoparticle or a liposome or other delivery vehicle or matrix. A number of biocompatible synthetic or naturally occurring polymeric materials are known in the art to be of use for drug delivery purposes. Examples include polylactide-co-glycolide, polycaprolactone, polyanhydride, cellulose derivatives, and copolymers or blends thereof. Liposomes, for example, which consist of phospholipids or other lipids, are nontoxic, physiologically acceptable and metabolizable carriers that are relatively simple to make and administer.

In some embodiments, a compound of the invention is co-administered with one or more other anti-tumor agents. Such agents may be chemotherapeutic agents, hormonal agents, anti-HER2 antibodies, etc. The agents may be administered in the same composition or in separate compositions.

### VII. Compositions and Methods for Inhibiting miRNA Expression

The invention provides compositions and methods relating to inhibiting microRNA expression in cells or subjects, e.g., animals. In certain embodiments the invention provides improvements in microRNA sponge technology. The invention provides a method of inhibiting activity of a miRNA in a mammalian cell such as a human or rodent cell, the method comprising stably expressing a nucleic acid comprising or encoding a plurality of binding sites for the miRNA in the cell. In some embodiments the nucleic acid is integrated into the genome. In some embodiments the nucleic acid is expressed using a viral vector, e.g., a retroviral vector, e.g., a lentiviral vector. In some embodiments the vector is a pBABE vector. In some embodiments at least 5 binding sites for the miRNA are provided, e.g., between 5 and 10 sites, up to, e.g., 20, 50, or more sites. The binding sites may have the same sequence or can include sites with different sequences. In some embodiments the cell is in culture. In some embodiments the cell is part of a multicellular organism, e.g., a human or rodent. In some embodiments, a miRNA sponge of the invention stably reduces function of a miRNA (e.g., as assessed by ability of the miRNA to inhibit expression of a target gene) by between 2 and 10-fold, e.g., by about 2.5-fold or about 5-fold. In some embodiments, the miRNA is one whose expression or over-expression has been implicated as a causative or contributing factor in a human disease, e.g., cancer, wherein inhibition of the miRNA may be of use to treat the disease. The invention further provides vectors, e.g., retroviral vectors, into which a plurality of binding sites for a miRNA of interest can be inserted at a position whereby they will be operably linked to appropriate expression control elements for stable expression in mammalian cells. The vectors of the invention can be used for research or therapeutic purposes. In some embodiments, the vectors are provided as kits.

### Examples

### Example 1: miR-31 Expression is Specifically Attenuated in Metastatic Breast Cancer Cell Lines

Note: Figures referred to as S3, S6, S7, S10, S11, S12, S13, S14, S15, and S17 in Examples 1-9 are found in Valastyan, S., et al. A pleiotropically acting microRNA, miR-31, inhibits breast cancer metastasis, Cell, 137(6):1032-46 (Valastyan 2009) and in USSN 61/120,322, to which this application claims priority.

To identify miRNAs that might regulate breast cancer metastasis, we selected 10 cancer-associated miRNAs for further characterization due to their concordant identification among expression profiling studies of clinical breast tumors (Iorio et al., 2005; Volinia et al., 2006), global analysis of miRNA copy-number variation in human breast carcinomas (Zhang et al., 2006), and localization of miRNA loci to cancer-relevant sites of chromosomal aberration (Calin et al., 2004) (Table 1). These studies did not stratify patients based on metastasis status.

**Table 1. Known Expression Patterns of Candidate miRNAs in Human Breast Tumors**

| **miRNA** | **Calin et al., 2004** | **Iorio et al., 2005** | **Zhang et al., 2006** | **Volinia et al., 2006** |
|---|---|---|---|---|
| miR-31 | downregulated^{a} | no change | downregulated | upregulated |
| miR-100 | downregulated | no change | upregulated | no change |
| miR-101 | downregulated | downregulated | downregulated | no change |
| miR-125b1 | downregulated | downregulated | downregulated | downregulated |
| miR-125b2 | downregulated | downregulated | downregulated | downregulated |
| miR-143 | downregulated | downregulated | downregulated | no change |
| miR-145 | downregulated | downregulated | downregulated | downregulated |
| miR-149 | downregulated | downregulated | downregulated | no change |
| miR-210 | downregulated | upregulated | downregulated | upregulated |
| miR-213 | no change | upregulated | upregulated | upregulated |

| | | | | |
|---|---|---|---|---|
| ^{a}Downregulated, upregulated, and no change refer to expression of the indicated miRNA in primary human breast tumor specimens relative to normal mammary tissue. | | | | |

Expression of the 10 candidate miRNAs was assayed in 15 human and mouse mammary cell lines, which included normal epithelial cells, tumorigenic but non-metastatic cells, and metastatic tumor cells (Table 2). The levels of a single miRNA, miR-31, were specifically attenuated in aggressive human breast cancer cells when compared to primary normal human mammary epithelial cells (HMECs). While non-metastatic tumor cells (HMLER, MCF7-Ras, and SUM-149) exhibited four-fold reduced miR-31, expression of this miRNA in metastatic SUM-159 and MDA-MB-231 cells was diminished by more than 100-fold (Figure 1A).

**Table 2. Characteristics of Mammary Epithelial Cell Lines Employed in This Study**

| **Cell Line** | **Origin** | **Tumorigenic^{a}?** | **Locally invasive^{a}?** | **Capable of forming micrometastases^{a}?** | **Capable of forming macrometastases^{a}?** |
|---|---|---|---|---|---|
| HMEC | Human; Primary mammary epithelial cells | No | No | No | No |
| HMLE | Human; Experimentally transformed | No | No | No | No |
| HMLER | Human; Experimentally transformed | Yes | No | No | No |
| MCF7-Ras | Human; Pleural effusion | Yes | No | No | No |
| SUM-149 | Human; Primary ductal carcinoma | Yes | No | No | No |
| SUM-159 | Human; Primary carcinoma | Yes | Yes | Yes | No |
| MDA-MB-231 | Human; Pleural effusion | Yes | Yes | Yes | Yes |
| NMuMG | Murine; Primary mammary epithelial cells | No | No | No | No |
| D2.OR | Murine; Spontaneously arising carcinoma | Yes | Yes | No | No |
| D2.1 | Murine; Spontaneously arising carcinoma | Yes | Yes | Yes | No |
| D2A1 | Murine; Spontaneously arising carcinoma | Yes | Yes | Yes | Yes |
| 67NR | Murine; Spontaneously arising carcinoma | Yes | No | No | No |
| 168FARN | Murine; Spontaneously arising carcinoma | Yes | Yes | No | No |
| 4TO7 | Murine; Spontaneously arising carcinoma | Yes | Yes | Yes | No |
| 4T1 | Murine; Spontaneously arising carcinoma | Yes | Yes | Yes | Yes |

| | | | | | |
|---|---|---|---|---|---|
| ^{a}Upon orthotopic implantation into immune-deficient host mice (human cell lines) or a syngenic background (murine cell lines). | | | | | |

Relative to its expression in normal murine mammary gland (NMuMG) cells, miR-31 levels in sub-lines derived from a single murine mammary tumor reflected their capacities to metastasize: miR-31 was reduced by two-fold in metastatic D2.1 and D2A1 cells, but not in non-aggressive D2.OR cells (Figure 1B). miR-31 levels were also inversely proportional to metastatic ability in four mouse mammary carcinoma sub-lines derived from a single spontaneously arising tumor: while miR-31 levels in non-aggressive 67NR cells were similar to those in NMuMG, miR-31 expression was progressively diminished upon acquisition of the capacity to invade locally (168FARN), to form micrometastases (4TO7), and to yield macroscopic metastases (4T1) (Figure 1B). Thus, miR-31 levels are specifically attenuated in aggressive breast cancer cells.

miR-31 expression was heterogeneous in 4T1 cell primary mammary tumors; of note, the proportion of cells expressing miR-31 was 10-fold reduced in lung metastases relative to the fraction of miR-31-positive cells in the primary tumors from which they were derived (Figure 1C). Also, five-fold fewer cells located near the invasive front of 4T1 cell mammary tumors expressed miR-31, compared to cells in the interior of these tumors (Figure 1D). These data raise the possibility that selective pressures diminish the prevalence of miR-31-expressing cells within the pool of successfully metastasizing cells during the course of metastatic progression.

### Example 2: miR-31 Expression Suppresses Metastasis-Relevant Traits in vitro

Given the inverse correlations between miR-31 levels and malignant phenotypes described in Example 1, we assessed the potential for anti-metastatic roles for miR-31. Thus, we cloned and stably expressed miR-31 in metastatic MDA-MB-231 human breast cancer cells ("231 cells"). This overexpression resulted in miR-31 levels comparable to those in HMECs (Figure 8A).

Ectopic miR-31 did not affect proliferation *in vitro,* but did reduce invasion by 20-fold and motility by 10-fold (Figures 2A and 8B-8C). These effects were specifically attributable to the biological activities of miR-31, as equivalent overexpression of a control miRNA, miR-145, failed to influence invasion or motility (Figure 2A and data not shown). Also, miR-31-expressing cells exhibited 60% diminished resistance to anoikis-mediated cell death (Figure 2B).

These defects could not be ascribed to toxicity resulting from ectopic miR-31 (Figure S1D). The consequences of miR-31 expression were not unique to 231 cells: miR-31 reduced invasion, motility, and anoikis resistance, yet did not affect proliferation, in aggressive SUM-159 human breast cancer cells (Figure 9). Hence, miR-31 impairs *in vitro* surrogates of metastatic ability.

### Example 3: miR-31 Expression Suppresses Metastasis in vivo

Due to its effects on *in vitro* traits associated with high-grade malignancy, we asked if ectopic miR-31 could inhibit metastasis in otherwise-aggressive cells. Thus, 231 cells expressing miR-31 were injected into the orthotopic site - the mammary fat pad - of mice. Unexpectedly, miR-31 enhanced primary tumor growth by 1.5-fold and correspondingly increased cell proliferation (Figure 2C and S3A). Control 231 cell primary tumors displayed evidence of local invasion; however, miR-31-expressing tumors were well-encapsulated and non-invasive (Figures 2D and 2E). These changes were not accompanied by altered neovascularization (Figure S3B).

Despite their ability to generate larger primary tumors, 231 cells expressing miR-31 were strikingly impaired in their capacity to seed lung metastases. miR-31-expressing cells formed 95% fewer lesions than did controls 62 days post-implantation (Figure 2F). Thus, miR-31 suppresses metastasis from an orthotopic site, ostensibly due, at least in part, to its ability to impede local invasion.

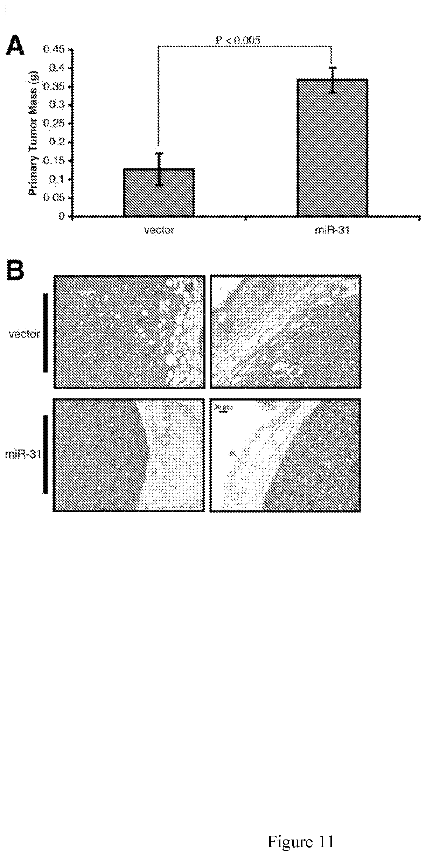

We addressed the possibility that miR-31's impact on these parameters was attributable to clonal variation in our 231 cells by expressing miR-31 in a single-cell-derived population isolated from the parental 231 cells (Figure 10A) (Minn et al., 2005). As before, when injected orthotopically, miR-31-expressing cells formed large, well-encapsulated primary tumors and also reduced lung metastasis by five-fold (Figures 10B-10D). Orthotopic injection of SUM-159 cells expressing miR-31 further corroborated our earlier findings: miR-31 enhanced primary tumor growth, yet miR-31-expressing tumors were more well-confined than control tumors (Figure 11). These observations indicated that the ability of miR-31-expressing cells to form larger, less invasive primary tumors, as well as to seed fewer metastases, is a specific consequence of the biological activities of miR-31.

We determined if miR-31's impact on metastasis was also attributable to effects on later steps of the invasion-metastasis cascade, independent of its influence on local invasion. Thus, we injected miR-31-expressing 231 cells directly into the circulation of mice, thereby circumventing the initial steps of local invasion and intravasation. After one day, miR-31-expressing cells were four-fold impaired in their ability to persist in the lungs (Figure 2G). This difference was not a consequence of an inability of miR-31-expressing cells to become lodged initially in the lung microvasculature, as equal numbers of miR-31-expressing and control cells were detected in the lungs 10 minutes and two hours post-injection (Figures 2G and S6A). These observations suggested that miR-31 regulates early post-intravasation events, such as intraluminal viability, extravasation, and/or initial survival in the lung parenchyma.

Three months after tail vein injection, miR-31-expressing 231 cells generated 40-fold fewer lung metastases than did controls (Figure 2G). We also observed a dramatic effect on the size of eventually formed lesions: after three months, miR-31-expressing cells generated only small micrometastases while control cells formed macroscopic metastases; this occurred despite the fact that miR-31-expressing and control cells established comparably sized micrometastases one month post-injection (Figures 2G and S6B). Such effects on lesion size implied that miR-31 affects metastatic colonization in addition to its influences on local invasion and early post-intravasation events.

### Example 4: Inhibition of miR-31 Promotes Metastasis-Relevant Traits in vitro

The observations described in the preceding Examples demonstrated that miR-31 expression deprives metastatic cells of attributes associated with high-grade malignancy. We next asked if miR-31 also prevents the acquisition of aggressive traits by otherwise-non-metastatic human breast cancer cells. To do so, we transiently inhibited miR-31 in non-invasive MCF7-Ras cells with either antisense oligonucleotides or miRNA sponges. The latter are expression constructs that carry miRNA recognition motifs in their 3' UTR that bind and thus titer miRNAs (Ebert et al., 2007). Both approaches inhibited miR-31 function by >4.5-fold (Figure S7A). Suppression of miR-31 enhanced invasion by 20-fold and motility by five-fold, but cell viability was unaffected by either inhibitor (Figures 3A and S7B).

Techniques for stable miRNA inhibition have been unavailable (Krützfeldt et al., 2006). To address this problem, we modified elements derived from the transiently expressed miRNA sponges, cloned them into a retroviral vector, and created MCF7-Ras cells that stably express the modified miRNA sponges. The miR-31 sponge reduced miR-31 function by 2.5-fold, but did not affect the activity of other known anti-metastatic miRNAs (Figures 12A and 12B). The relatively modest suppression of miR-31 conferred by stable sponge expression elicited strong responses: invasion was enhanced by 12-fold, motility by eight-fold, and anoikis resistance by 2.5-fold (Figures 3B and 12C). The miR-31 sponge failed to alter *in vitro* proliferation (Figure 12D).

When stably expressed in immortalized HMECs or tumorigenic but non-metastatic SUM-149 human breast cancer cells, the miR-31 sponge elicited increased invasion, motility, and anoikis resistance without affecting proliferation (Figure 13 and data not shown). Collectively, these data indicated that sustained miR-31 activity is necessary to prevent the acquisition of aggressive traits by both tumor cells and untransformed breast epithelial cells.

### Example 5: Inhibition of miR-31 Promotes Metastasis in vivo

We exploited our ability to stably inhibit miRNAs in order to assess whether miR-31 activity is required to prevent metastasis *in vivo.* To do so, otherwise-non-metastatic MCF7-Ras cells stably expressing the miR-31 sponge were orthotopically implanted into mice. Inhibition of miR-31 failed to alter *in vivo* proliferation and primary tumor growth (Figures 3C and S10A). Primary tumors derived from miR-31 sponge-expressing cells were poorly encapsulated and locally invasive, while control MCF7-Ras tumors appeared well-confined and non-invasive (Figures 3D and 3E). Again, neovascularization did not differ (Figure S10B).

Strikingly, miR-31 sponge-expressing MCF7-Ras cells metastasized to the lungs in significant numbers, while control tumor-bearing host lungs were largely devoid of tumor cells; cells with impaired miR-31 activity formed 10-fold more lesions than did controls (Figure 3F). Hence, continuous miR-31 function is required to prevent metastasis from an orthotopic site.

We asked if loss of miR-31 activity also promoted metastasis by intervening at steps of the invasion-metastasis cascade subsequent to local invasion. Thus, we intravenously injected mice with miR-31 sponge-expressing MCF7-Ras cells. Within one day, miR-31 inhibition enhanced cell number in the lungs by six-fold; similarly, at later times after injection, miR-31 sponge-expressing cells were 10-fold more prevalent in the lungs than were controls (Figure 3G). The differing metastatic abilities of control and miR-31 sponge-expressing cells did not arise due to failure of control cells to become lodged initially in the lung vasculature, as equal numbers of cells from each cohort were present 10 minutes after injection (Figures 3G and S11).

Suppression of miR-31 also affected lesion size four months after tail vein injection: whereas control cells formed only small micrometastases, miR-31 sponge-expressing cells produced macroscopic metastases (Figure 3G). Together, these data extended and reinforced our ectopic expression studies by demonstrating that miR-31 affects local invasion, early post-intravasation events, and metastatic colonization.

### Example 6: miR-31 Directly Regulates a Cohort of Pro-Metastatic Genes

miR-31's ability to impede multiple steps of the invasion-metastasis cascade might derive from its ability to pleiotropically regulate genes involved in diverse aspects of metastatic dissemination. To identify effectors of miR-31, we used two algorithms that predict the mRNA targets of a miRNA - PicTar (Krek et al., 2005) and TargetScan (Grimson et al., 2007). Based on the representation of miR-31 sites in their 3' UTRs, >200 mRNAs were predicted to be regulated by miR-31. Gene Ontology (Ashburner et al., 2000) revealed that these targets included a disproportionately large number of genes encoding proteins with roles in motility-related processes, such as cell adhesion, cytoskeletal remodeling, and cell polarity (data not shown).

Guided by this Gene Ontology analysis, we cloned the 3' UTRs of 16 putative miR-31 targets from these overrepresented categories, including several implicated in tumor invasion (Sahai and Marshall, 2002; McClatchey, 2003), into a luciferase construct. Reporter assays using miR-31-expressing 231 cells revealed that miR-31 repressed six of the UTRs: frizzled3 (Fzd3), integrin α5 (ITGA5), myosin phosphatase-Rho interacting protein (M-RIP), matrix metallopeptidase 16 (MMP16), radixin (RDX), and RhoA (Figure 4A). Mutation of the putative miR-31 site(s) in these six 3' UTRs (Table 3) abrogated responsiveness to miR-31 (Figure 4B). In the case of RhoA, whose UTR contains two miR-31 sites separated by 152 nucleotides, mutation of either motif abolished miR-31-responsiveness (Figure 4B), suggesting functional interaction between the sites (Grimson et al., 2007).

**Table 3. Site-Specific Mutation of miR-31 Binding Sites in 3' UTR Reporter Constructs**

| **3' UTR** | **Wild Type Sequence** | **Mutagenized Sequence** |
|---|---|---|
| Fzd3 | TCTTGCCA (SEQ ID NO: 5) | TC**GGCG**CA (SEQ ID NO: 6) |
| ITGA5 | CTTGCCA (SEQ ID NO: 7) | C**AAAAG**A (SEQ ID NO: 8) |
| M-RIP | ATCTTGC (SEQ ID NO: 9) | ATC**GGCG** (SEQ ID NO: 10) |
| MMP16 | CTTGCCA (SEQ ID NO: 11) | C**AAACAG** (SEQ ID NO: 12) |
| RDX | CTTGCCA (SEQ ID NO: 13) | **TCCA**CCA (SEQ ID NO: 14) |
| RhoA (site 1)^{a} | ATCTTGC (SEQ ID NO: 15) | **GGTAG**GC (SEQ ID NO: 16) |
| RhoA (site 2)^{b} | TCTTGC (SEQ ID NO: 17) | C**GCC**GC (SEQ ID NO: 18) |

| | | |
|---|---|---|
| ^{a}The miR-31 motif at nucleotides 145-151 of the human RhoA 3' UTR. ^{b}The miR-31 motif spanning nucleotides 303-309 of the RhoA 3' UTR. Bold (and red if in color): nucleotides altered in the mutagenized reporter constructs. | | |

Endogenous Fzd3, ITGA5, MMP16, RDX, and RhoA protein levels were assayed in miR-31-expressing 231 cells. miR-31 repressed the levels of these proteins by 40-60% (Figure 4C). miR-31's effects on levels of the M-RIP protein could not be evaluated due to the lack of appropriate antibodies. Also, miR-31 reduced the endogenous mRNA levels of these six targets by two-fold in SUM-159 cells, as well as Fzd3, ITGA5, MMP16, RDX, and RhoA mRNA levels in 231 cells (Figure 4D). miR-31 did not affect CXCL12 mRNA levels - a computationally predicted miR-31 target found not to be regulated by this miRNA - in either cell type (Figures 4A and 4D). These data indicated that miR-31 directly regulates endogenous Fzd3, ITGA5, M-RIP, MMP16, RDX and RhoA expression in human breast cancer cells.

We determined if concomitant repression of Fzd3, ITGA5, M-RIP, MMP16, RDX, and RhoA correlated with disease progression in clinical breast cancers by examining expression profiling data from 295 primary breast tumors (Table 4) (van de Vijver et al., 2002). To do so, we constructed a miR-31 target signature based on coordinate differential expression of these six genes. Within this cohort, high expression of the miR-31 target signature was associated with metastasis, as well as poor survival, relative to signature-negative tumors; five-year survival among patients negative for the target signature was 90%, while >35% of target signature-positive patients succumbed to their disease over this interval (Figures 5A and 5B). Thus, coordinate repression of Fzd3, ITGA5, M-RIP, MMP16, RDX, and RhoA correlated with more favorable outcome in clinical breast tumors.

**Table 4. Fold-Change Values in 295 Human Breast Tumors (van de Vijver et al., 2002)**

| **Gene** | **Fold-Change (Range)** | **Fold-Change (Quartile₃**-**Quartile₁)** | **Fold-Change (Target Signature-Positive vs. Signature-Negative)** |
|---|---|---|---|
| Fzd3 | 7.21 | 1.62 | 2.75 |
| ITGA5 | 11.56 | 1.61 | 3.38 |
| M-RIP | 8.24 | 1.37 | 2.32 |
| MMP16 | 14.15 | 1.28 | 2.54 |
| RDX | 13.18 | 1.62 | 3.75 |
| RhoA | 5.7 | 1.43 | 2.39 |

To assess the functional contributions of these miR-31 targets to aggressive phenotypes, we first examined if their inhibition affected the invasion or motility of 231 cells. Transfection with siRNAs potently reduced target protein levels without affecting cell viability (Figures S12A and S12B). siRNAs targeting Fzd3, ITGA5, RDX, or RhoA reduced invasion and motility, while siRNAs against M-RIP or MMP16 failed to affect these traits (Figures 5C and S12C).

We asked if inhibition of these effectors compromised resistance to anoikis. siRNAs against ITGA5, RDX, or RhoA sensitized 231 cells to anoikis; in contrast, siRNAs targeting Fzd3, M-RIP, or MMP16 had no effect on anoikis resistance (Figure 5D). Hence, suppression of Fzd3, ITGA5, RDX, or RhoA impaired metastasis-relevant traits *in vitro.*

### Example 7: Re-Expression of Fzd3, ITGA5, RDX, and RhoA Reverses miR-31-Dependent Metastasis-Relevant Phenotypes in vitro

To determine whether *in vitro* phenotypes associated with miR-31 expression could be reversed via restoration of Fzd3, ITGA5, M-RIP, MMP16, RDX, or RhoA levels, we transfected miR-31-expressing 231 cells with individual expression constructs rendered miRNA-insensitive by deletion of their 3' UTRs; this was not cytotoxic (Figures S13A-S13B and data not shown). In miR-31-expressing cells, Fzd3, ITGA5, RDX, or RhoA reversed, at least partially, miR-31-imposed invasion and motility defects; in contrast, M-RIP or MMP16 had no effect on these traits (Figures 5E and S13C). Surprisingly, re-expression of RDX or RhoA completely rescued miR-31-mediated invasion and motility defects. Expression of the six targets failed to enhance the invasion or motility of control 231 cells (Figures 5E and S13C).

We evaluated if re-expression of any of the six targets rescued miR-31's effects on anoikis. ITGA5, RDX, or RhoA reversed, at least in part, anoikis susceptibility resulting from ectopic miR-31; in contrast, Fzd3, M-RIP, or MMP16 failed to affect this trait (Figure 5F). In fact, ITGA5 or RhoA completely rescued miR-31-dependent anoikis phenotypes. The six targets did not enhance anoikis resistance in control 231 cells (Figure 5F). Hence, Fzd3, ITGA5, RDX, and RhoA are functionally relevant effectors of miR-31 for conferring malignant traits *in vitro.*

### Example 8: Re-Expression of RhoA Partially Reverses miR-31-Imposed Metastasis Defects in vivo

RhoA afforded the most pronounced reversal of miR-31-mediated phenotypes. Thus, we stably re-expressed miRNA-resistant RhoA in 231 cells that already had been infected with either miR-31 or control vector (Figures S14A and S14B). RhoA did not affect proliferation *in vitro,* but did abrogate miR-31-imposed invasion, motility, and anoikis resistance defects (Figures S14C-S14F).

To ascertain if restored RhoA levels reversed *in vivo* metastasis phenotypes ascribable to miR-31, we orthotopically injected mice with 231 cells expressing combinations of miR-31, RhoA, and control vectors. As observed previously, miR-31 enhanced primary tumor growth (Figure 6A). RhoA initially augmented primary tumor growth in the presence of ectopic miR-31, but failed to do so in control 231 cells (Figure 6A). In consonance with our earlier findings, control 231 primary tumors were locally invasive, while miR-31-expressing tumors were non-invasive (Figures 6B and 6C). In control 231 cells, ectopic RhoA failed to exacerbate the extent of local invasion; in contrast, RhoA abolished the previously encapsulated appearance of miR-31-expressing tumors and enabled invasion into surrounding normal tissue (Figures 6B and 6C).

Re-expression of RhoA restored lung metastasis in miR-31-expressing 231 cells to 75% of control cell levels, while RhoA failed to enhance metastasis in control 231 cells (Figure 6D). Thus, re-expression of RhoA partially, yet robustly, reverses metastasis-suppression imposed by miR-31. The observed magnitude of rescue is surprising, as RhoA is only one member of a larger cohort of metastasis-relevant genes repressed by miR-31.

By intravenously injecting mice with 231 cells expressing miR-31 and/or RhoA, we gauged if RhoA-mediated reversal of miR-31-imposed metastasis defects was solely attributable to effects on local invasion. While expression of miR-31 and/or RhoA failed to affect the initial lodging of tumor cells in the lung vasculature, the number of cells that persisted in the lungs differed within one day of injection (Figures 6E and S15). As before, miR-31 inhibited both the number of metastases formed and their eventual size (Figure 6E). While expression of RhoA in control 231 cells failed to enhance metastasis, RhoA restored the number of lung metastases to 60% of control cell levels in miR-31-expressing cells; however, RhoA did not facilitate the formation of macroscopic metastases in cells with ectopic miR-31 (Figure 6E).

Together, these data indicated that miR-31's ability to inhibit metastasis is attributable, in significant part, to its capacity to inhibit RhoA. miR-31-mediated repression of RhoA affects both local invasion and early post-intravasation events. However, these data also implied that the full spectrum of miR-31's effects on metastasis are elicited only via the coordinate repression of multiple targets, as suppression of RhoA alone could not explain the complete impact of miR-31 on the number of metastases formed or its effects on metastatic colonization.

### Example 9: miR-31 Expression Correlates Inversely With Metastasis in Human Breast Tumors

Because established cell lines and xenograft studies cannot fully recapitulate clinical malignancy, we extended our analyses by assaying miR-31 expression in specimens from 56 human breast cancer patients (Table 5; Median follow-up = 59 months). Relative to grade-matched estrogen receptor (ER)⁺ tumors, which are associated with more favorable disease outcome (Sørlie et al., 2001), basal-like tumors exhibited 40% reduced miR-31; no difference in miR-31 levels was observed between ER⁺ and HER2⁺ tumors (Figure 14).

**Table 5. Annotation of Clinical Breast Tumor Specimens**

| **#** | **Age^{a}** | **Histological Subtype** | **Molecular Subtype** | **Grade** | **ER** | **PR** | **HER2** | **LN Status** | **Metastatic Recurrence?** | **Sites of Metastasis** |
|---|---|---|---|---|---|---|---|---|---|---|
| 1 | 54 | D | HER | II | n | n | p | n | No | None |
| 2 | 57 | D | HER | II | n | n | p | p | Yes | Viscera |
| 3 | 85 | D | HER | II | n | n | p | U | Yes | Pleura |
| 4 | 63 | D | HER | III | n | n | p | p | No | None |
| 5 | 41 | D | HER | III | n | n | p | n | No | None |
| 6 | 44 | D | HER | III | n | n | n | n | No | None |
| 7 | 37 | D | HER | III | n | n | p | n | No | None |
| 8 | 55 | D | HER | III | n | n | p | p | No | None |
| 9 | 42 | D | HER | III | p | n | p | p | No | None |
| 10 | 45 | D | HER | III | n | n | p | n | No | None |
| 11 | 47 | D | HER | III | p | p | p | p | No | None |
| 12 | 56 | D | HER | III | n | n | p | n | No | None |
| 13 | 49 | D | HER | III | p | p | p | n | No | None |
| 14 | 53 | D | HER | III | n | n | p | p | Yes | Viscera |
| 15 | 45 | D | HER | III | n | n | p | p | Yes | Viscera |
| 16 | 62 | D | ERLG | I | p | p | n | n | No | None |
| 17 | 40 | D | ERLG | I | p | n | n | p | No | None |
| 18 | 37 | D | ERHG | I | p | p | n | p | No | None |
| 19 | 30 | D | ERHG | II | p | p | p | p | No | None |
| 20 | 37 | D | ERHG | II | p | p | n | p | No | None |
| 21 | 74 | D | ERHG | II | n | n | n | n | No | None |
| 22 | 64 | D | ERHG | III | p | p | p | n | No | None |
| 23 | 57 | D | ERHG | III | p | p | lp | p | No | None |
| 24 | 78 | D | ERHG | III | p | p | n | p | No | None |
| 25 | 55 | D | ERHG | III | p | p | n | p | Yes | Bone only |
| 26 | 57 | D | Basal | II | n | n | n | n | No | None |
| 27 | 41 | D | Basal | III | n | n | n | n | No | None |
| 28 | 40 | D | Basal | III | n | n | n | n | No | None |
| 29 | 49 | D | Basal | III | n | n | p | n | No | None |
| 30 | 78 | D | Basal | III | n | n | n | n | No | None |
| 31 | 48 | D | Basal | III | n | n | n | n | No | None |
| 32 | 48 | D | Basal | III | n | n | n | n | No | None |
| 33 | 65 | D | Basal | III | n | n | n | n | No | None |
| 34 | U | D | Basal | III | n | n | n | n | No | None |
| 35 | 79 | D | Basal | III | n | n | n | n | Yes | Bone only |
| 36 | 42 | D | Basal | III | n | n | n | p | Yes | Viscera |
| 37 | 43 | D | Basal | III | n | n | n | n | Yes | Viscera |
| 38 | 53 | D | Basal | III | n | n | n | n | Yes | Viscera |
| 39 | 64 | D | Basal | III | n | n | n | n | Yes | Viscera |
| 40 | 72 | D | U | III | n | n | n | n | U | U |
| 41 | 71 | L | HER | III | p | p | p | p | No | None |
| 42 | 62 | L | ERLG | I | p | p | n | n | No | None |
| 43 | 81 | L | ERLG | I | p | pl | n | p | No | None |
| 44 | 62 | L | ERLG | I | p | p | n | n | No | None |
| 45 | 45 | L | ERLG | II | p | p | n | p | No | None |
| 46 | 45 | L | ERLG | II | p | p | n | n | No | None |
| 47 | 60 | L | ERHG | I | p | n | n | p | Yes | Bone only |
| 48 | 46 | L | ERHG | III | p | p | lp | p | No | None |
| 49 | 32 | M | HER | II | p | p | p | p | No | None |
| 50 | 44 | M | HER | II | p | pl | p | p | Yes | Viscera |
| 51 | 44 | M | HER | II | p | p | p | p | Yes | Viscera |
| 52 | 46 | M | ERLG | II | p | p | n | p | Yes | Bone only |
| 53 | 58 | M | ERHG | II | p | p | n | p | No | None |
| 54 | 38 | M | ERHG | III | pl | pl | n | p | Yes | Viscera |
| 55 | 48 | M | Basal | III | p | p | n | p | No | None |
| 56 | 68 | M | Basal | III | n | n | p | p | No | None |

| | | | | | | | | | | |
|---|---|---|---|---|---|---|---|---|---|---|
| ^{a}At time of initial presentation. ER = Estrogen receptor; PR = Progesterone receptor; LN = lymph node; U = Unknown; D = Ductal; L = Lobular; M = Mixed type; ERLG = ER-positive low-grade; ERHG = ER-positive high-grade; p = positive; n = negative; pl = positive-low; lp = low-positive. | | | | | | | | | | |

When these 56 tumors were stratified based on clinical progression, we found that miR-31 expression was diminished in primary tumors that subsequently metastasized, when compared to normal breast tissue and primary tumors that did not recur; moreover, low miR-31 levels correlated strongly with reduced distant disease-free survival relative to tumors with high miR-31 (Figures 7A and 7B). Similarly, within this cohort of tumors, high RhoA expression was associated with an increased incidence of distant metastasis (Figure S17).

The association of low miR-31 levels with metastasis persisted independent of both tumor grade and molecular subtype (Figure 15). Such grade- and subtype-independence is quite surprising, as clinically utilized prognostic markers for breast cancer largely correlate with these parameters; furthermore, currently available markers do not identify a worse-prognosis group within the more aggressive basal-like or HER2⁺ subtypes (Desmedt et al., 2008). Consequently, miR-31 appears to be a ubiquitous marker for aggressive disease in primary breast carcinomas, and levels of this miRNA may therefore represent a rather uniquely instructive prognostic factor for human breast cancer. In sum, miR-31 expression in primary breast tumors correlated inversely with metastatic recurrence. Thus, based on the data obtained from this set of human breast tumor samples, miR-31 appears to represent a marker for metastasis in a variety of breast cancer subtypes based on the data obtained from this set of human breast tumor samples. Further assessment of its utility as a prognostic indicator may involve extension of these initial observations such as analysis of larger cohorts.

We next assessed the heterogeneity of miR-31 expression in human primary breast tumors, as well as distant metastases arising in the same patients. miR-31 was expressed in 65% of the cells in these primary tumors; however, miR-31 was detected in only 12-30% of cells in patient-matched distant metastases (Figure 7C). These data raise the possibility that selective pressures operating over the course of breast cancer progression diminish the representation of miR-31-expressing cells within the population of successfully metastasizing cells.

Finally, we asked if expression of ITGA5, RDX, and RhoA was also heterogeneous in primary human breast tumors. RDX and RhoA were expressed in 60-75% of cells in the primary tumors examined, while ITGA5 was detected in >80% of cells (Figure 7D). Distant metastases were more homogeneous for the expression of RDX and RhoA than the primary tumors from which they were derived, as >90% of cells in the metastases expressed RDX and RhoA (Figure 7D). Similarly, >90% of cells in the metastases expressed ITGA5; however, the widespread ITGA5 expression observed in the patient-matched primary tumors complicates interpretation of its expression in distant metastases (Figure 7D).

### Discussion of Examples 1-9

miRNAs can modulate a wide variety of biological processes. In the above Examples, we demonstrate that a single human miRNA, miR-31, is endowed with the ability to concomitantly repress multiple pro-metastatic targets and to thereby inhibit several distinct steps of the invasion-metastasis cascade. Moreover, miR-31 levels correlate inversely with metastatic recurrence in a cohort of human breast tumors, an association that appears to persist independent of both tumor grade and subtype.

In contrast to various other miRNA that have been reported to have pro- or anti-metastatic functions, miR-31 obstructs metastasis without exerting confounding influences on primary tumor development. As such, *mir-31* might aptly be categorized as a "metastasis suppressor gene" (Steeg, 2002). This unique aspect of miR-31 function, among others, raises questions regarding the still-uncharacterized role of this miRNA in normal cell and organismic physiology. Of significance, loss of miR-31 activity enhances invasiveness, motility, and anoikis resistance in untransformed human mammary epithelial cells. Hence, inactivation of miR-31 in normal epithelium may facilitate dissemination prior to transformation to a fully neoplastic state. This suggests one putative mechanism by which the invasion-metastasis cascade could be initiated very early during the course of tumor progression, a phenomenon that has recently been observed in clinical breast tumors (Hüsemann et al., 2008).

Given the capacity of miR-31 to enhance primary tumor growth, an oncogenic role for this miRNA (mechanistically independent of its metastasis-suppressive functions) cannot be formally excluded. Such duality of action is not unprecedented (Massagué, 2008), and is consistent with notions that metastasis- and tumorigenesis-enabling attributes can be biologically distinct and acquired via independent selective pressures during malignant progression.

Previous studies have described effects of specific miRNAs on an early stage of the invasion-metastasis cascade - local invasion. The present work demonstrates that miRNAs can also influence later steps of metastasis and that an individual miRNA can intervene at multiple distinct stages of the invasion-metastasis cascade. miR-31 regulates the local invasion of primary mammary tumors, as well as intraluminal survival, extravasation, and/or initial viability in a foreign microenvironment. miR-31 also suppresses colonization - the final and rate-limiting step of metastasis (Fidler, 2003). miR-31-imposed suppression of RhoA partially explains the effects of this miRNA on local invasion and early post-intravasation events; however, the mechanisms by which miR-31 suppresses metastatic colonization at least in part depend on its effects on other genes.

### EXPERIMENTAL PROCEDURES used in Examples 1-9

**Cell Culture.** MDA-MB-231 and MCF7-Ras cells were obtained from the ATCC and cultured under standard conditions. HMEC and HME cells have been described (Ma et al., 2007). SCP3 cells were obtained from J. Massagué (Minn et al., 2005). SUM-149 and -159 cells were provided by S. Ethier (Ma et al., 2007). D2 cells have been described (Morris et al., 1993). 67NR, 168FARN, 4TO7, and 4T1 cells were obtained from F. Miller (Aslakson and Miller, 1992).

**miRNA Detection.** Total RNA, inclusive of the small RNA fraction, was extracted from cultured cells with a *mir*Vana miRNA Isolation Kit (Ambion). RT-PCR-based detection of mature miR-31 and 5S rRNA was achieved with a *mir*Vana miRNA Detection Kit and gene-specific primers (Ambion).

**miRNA *in situ* Hybridization.** miRNA expression was assessed from paraffin sections using a protocol adapted from Silahtaroglu et al. (2007). Briefly, after a four hour pre-hybridization, a 5' FITC-labeled miRCURY LNA probe targeting miR-31 (Exiqon) was hybridized to proteinase K-treated 10 µm sections at 55°C for 12 hours. Slides were then incubated with anti-FITC-HRP (PerkinElmer), and the resulting signal was intensified with the TSA Plus Fluorescein System (PerkinElmer).

**Human Breast Tumors.** Primary breast tumors, distant metastases, and normal breast tissue were collected and processed in compliance with a protocol approved by the Brigham and Women's Hospital IRB. Fresh tissue was harvested from patients, OCT-embedded, snap-frozen, and preserved at -80°C. Recurrent cases were primary tumors from patients that developed distant metastases. For each recurrent case, two non-recurrent cases were selected to control for date of diagnosis, molecular subtype, lymph node status, and time of follow-up. Total RNA was isolated from 35 µm sections via TRIzol extraction and a *mir*Vana miRNA Isolation Kit. To discern if miR-31 levels correlate with distant metastasis, primary tumors were classified as miR-31-positive or -negative. Tumors were considered miR-31-positive or -negative if the normalized expression of miR-31 resided in the top or bottom 30% of tumors in this cohort, respectively. Similarly, tumors were classified as RhoA-high or -low if their RhoA levels were in the top or bottom 30% of tumors examined.

**Invasion and Motility Assays.** For invasion assays, 1.0 x 10⁵ cells were seeded in a Matrigel-coated chamber with 8.0 µm pores (BD Biosciences); for motility assays, 5.0 x 10⁴ cells were plated atop uncoated membranes with 8.0 µm pores (BD Biosciences). Cells were seeded in serum-free media, and translocated toward complete growth media for 20 hours. Fugene6 (Roche) was used to transfect cells 24 hours prior to plating. 200 nM miRIDIAN miRNA Inhibitors (Dharmacon) were employed to transiently inhibit miR-31. SMARTpool siRNAs against Fzd3, ITGA5, M-RIP, MMP16, RDX, or RhoA (Dharmacon) were provided at 100 nM. Antisense oligonucleotides and siRNAs were transfected 48 hours prior to seeding using Oligofectamine (Invitrogen).

**Anoikis Assays.** Anoikis resistance was evaluated by seeding 7.5 x 10⁴ cells in ultra-low attachment plates (Corning). After 24 hours of anchorage-independent culture, cells were resuspended in 0.4% trypan blue (Sigma) and cell viability was assessed.

**Animal Studies.** All research involving animals complied with protocols approved by the MIT Committee on Animal Care. For spontaneous metastasis assays, age-matched female NOD/SCID mice (propagated on-site) were bilaterally injected into the mammary fat pad with the indicated number of tumor cells in 1:2 Matrigel (BD Biosciences) plus normal growth media. For experimental metastasis assays, age-matched female NOD/SCID mice were injected with 5.0 x 10⁵ cells (resuspended in PBS) via the tail vein. Metastasis was quantified using a fluorescent microscope within three hours of specimen isolation.

**Luciferase Assays.** 5.0 x 10⁴ cells were co-transfected with 50 ng of the indicated pIS1 *Renilla* luciferase construct and 50 ng of a pIS0 firefly luciferase normalization control. Lysates were collected 24 hours post-transfection, and *Renilla* and firefly luciferase activities were measured with a Dual-Luciferase Reporter System (Promega).

**Immunoblots.** Lysates were resolved by electrophoresis, transferred to a PVDF membrane, and probed with antibodies against β-actin (Santa Cruz), Fzd3 (Abcam), ITGA5 (Santa Cruz), MMP16 (Abcam), RDX (Cell Signaling), or RhoA (Santa Cruz).

**miR-31 Target Signature.** Expression profiling of 295 human breast tumors (van de Vijver et al., 2002) was used to categorize tumors as miR-31 target signature-positive or -negative. Tumors were considered target signature-positive or -negative if the normalized expression of multiple of the six miR-31 targets herein identified resided in the top or bottom 15% of tumors in this cohort, respectively.

**Immunohistochemistry.** Detection of Ki-67 (Pharmingen), MECA-32 (U. of Iowa), ITGA5 (Santa Cruz), RDX (Santa Cruz), or RhoA (Abcam) was performed on 5 µm paraffin sections using the indicated antibodies, Vectastain Elite ABC kits (Vector), and ImmPACT DAB Substrate (Vector).

**Statistical Analyses.** Data are presented as mean ± s.e.m. Unless otherwise noted, Student's t-test was used for comparisons, with P <0.05 considered significant.

**Plasmid Construction and Generation of Stable Cell Lines.** The human miR-31 gene was PCR-amplified from genomic DNA and cloned into pcDNA3.1 (Invitrogen), then subcloned into the BamHI and SalI sites of the pBABE-puro retroviral vector (Morgenstern and Land, 1990). Synthetic miR-31, miR-126, miR-206, and miR-335 binding site-containing *Renilla* luciferase reporter genes were constructed by annealing, purifying, and cloning corresponding short oligonucleotides into the SacI and AgeI sites of the 3' UTR of a pIS1 vector backbone provided by D. Bartel (Farh et al., 2005). Transient CMV-driven miRNA sponge backbones were provided by P. Sharp; a control sponge containing tandem non-targeting binding sites was identical to that utilized previously (Ebert et al., 2007), while the miR-31 sponge was constructed by annealing, purifying, and cloning oligonucleotides containing seven tandem "bulged" (at positions 9-12) miR-31 binding motifs into the XhoI and ApaI sites of the CMV sponge backbone. For stable expression studies, the BamHI-ApaI fragments of the miR-31 and control CMV sponge constructs were subcloned into the pBABE-puro retroviral vector. Luciferase reporter genes driven by the 3' UTRs of the indicated computationally predicted miR-31 targets were created via PCR-based amplification from human genomic DNA, and then cloned into AgeI, SacI, SpeI, and/or XbaI sites within the 3' UTR of the pIS1 *Renilla* luciferase reporter backbone. The indicated mutagenized miR-31 recognition site-containing luciferase reporter genes were generated with a QuikChange II XL Site-Directed Mutagenesis Kit (Stratagene), in accordance with the manufacturer's instructions. For transient overexpression experiments, previously described constructs encoding Fzd3 (Deardorff et al., 2001), ITGA5 (Kuwada and Li, 2000), M-RIP (Surks et al., 2003), MMP16 (Hotary et al., 2006), RDX (Batchelor et al., 2004), and RhoA (Subauste et al., 2000) were provided by P. Klein, S. Kuwada, H. Surks, S. Weiss, S. Crouch, and G. Bokoch, respectively. For stable overexpression studies, RhoA was directly subcloned from pcDNA3 (Invitrogen) into the retroviral vector pBABE-zeo (Morgenstern and Land, 1990).

All stable cell lines were generated via retroviral infection using HEK293T cells, as has been previously described (Elenbaas et al., 2001). GFP-labeled MDA-MB-231 and MCF7-Ras cells were created via infection with pWZL-blast-EGFP.

**Measurements of *in vitro* Cell Proliferation.** *In vitro* proliferative kinetics were assayed by seeding 7.5 x 10⁴ cells per well in 6-well plates. Total cell number was assessed every two to three days, as indicated, by trypsinization and manual counting with a hemocytometer.

**Cell Viability Assays.** The impact of transient transfection with various constructs and reagents on cell viability was assessed by a trypan blue dye exclusion assay. Briefly, in parallel to the corresponding invasion, motility, and anoikis assays, 2.0 x 10⁵ cells were seeded in 6-well plates, and then transfected with the indicated expression constructs, siRNAs, or antisense oligonucleotides, as described in the Experimental Procedures. After 24 hours (expression constructs) or 48 hours (siRNAs and antisense oligonucleotides), cells were trypsinized, resuspended in 0.4% trypan blue staining solution (Sigma), and manually counted using a hemocytometer.

**cDNA Synthesis and Real Time RT-PCR.** Where indicated, cDNA was prepared from 200 ng of total RNA using the SuperScript III First-Strand Synthesis System (Invitrogen), and target levels were assessed via SYBR Green-based real time RT-PCR (Applied Biosystems).

**Oligonucleotide Sequences.** Oligonucleotides employed in this study were:
cloning miR-31 from genomic DNA, ACAATACATAGCAGGACAGGAAGTAAGGAAGGTG (SEQ ID NO: 19) and CATCTTCAAAAGCGGACACTCTAAGGAAGACTATGTTG (SEQ ID NO: 20); cloning miR-145 from genomic DNA, TGCTACAGATGGGGCTGGATGCAGAA (SEQ ID NO: 21) and TAAGCCCTCTTACCTCCAGGGACAGC (SEQ ID NO: 22); miR-31 synthetic binding site, AATGGCGAGCTCAGGCAAGATGCTGGCATAGCTACCGGTAATGGC (SEQ ID NO: 23) and GCCATTACCGGTAGCTATGCCAGCATCTTGCCTGAGCTCGCCATT (SEQ ID NO: 24); cloning modified miRNA sponges to pBABE-puro, AGACCCAAGCTGGCTAGCGTTTAAACTTAAGCTTG (SEQ ID NO: 25) and AATGGCGTCGACTAGAAGGCACAGTCGAGGCT (SEQ ID NO: 26); miR-126 synthetic binding site, GCCATTGAGCTCTCGTACCGTGAGTAATAATGCGACCGGTGCCATT (SEQ ID NO: 27) and AATGGCACCGGTCGCATTATTACTCACGGTACGAGAGCTCAATGGC (SEQ ID NO: 28); miR-206 synthetic binding site, GCCATTGAGCTCTGGAATGTAAGGAAGTGTGTGGACCGGTGCCATT (SEQ ID NO: 29) and AATGGCACCGGTCCACACACTTCCTTACATTCCAGAGCTCAATGGC (SEQ ID NO: 30); miR-335 synthetic binding site, GCCAGTGAGCTCTCAAGAGCAATAACGAAAAATGTACCGGTGCCAGT (SEQ ID NO: 31) and ACTGGCACCGGTACATTTTTCGTTATTGCTCTTGAGAGCTCACTGGC (SEQ ID NO: 32); cloning Arp2/3s5 3' UTR from genomic DNA, TTAACCGAGCTCTCTGGCAGGAAGTGGAT (SEQ ID NO: 33) and TTGGCCTCTAGAGAGTTTATAGAATTTCTGCACCAGTTTGC (SEQ ID NO: 34); cloning CXCL12 3' UTR from genomic DNA, TTAAGGACCGGTGCACAACAGCCAAAAAGGA (SEQ ID NO: 35)and TGGGCCACTAGTAAGCTCCATCACTAACAACTAATGA (SEQ ID NO: 36); cloning Ets-1 3' UTR from genomic DNA, TTAACCGAGCTCTGGCACTGAAGGGGCT (SEQ ID NO: 37) and TCGGCCACCGGTATGAATGAAATTCTTTGT (SEQ ID NO: 38); cloning Fzd3 3' UTR from genomic DNA, TTGGCCGAGCTCTTTGTCTTGTCTAAGGT (SEQ ID NO: 39) and TTGGCCACCGGTAAGAAAGCTACCAATTCTTATTTG (SEQ ID NO: 40); cloning HoxC13 3' UTR from genomic DNA, TTAATAGAGCTCCCACCCACCCGCTGCT (SEQ ID NO: 41) and TTAATAGAGCTCCCACCCACCCGCTGCT (SEQ ID NO: 42); cloning ITGA5 3' UTR from genomic DNA, TTAACCGAGCTCGTCCTCCCAATTTCAGACTC (SEQ ID NO: 43) and TTAACCACCGGTCTAGTTCTGGTCAGTGGGGGCACT (SEQ ID NO: 44); cloning JAZF1 3' UTR from genomic DNA, TTGGCCGAGCTCCATGCTGGTCATAACTG (SEQ ID NO: 45) and TTAACCACCGGTGGGTCAGAGGCAGTTTA (SEQ ID NO: 46); cloning KLF13 3' UTR from genomic DNA, TATATAACCGGTGCCCGCCACAGCCATGA (SEQ ID NO: 47) and TGCGGCCGCGCCCGGACTAGTAAAATAATGAATCATAAATTTTAT (SEQ ID NO: 48); cloning M-RIP 3' UTR from genomic DNA, TTAACCGAGCTCAGAGTCTGAAGGAAGGCCT (SEQ ID NO: 49) and TTAAATACCGGTACCAAGAAAGGAACGAGCGGA (SEQ ID NO: 50); cloning MMP16 3' UTR from genomic DNA, TTAACCGAGCTCGCAGGAGTTTGTGGTAACTT (SEQ ID NO: 51) and TTGGCCACCGGTATCCACCACATTGTGTT (SEQ ID NO: 52); cloning NFAT5 3' UTR from genomic DNA, TTGGCCACCGGTAAATTCCACGAAGAAAATCCTG (SEQ ID NO: 53) and TGGGCCTCTAGAAACTTTCAGTGTTTTATTTTTGACTGCAGCTG (SEQ ID NO: 54); cloning Numb 3' UTR from genomic DNA, TTCCGGACCGGTAAGCAATCATTATGGCTATGT (SEQ ID NO: 55) and TCGGCCACTAGTAAAAGCTTCTACCATGAACATT (SEQ ID NO: 56); cloning RDX 3' UTR from genomic DNA, TTGGCCACCGGTGAGCTGTTATTTTGCATATATG (SEQ ID NO: 57) and TTGGCCACTAGTGAAGGCATGAGCTTTTGTCACTTTATTG (SEQ ID NO: 58); cloning Ret 3' UTR from genomic DNA, TTGGCCACCGGTCATTTCTTTGTGAAAGGT (SEQ ID NO: 59)and TTGGCCACTAGTGCAATGAAGAATGACAAGAAGCT (SEQ ID NO: 60); cloning RhoA 3' UTR from genomic DNA, TTAACCGAGCTCAACCTTGCTGCAAGCACA (SEQ ID NO: 61) and TTGGCCACCGGTAGAAAACTGCCTTTATTCTATTAGTAGTTGG (SEQ ID NO: 62); cloning YY1 3' UTR from genomic DNA, TTGGCCGAGCTCAAAGAAGAGAGAAGACCCTTCT (SEQ ID NO: 63) and TTGGCCACCGGTCTTTAGGATTGCTATTTTATTGTTGCCCT (SEQ ID NO: 64); Fzd3 3' UTR mutagenesis, CTACAGTGAGATGTGATCGGCGCAAAGCCACCAGACCTTGGCTTCC (SEQ ID NO: 64) and GGAAGCCAAGGTCTGGTGGCTTTGCGCCGATCACATCTCACTGTAG (SEQ ID NO: 65); ITGA5 3'UTR mutagenesis, CTGCAAAGATCTGTCCTCAGCCAAAAGAGAGATCCAAAAGAAGCCCCCAG (SEQ ID NO: 66) and CTGGGGGCTTCTTTTGGATCTCTCTTTTGGCTGAGGACAGATCTTTGCAG (SEQ ID NO: 67); M-RIP 3' UTR mutagenesis, GTTTGTTTTTTATTAAATCGGCGACAAAATCCCCGGCCCCTCTCC (SEQ ID NO: 68) and GGAGAGGGGCCGGGGATTTTGTCGCCGATTTAATAAAAAACAAAC (SEQ ID NO: 69); MMP16 3' UTR mutagenesis, GTGTTTATAACAAACAGAAATGATGTTACCGGCCAAAATTTTTCTGGC (SEQ ID NO: 70) and GCCAGAAAAATTTTGGCCGGTAACATCATTTCTGTTTGTTATAAACAC (SEQ ID NO: 71); RDX 3' UTR mutagenesis, GATATGATGGAATGCATCCCACCAGCGGAAAGCACTTACACCAGTTTGACTGTG (SEQ ID NO: 72) and CACAGTCAAACTGGTGTAAGTGCTTTCCGCTGGTGGGATGCATTCCATCATATC (SEQ ID NO: 73); RhoA 3' UTR site one mutagenesis, CCTAAGATTACAAATCAGAAGTCAGGCGGCTACCAGTATTTAGAAGCCAAC (SEQ ID NO: 74) and GTTGGCTTCTAAATACTGGTAGCCGCCTGACTTCTGATTTGTAATCTTAGG (SEQ ID NO: 75); RhoA 3' UTR site two mutagenesis, GGCGCTAATTCAAGGAATTTCTTAACTCGCCGCTTCTTTCTAGAAAGAGAAACAGTGG (SEQ ID NO: 76) and CCAACTGTTTCTCTTTCTAGAAAGAAGCGGCGAGTTAAGAAATTCCTTGAATTAGCGCC (SEQ ID NO: 77); CXCL12 RT-PCR, TGAGAGCTCGCTTTGAGTGACTGGGT (SEQ ID NO: 78) and ATACCACCAGGACCTTCTGTGGATCGCA (SEQ ID NO: 79); Fzd3 RT-PCR, TCCATCCCTGCACAATATAAGGCTTCCACA (SEQ ID NO: 80) and TCTCAATGCATCAACATCGTAGAGGCCAAC (SEQ ID NO: 81); GAPDH RT-PCR, TCACCAGGGCTGCTTTTAAC (SEQ ID NO: 82) and GACAAGCTTCCCGTTCTCAG (SEQ ID NO: 83); ITGA5 RT-PCR, AACTCATCATGGCCAGTGAGGGTAAGGGT (SEQ ID NO: 84) and ATCCTTAATGGCTCAGACATTCGATCCCTCTACAACT (SEQ ID NO: 85); M-RIP RT-PCR, AGGCAGAGCACATGGAGACCAATGCA (SEQ ID NO: 86) and AGTCAGCCAGCCTTTCTTGAAATTCAGCA (SEQ ID NO: 87); MMP16 RT-PCR, AGTACGGCTACCTTCCACCGACTGA (SEQ ID NO: 88) and TACCTCTTGTCTGGTCAGGTACACCGCAT (SEQ ID NO: 89); RDX RT-PCR, GAATCAGGAGCAGCTAGCAGCAGAACTT (SEQ ID NO: 90)and TTGGTCTTTTCCAAGTCTTCCTGGGCTGCA (SEQ ID NO: 91); RhoA RT-PCR, AGGTGGATGGAAAGCAGGTAGAGTTGGCT (SEQ ID NO: 92)and AGGATGATGGGCACGTTGGGACAGA (SEQ ID NO: 93).

### REFERENCE LIST 1

Asangani, I.A., Rasheed, S.A., Nikolova, D.A., Leupold, J.H., Colburn, N.H., Post, S., and Allgayer, H. (2008). MicroRNA-21 post-transcriptionally downregulates tumor suppressor Pdcd4 and stimulates invasion, intravasation and metastasis in colorectal cancer. Oncogene 27, 2128-2136.
Ashburner, M., Ball, C.A., Blake, J.A., Botstein, D., Butler, H., Cherry, J.M., Davis, A.P., Dolinski, K., Dwight, S.S., Eppig, J.T., et al. (2000). Gene ontology: tool for the unification of biology. Nat Genet. 25, 25-29.
Aslakson, C.J. and Miller, F.R. (1992). Selective events in the metastatic process defined by analysis of the sequential dissemination of subpopulations of a mouse mammary tumor. Cancer Res. 52, 1399-1405.
Bartel, D.P. (2009). MicroRNAs: target recognition and regulatory functions. Cell 136, 215-233.
Blenkiron, C., Goldstein, L.D., Thorne, N.P., Spiteri, I., Chin, S.F., Dunning, M.J., Barbosa-Morais, N.L., Teschendorff, A.E., Green, A.R., Ellis, I.O., et al. (2007). MicroRNA expression profiling of human breast cancer identifies new markers of tumor subtype. Genome Biol. 8, R214.
Calin, G.A., Sevignani, C., Dumitru, C.D., Hyslop, T., Noch, E., Yendamuri, S., Shimizu, M., Rattan, S., Bullrich, F., Negrini, M., et al. (2004). Human microRNA genes are frequently located at fragile sites and genomic regions involved in cancers. PNAS 101, 2999-3004.
Calin, G.A. and Croce, C.M. (2006). MicroRNA signatures in human cancers. Nat Rev Cancer 6, 857-866.
Desmedt, C., Haibe-Kains, B., Wirapati, P., Buyse, M., Larsimont, D., Bontempi, G., Delorenzi, M., Piccart, M., and Sotiriou, C. 2008. Biological processes associated with breast cancer clinical outcome depend on the molecular subtypes. Clin Cancer Res. 14, 5158-5165.
Ebert, M.S., Neilson, J.R., and Sharp, P.A. (2007). MicroRNA sponges: competitive inhibitors of small RNAs in mammalian cells. Nat Methods 4, 721-726.
Fidler, I.J. (2003). The pathogenesis of cancer metastasis: the 'seed and soil' hypothesis revisited. Nat Rev Cancer 3, 453-458.
Grimson, A., Farh, K.K., Johnston, W.K., Garrett-Engele, P., Lim, L.P., and Bartel, D.P. (2007). MicroRNA targeting specificity in mammals: determinants beyond seed pairing. Mol Cell 27, 91-105.
Gupta, G.P. and Massagué, J. (2006). Cancer metastasis: building a framework. Cell 127, 679-695.
Huang, Q., Gumireddy, K., Schrier, M., le Sage, C., Nagel, R., Nair, S., Egan, D.A., Li, A., Huang, G., Klein-Szanto, A.J., et al. (2008). The microRNAs miR-373 and miR-520c promote tumour invasion and metastasis. Nat Cell Biol. 10, 202-210.
Hüsemann, Y., Geigl, J.B., Schubert, F., Musiani, P., Meyer, M., Burghart, E., Forni, G., Eils, R., Fehm, T., Riethmüller, G., et al. (2008). Systemic spread is an early step in breast cancer. Cancer Cell 13, 58-68.
Iorio, M.V., Ferracin, M., Liu, C.G., Veronese, A., Spizzo, R., Sabbioni, S., Magri, E., Pedriali, M., Fabbri, M., Campiglio, M., et al. (2005). MicroRNA gene expression deregulation in human breast cancer. Cancer Res. 65, 7065-7070.
Kondo, N., Toyama, T., Sugiura, H., Fujii, Y., and Yamashita, H. (2008). miR-206 Expression is down-regulated in estrogen receptor α-positive human breast cancer. Cancer Res. 68, 5004-5008.
Krek, A., Grün, D., Poy, M.N., Wolf, R., Rosenberg, L., Epstein, E.J., MacMenamin, P., da Piedade, I., Gunsalus, K.C., Stoffel, M., et al. (2005). Combinatorial microRNA target predictions. Nat Genet. 37, 495-500.
Krützfeldt, J., Poy, M.N., and Stoffel, M. (2006). Strategies to determine the biological function of microRNAs. Nat Genet. 38, S14-S19.
Lujambio, A., Calin, G.A., Villanueva, A., Ropero, S., Sánchez-Céspedes, M., Blanco, D., Montuenga, L.M., Rossi, S., Nicoloso, M.S., Faller, W.J., et al. (2008). A microRNA DNA methylation signature for human cancer metastasis. PNAS 105, 13556-13561.
Ma, L., Teruya-Feldstein, J., and Weinberg, R.A. (2007). Tumour invasion and metastasis initiated by microRNA-10b in breast cancer. Nature 449, 682-688.
Massagué, J. (2008). TGFβ in cancer. Cell 134, 215-230.
Mattie, M.D., Benz, C.C., Bowers, J., Sensinger, K., Wong, L., Scott, G.K., Fedele, V., Ginzinger, D., Getts, R., and Haqq, C. (2006). Optimized high-throughput microRNA expression profiling provides novel biomarker assessment of clinical prostate and breast cancer biopsies. Mol Cancer 19, 5-24.
McClatchey, A.I. (2003). Merlin and ERM proteins: unappreciated roles in cancer development? Nat Rev Cancer 3, 877-883.
Minn, A.J., Kang, Y., Serganova, I., Gupta, G.P., Giri, D.D., Doubrovin, M., Ponomarev, V., Gerald, W.L., Blasberg, R., and Massagué, J. (2005). Distinct organ-specific metastatic potential of individual breast cancer cells and primary tumors. J Clin Invest. 115, 44-55.
Morris, V.L., Tuck, A.B., Wilson, S.M., Percy, D., and Chambers, A.F. (1993). Tumor progression and metastasis in murine D2 hyperplastic alveolar nodule mammary tumor cell lines. Clin Exp Metastasis 11, 103-112.
Sahai, E. and Marshall, C.J. (2002). Rho-GTPases and cancer. Nat Rev Cancer 2, 133-142. Sanchez-Carbayo, M., Socci, N.D., Lozano, J., Saint, F., and Cordon-Cardo, C. (2006). Defining molecular profiles of poor outcome in patients with invasive bladder cancer using oligonucleotide microarrays. J Clin Oncol. 24, 778-789.
Sathyan, P., Golden, H.B., and Miranda, R.C. (2007). Competing interactions between micro-RNAs determine neural progenitor survival and proliferation after ethanol exposure. J Neurosci. 27, 8546-8557.
Si, M.L., Zhu, S., Wu, H., Lu, Z., Wu, F., and Mo, Y.Y. (2007). miR-21-mediated tumor growth. Oncogene 26, 2799-2803.
Silahtaroglu, A.N., Nolting, D., Dyrskjøt, L., Berezikov, E., Møller, M., Tommerup, N., and Kauppinen, S. (2007). Detection of microRNAs in frozen tissue sections by fluorescence in situ hybridization using LNA probes and tyramide signal amplification. Nat Protoc. 2, 2520-2528.
Sørlie, T., Perou, C.M., Tibshirani, R., Aas, T., Geisler, S., Johnsen, H., Hastie, T., Eisen, M.B., van de Rijn, M., Jeffrey, S.S., et al. (2001). Gene expression patterns of breast carcinomas distinguish tumor subclasses with clinical implications. PNAS 98, 10869-10874.
Steeg, P.S. (2003). Metastasis suppressors alter the signal transduction of cancer cells. Nat Rev Cancer 3, 55-63.
Tavazoie, S.F., Alarcón, C., Oskarsson, T., Padua, D., Wang, Q., Bos, P.D., Gerald, W.L., and Massagué, J. (2008). Endogenous human microRNAs that suppress breast cancer metastasis. Nature 451, 147-152.
van de Vijver, M.J., He, Y.D., van't Veer, L.J., Dai, H., Hart, A.A., Voskuil, D.W., Schreiber, G.J., Peterse, J.L., Roberts, C., Marton, M.J., et al. (2002). A gene-expression signature as a predictor of survival in breast cancer. NEJM 347, 1999-2009.
Ventura, A. and Jacks, T. (2009). MicroRNAs and cancer: short RNAs go a long way. Cell 136, 586-591.
Volinia, S., Calin, G.A., Liu, C.G., Ambs, S., Cimmino, A., Petrocca, F., Visone, R., Iorio, M., Roldo, C., Ferracin, M., et al. (2006). A microRNA expression signature of human solid tumors defines cancer gene targets. PNAS 103, 2257-2261.
Voorhoeve, P.M., le Sage, C., Schrier, M., Gillis, A.J., Stoop, H., Nagel, R., Liu, Y.P., van Duijse, J., Drost, J., Griekspoor, A., et al. (2006). A genetic screen implicates miRNA-372 and miRNA-373 as oncogenes in testicular germ cell tumors. Cell 124, 1169-1181.
Yan, L.X., Huang, X.F., Shao, Q., Huang, M.Y., Deng, L., Wu, Q.L., Zeng, Y.X., and Shao, J.Y. (2008). MicroRNA miR-21 overexpression in human breast cancer is associated with advanced clinical stage, lymph node metastasis and patient poor prognosis. RNA 14, 2348-2360.
Zhang, L., Huang, J., Yang, N., Greshock, J., Megraw, M.S., Giannakakis, A., Liang, S., Naylor, T.L., Barchetti, A., Ward, M.R., et al. (2006). microRNAs exhibit high frequency genomic alterations in human cancer. PNAS 103, 9136-9141.
Zhu, S., Wu, H., Wu, F., Nie, D., Sheng, S., and Mo, Y.Y. (2008). MicroRNA-21 targets tumor suppressor genes in invasion and metastasis. Cell Res. 18, 350-359.
Batchelor, C.L., Woodward, A.M., and Crouch, D.H. (2004). Nuclear ERM (ezrin, radixin, moesin) proteins: regulation by cell density and nuclear import. Exp Cell Res. 296, 208-222.
Deardorff, M.A., Tan, C., Saint-Jeannet, J.P., and Klein, P.S. (2001). A role for frizzled 3 in neural crest development. Development 128, 3655-3663.
Ebert, M.S., Neilson, J.R., and Sharp, P.A. (2007). MicroRNA sponges: competitive inhibitors of small RNAs in mammalian cells. Nat Methods 4, 721-726.
Elenbaas, B., Spirio, L., Koerner, F., Fleming, M.D., Zimonjic, D.B., Donaher, J.L., Popescu, N.C., Hahn, W.C., and Weinberg, R.A. (2001). Human breast cancer cells generated by oncogenic transformation of primary mammary epithelial cells. Genes Dev. 15, 50-65.
Farh, K.K., Grimson, A., Jan, C., Lewis, B.P., Johnston, W.K., Lim, L.P., Burge, C.B., and Bartel, D.P. (2005). The widespread impact of mammalian MicroRNAs on mRNA repression and evolution. Science 310, 1817-1821.
Hotary, K., Li, X.Y., Allen, E., Stevens, S.L., and Weiss, S.J. (2006). A cancer cell metalloprotease triad regulates the basement membrane transmigration program. Genes Dev. 20, 2673-2686.
Kuwada, S.K. and Li, X. (2000). Integrin alpha5/beta1 mediates fibronectin-dependent epithelial cell proliferation through epidermal growth factor receptor activation. Mol Biol Cell 11, 2485-2496.
Morgenstern, J.P. and Land, H. (1990). Advanced mammalian gene transfer: high titre retroviral vectors with multiple drug selection markers and a complementary helper-free packaging cell line. Nucleic Acids Res. 18, 3587-3596.
Subauste, M.C., Von Herrath, M., Benard, V., Chamberlain, C.E., Chuang, T.H., Chu, K., Bokoch, G.M., and Hahn, K.M. (2000). Rho family proteins modulate rapid apoptosis induced by cytotoxic T lymphocytes and Fas. J Biol Chem. 275, 9725-9733.
Surks, H.K., Richards, C.T., and Mendelsohn, M.E. (2003). Myosin phosphatase-Rho interacting protein. A new member of the myosin phosphatase complex that directly binds RhoA. J Biol Chem. 278, 51484-51493.

### Example 10: Further Analysis of miR-31 in Human Breast Cancer

Example 9 is repeated using a larger cohort of breast tumors. The 5-year and 10-year metastasis-free survival and overall survival rates among patients with high or low miR-31 expression are compared using appropriate statistical tests.

### Example 11: Concomitant Suppression of Three Target Genes Can Explain the Impact of miR-31 on Metastasis

Note: Figures and Table referred to in Example 11 are found in Valastyan, S., et al. Concomitant Suppression of Three Target Genes Can Explain the Impact of a MicroRNA on Metastasis, Genes & Development, 23(22):2592-7, 2009. Epub Oct 29, 2009 (Valastyan 2009b).

As described above, we demonstrated that miR-31 regulates six mRNAs that encode proteins with roles in cell motility and tumor progression: frizzled3 (Fzd3), integrin-α5 (ITGA5), matrix metallopeptidase 16 (MMP16), myosin phosphatase-Rho interacting protein (M-RIP), radixin (RDX), and RhoA (Valastyan et al. 2009). To begin to address whether miR-31-imposed inhibition of one or more of these effectors might be responsible for mediating miR-31's anti-metastatic influences, we stably suppressed these six mRNAs individually in otherwise-metastatic MDA-MB-231 human breast cancer cells ("231 cells") using short hairpin RNAs (shRNAs). 231 cells are largely devoid of endogenous miR-31 and robustly express these six effectors; moreover, ectopic miR-31 impairs metastasis by these cells.

For each gene, we derived multiple cell lines that stably expressed a distinct shRNA targeting unique sequences in the encoded mRNA in order to minimize confounding influences from shRNA off-target effects (Supplemental Fig. 1A and 2A). At least one shRNA against each of the six effectors reduced its target's level by a factor comparable to that elicited by miR-31 expression (Valastyan et al. 2009). This allowed us to reasonably approximate the consequences of miR-31's actions on each individual downstream effector.

These shRNA-expressing 231 cells were subjected to *in vitro* assays that model traits important for metastasis. We observed that individual suppression of ITGA5, RDX, or RhoA reduced invasion, motility, and resistance to anoikis-mediated cell death *in vitro*; in contrast, the Fzd3, MMP16, or M-RIP shRNAs failed to substantially affect these behaviors (Supplemental Fig. 1B-D and 2B-D). For shRNAs that conferred measurable responses, the magnitude of these responses was directly correlated with the extent of knock-down achieved, suggesting that these effects arose as a specific consequence of reduced levels of the targeted protein. Inhibition of Fzd3, ITGA5, MMP16, M-RIP, RDX, or RhoA failed to affect *in vitro* proliferation (Supplemental Fig. 1E and 2E). Also, the responses evoked by the ITGA5, RDX, and RhoA shRNAs could not be ascribed to saturation of the miRNA biogenesis machinery, as mature levels of eight control miRNAs were unaffected in these cells (Supplemental Fig. 3).

We determined whether suppression of these six mRNAs altered metastatic capacity *in vivo* by intravenously injecting the shRNA-expressing 231 cells into mice. One month later, cells bearing shRNAs targeting ITGA5, RDX, or RhoA had generated 80%, 85%, and 55% fewer lung metastases than controls, respectively; however, downregulation of Fzd3, MMP16, or M-RIP did not affect the number of metastases spawned (Supplemental Fig. 4). Thus, inhibition of ITGA5, RDX, or RhoA - but not Fzd3, MMP16, or M-RIP - affects *in vitro* surrogates of metastatic capacity as well as *in vivo* metastasis.

To extend these analyses, we stably re-expressed miRNA-insensitive versions of the mRNAs encoding Fzd3, ITGA5, MMP16, M-RIP, RDX, or RhoA individually in 231 cells that already expressed either miR-31 or control vector (Supplemental Fig. 5A). This allowed us to gauge the ability of each of these effectors - when re-expressed - to reverse miR-31's impact on *in vivo* metastasis. When introduced into the venous circulation of mice, miR-31-expressing cells formed 85% fewer lung metastases than controls one month post-injection (Supplemental Fig. 5B), consistent with our prior findings (Valastyan et al. 2009). Individual re-expression of ITGA5, RDX, or RhoA restored the number of lung metastases in miR-31-expressing cells to 55%, 50%, and 65% of control levels, respectively; in contrast, Fzd3, MMP16, or M-RIP failed to increase lesion number (Supplemental Fig. 5B). Overexpression of ITGA5, RDX, or RhoA did not further enhance metastasis in control 231 cells (Supplemental Fig. 5B), suggesting that signaling from these pathways was already saturated in 231 cells, as has previously been established for RhoA-controlled networks (Pillé et al. 2005). Together, these findings implied that although miR-31 is capable of suppressing numerous mRNA species, its ability to regulate only a subset of these effectors appears to be crucial for its capacity to impair metastasis.

In support of this notion, when stably re-expressed in 231 cells, Fzd3, MMP16, or M-RIP failed to reverse miR-31-imposed attenuation of invasion, motility, and anoikis resistance *in vitro* (Supplemental Fig. 6); in contrast, our prior work revealed that restored levels of ITGA5, RDX, or RhoA rescued, at least partially, miR-31-evoked defects in these phenotypes (Valastyan et al. 2009). Based on these *in vitro* and *in vivo* re-expression data, as well as the above-described *in vitro* and *in vivo* loss-of-function findings, we focused our subsequent analyses on the ability of inhibition of ITGA5, RDX, and RhoA to account for miR-31's anti-metastatic activities.

To this end, we investigated the consequences of suppressing ITGA5, RDX, or RhoA individually in an orthotopic injection assay. Accordingly, we implanted 231 cells expressing shRNAs targeting either ITGA5, RDX, or RhoA into the mammary fat pads of mice. Suppression of ITGA5 or RhoA did not affect primary tumor growth; conversely, inhibition of RDX reduced the size of resulting mammary tumors (Fig. 1A). After normalizing for differences in primary tumor growth, cells expressing shRNAs against ITGA5, RDX, or RhoA formed 85%, 70%, and 50% fewer lung metastases than controls 2.5 months after injection, respectively (Fig. 1B). Thus, inhibition of ITGA5, RDX, or RhoA each impedes metastasis; however, this assay did not reveal the particular step(s) of the invasion-metastasis cascade that were impaired due to suppression of ITGA5, RDX, or RhoA.

In our previous work, we observed that miR-31 impinges upon three steps of the invasion-metastasis cascade *in vivo*: local invasion, early post-intravasation events (intraluminal viability, extravasation, and/or initial survival in distant tissues), and colonization (Valastyan et al. 2009). Consequently, we evaluated whether the individual suppression of ITGA5, RDX, or RhoA was sufficient to recapitulate one or more of miR-31's multiple effects on the metastatic process. We found that 231 cells containing shRNAs against either ITGA5, RDX, or RhoA formed primary tumors that appeared histologically invasive and were indistinguishable from controls (Fig. 1C). Thus, inhibition of ITGA5, RDX, or RhoA alone does not abolish local invasion *in vivo.*

Putative effects on early post-intravasation events were examined by quantifying shRNA-expressing 231 cells in the lungs one day after intravenous injection. Cells with either suppressed ITGA5 or RhoA were 40% and 30% less prevalent than controls, respectively; however, RDX knock-down did not reduce persistence in the lungs (Fig. 1D). These effects were not attributable to a differential ability of the cells to become lodged initially in the lung microvasculature, as equal numbers of cells were detected in the lungs 10 minutes after intravenous injection (Supplemental Fig. 7). These data indicated that inhibition of either ITGA5 or RhoA impairs early post-intravasation events *in vivo.*

To investigate potential effects on colonization (i.e., the capacity of disseminated single cells to yield large, multi-cellular metastases), the sizes of lung metastases in intravenously injected animals was analyzed three months after implantation. 231 cells expressing either ITGA5 or RDX shRNAs formed only small micrometastases, while RhoA shRNA-containing cells generated macroscopic metastases comparable to those spawned by control cells (Fig. 1E). Hence, suppression of either ITGA5 or RDX alone prevents colonization *in vivo.*

Together, these observations revealed that while individual suppression of ITGA5, RDX, or RhoA impairs one or more steps of the invasion-metastasis cascade, inhibition of any one of these proteins alone is unable to phenocopy the full spectrum of miR-31's impact on metastasis. This suggested that miR-31 may achieve its influences on multiple distinct stages of the metastatic process via concomitant suppression of several downstream effectors. Provocatively, our loss-of-function analyses indicated that ITGA5, RDX, and RhoA act during at least partially distinct steps of the invasion-metastasis cascade (e.g., RhoA affected early post-intravasation events but not colonization, while RDX had no impact on early post-intravasation events but altered colonization); hence, their concurrent regulation provides a plausible mechanism by which miR-31 might elicit its multiple anti-metastatic effects.

To test this hypothesis, we stably re-expressed miRNA-insensitive mRNAs encoding ITGA5, RDX, and RhoA together in combination - along with either miR-31 or control vector - in 231 cells. When these cells were orthotopically injected into mice, miR-31 enhanced primary tumor growth, recapitulating our prior findings (Valastyan et al. 2009); simultaneous re-expression of ITGA5, RDX, and RhoA failed to alter the size of miR-31-containing or control primary tumors (Fig. 2A). Despite their ability to generate larger primary tumors, miR-31-expressing 231 cells were impaired by >80% in their ability to spawn lung metastases (Fig. 2B). ITGA5, RDX, and RhoA did not enhance metastasis in control 231 cells; however, concomitant re-expression of ITGA5, RDX, and RhoA in 231 cells containing miR-31 completely abrogated miR-31-imposed metastasis suppression (Fig. 2B). These data implied that the impact of miR-31 on *in vivo* metastasis can be explained by miR-31's capacity to inhibit a cohort of three downstream effectors. This was quite surprising, as computational algorithms predict that miR-31 regulates >200 mRNAs, many of which encode proteins that function in metastasis-relevant processes (Krek et al. 2005; Grimson et al. 2007).

Since the combined re-expression of ITGA5, RDX, and RhoA entirely abolished miR-31-evoked metastasis suppression, we also determined whether these three effectors were able to reverse a subset of miR-31's influences on metastasis when re-expressed either individually or in different combinations. Thus, we created 231 cells stably expressing miR-31 or control vector plus all possible permutations of zero, one, two, or three of these miR-31 targets (all rendered miRNA-resistant) (Supplemental Fig. 8). miR-31, ITGA5, RDX, and RhoA failed to affect cell proliferation *in vitro* (Supplemental Fig. 9A). However, individual re-expression of ITGA5, RDX, or RhoA rescued, at least partially, *in vitro* defects in invasion, motility, and anoikis resistance conferred by ectopic miR-31; the extent of reversal was more pronounced when multiple effectors were re-expressed in combination (Supplemental Fig. 9B-D).Thus, ITGA5, RDX, and RhoA control *in vitro* behaviors important for metastasis downstream of miR-31.

To assay the respective abilities of all possible combinations of re-expressed ITGA5, RDX, and/or RhoA to reverse miR-31's influences on *in vivo* metastasis, 231 cells expressing miR-31, ITGA5, RDX, and/or RhoA were orthotopically implanted into mice. miR-31 generally promoted primary tumor growth, while restored levels of ITGA5, RDX, and RhoA failed to consistently affect the growth of primary tumors (Fig. 3A and Supplemental Table 1). miR-31 reduced the incidence of metastatic lesions in the lungs by >90% (Fig. 3B). When individually re-expressed in miR-31-containing cells, ITGA5, RDX, or RhoA increased metastasis to 40%, 45%, and 65% of control levels, respectively; re-expression of any two of these targets in miR-31-positive cells yielded 85% as many metastases as controls (Fig. 3B). As before, concomitant re-expression of ITGA5, RDX, and RhoA in cells containing miR-31 restored the number of lung metastases to 100% of that observed in controls (Fig. 3B). Hence, these three effectors make distinct contributions to *in vivo* metastasis that can collaborate to explain miR-31's influence on this process; however, these observations failed to delineate the specific step(s) of the invasion-metastasis cascade affected by various combinations of re-expressed ITGA5, RDX, and/or RhoA.

miR-31 affects three steps of the invasion-metastasis cascade *in vivo*: local invasion, early post-intravasation events, and colonization (Valastyan et al. 2009). To investigate whether ITGA5, RDX, and RhoA - when overexpressed - could synergize to reverse miR-31's effects on local invasion, we examined the histological appearance of primary tumors that developed in orthotopically injected mice. Whereas control 231 cell tumors displayed clear evidence of invasion, miR-31-expressing tumors were well-confined (Fig. 3C), as we previously documented (Valastyan et al. 2009). While ITGA5, RDX, and RhoA did not alter invasion in control 231 cell tumors, combined re-expression of these three targets abolished the previously well-encapsulated phenotype of miR-31-expressing tumors (Fig. 3C). miR-31-containing cells with restored levels of either RDX or RhoA alone formed primary tumors that appeared invasive, though reversal of miR-31-imposed invasion defects was incomplete; ITGA5 did not affect encapsulation (Fig. 3C). These observations revealed that miR-31-dependent attenuation of local invasion can be attributed to miR-31's ability to regulate RDX and RhoA. Ostensibly, in light of our shRNA studies (Fig. 1C), RDX and RhoA function redundantly - either with one another or with additional, still-unidentified miR-31 targets - to promote invasion *in vivo.*

We also examined whether re-expression of these three targets could reverse the impact of miR-31 on early post-intravasation events. To do so, we introduced 231 cells into the venous circulation of mice and assayed the number of cells in the lungs one day after injection. Consistent with our previous findings (Valastyan et al. 2009), miR-31-expressing cells were five-fold impaired in their ability to persist in the lungs (Fig. 4A), indicating that miR-31 impeded one or more early post-intravasation events. ITGA5, RDX, and RhoA failed to affect early post-intravasation events in control 231 cells (Fig. 4A). In contrast, individual re-expression of either ITGA5 or RhoA restored the number of miR-31-expressing cells in the lungs to 50% of control levels; RDX did not augment the ability of cells containing miR-31 to persist in the lungs at this timepoint (Fig. 4A). Simultaneous reintroduction of ITGA5 and RhoA in miR-31-expressing cells sufficed to completely override miR-31-imposed obstruction of early post-intravasation events (Fig. 4A). These effects were not a consequence of an altered ability of ITGA5-, RDX-, RhoA-, and/or miR-31-expressing cells to become lodged initially in the lung microvasculature, as equal numbers of cells were detected in the lungs 10 minutes after intravenous injection (Supplemental Fig. 10). These data provided evidence that miR-31-evoked suppression of early post-intravasation events can be ascribed to miR-31's ability to modulate ITGA5 and RhoA.

Three months after intravenous injection, control 231 cells generated large macroscopic metastases while miR-31-expressing cells yielded only small micrometastases (Fig. 4B). Hence, miR-31 prevented disseminated tumor cells from re-initiating their proliferative program at the site of metastasis, in consonance with miR-31's reported influence on colonization (Valastyan et al. 2009). Concomitant re-expression of ITGA5, RDX, and RhoA in miR-31-containing cells abrogated miR-31-imposed suppression of colonization, yet overexpression of these three targets in control 231 cells failed to increase lesion size (Fig. 4B). Individually restored levels of either ITGA5 or RDX in miR-31-expressing cells reversed miR-31's effects on colonization; RhoA did not affect this parameter (Fig. 4B). Thus, the ability of miR-31 to inhibit colonization can derive from its capacity to suppress ITGA5 and RDX.

In this same assay, miR-31-expressing 231 cells formed 20-fold fewer lung metastases than controls (Fig. 4C). When individually re-expressed in miR-31-containing cells, ITGA5, RDX, or RhoA increased the number of metastases formed to 60%, 60%, and 50% of control levels, respectively (Fig. 4C). Restored levels of pairwise combinations of these three targets in miR-31-expressing cells enhanced lesion number to >70% of controls; importantly, simultaneous re-expression of ITGA5, RDX, and RhoA in miR-31-containing cells completely abolished miR-31-mediated metastasis suppression (Fig. 4C). Taken together, the preceding experiments indicated that the impact of miR-31 on metastasis can be entirely explained by miR-31's capacity to regulate ITGA5, RDX, and RhoA; these three targets act at partially overlapping steps of the invasion-metastasis cascade downstream of miR-31 *in vivo* (Table 1).

It remained possible that the ability of ITGA5, RDX, and RhoA to override miR-31's actions arose due to some peculiarity of the 231 cell system. To address this, we extended our analyses to SUM-159 human breast cancer cells. Like 231 cells, SUM-159 cells lack endogenous miR-31, are highly aggressive *in vitro,* and display impaired invasion, motility, and anoikis resistance upon ectopic miR-31 (Valastyan et al. 2009). We created SUM-159 cells stably expressing all 16 potential combinations of either miR-31 or control vector plus miRNA-resistant mRNAs encoding ITGA5, RDX, and/or RhoA; all lines displayed comparable *in vitro* proliferative kinetics (Supplemental Fig. 11A-B). Consistent with our observations in 231 cells, individual re-expression of ITGA5, RDX, or RhoA in miR-31-containing SUM-159 cells rescued, at least partially, *in vitro* defects in invasion, motility, and anoikis resistance attributable to ectopic miR-31; as before, the extent of rescue was more pronounced when multiple effectors were concomitantly re-expressed (Supplemental Fig. 11C-E). Hence, the ability of ITGA5, RDX, and RhoA re-expression to override the actions of miR-31 is not confined to 231 cells.

Whereas individual re-expression of ITGA5, RDX, or RhoA largely reversed certain miR-31-imposed metastasis-relevant defects *in vitro* (Supplemental Fig. 9 and 11), individual restoration of ITGA5, RDX, or RhoA levels only partially rescued miR-31's effects on metastasis *in vivo* (Fig. 3-4). This underscores the fact that available *in vitro* assays inadequately model the fully complexity of *in vivo* metastasis; caution must therefore be exercised when deploying these techniques, particularly in the absence of parallel *in vivo* analyses.

Collectively, the findings described in this Example suggest that miR-31's effects on metastasis can be explained by its ability to suppress a relatively modest number of downstream targets. In the present case, the relevant effectors comprise only a small percentage of the total roster of mRNAs targeted by this miRNA. While our data indicate that ITGA5, RDX, and RhoA represent a minimal cohort of effectors whose regulation is sufficient to account for miR-31's impact on metastasis, these observations do not preclude the existence of additional miR-31 targets that impinge upon metastasis-relevant pathways in a manner that ostensibly is functionally redundant with the actions of ITGA5, RDX, and/or RhoA. Also, it is possible that one or more *bona fide* targets of miR-31 that have metastatic relevance fail to be significantly downregulated by this miRNA in 231 cells. Overall, due to the fact that metastases are responsible for the overwhelming majority of patient mortality from carcinomas, this work further highlights that modulation of miR-31 and its effectors may prove clinically useful.

**Plasmid Construction**. miR-31 was expressed from pBABE-puro, as we have elaborated upon (Valastyan et al. 2009). Constructs encoding Fzd3 (Deardorff et al. 2001), ITGA5 (Kuwada and Li 2000), MMP16 (Hotary et al. 2006), M-RIP (Surks et al. 2003), RDX (Batchelor et al. 2004), and RhoA (Subauste et al. 2000) - but lacking endogenous 3' UTR sequences and thus rendering them miRNA-resistant - were subcloned into pBABE-hygro, pBABE-neo, pBABE-hygro, pBABE-hygro, pBABE-hygro, and pBABE-zeo, respectively (Elenbaas et al. 2001). shRNAs targeting Fzd3, ITGA5, MMP16, M-RIP, RDX, and RhoA were expressed from pLKO.1-puro (Open Biosystems).

**Immunoblotting.** Lysates were resolved by NuPAGE gel electrophoresis (Invitrogen), transferred to a PVDF membrane, and probed with antibodies against β-actin (Santa Cruz), ITGA5 (Santa Cruz), RDX (Cell Signaling), or RhoA (Abcam).

**MicroRNA Detection and RT-PCR.** Total RNA, including small RNAs, was extracted from the indicated 231 cells with a *mir*Vana MicroRNA Isolation Kit (Ambion). RT-PCR-based detection of mature microRNAs and the 5S rRNA was achieved via use of a *mir*Vana MicroRNA Detection Kit and gene-specific primer sets (Ambion). For detection of Fzd3, GAPDH, ITGA5, MMP16, M-RIP, RDX, and RhoA transcript levels, cDNA was prepared from 200 ng of total RNA using the SuperScript III First-Strand Synthesis System (Invitrogen), and quantitated by SYBR Green real time RT-PCR (Applied Biosystems).

**Invasion and Motility Assays.** For invasion assays, 1.0 x 10⁵ cells were plated in Matrigel-coated chambers with 8.0 µm pores (BD Biosciences); for motility assays, 5.0 x 10⁴ cells were seeded atop uncoated membranes with 8.0 µm pores (BD Biosciences). Cells were seeded in serum-free media, and translocated toward complete growth media for 20 hours. Non-invaded or non-migrated cells were then physically removed; successfully translocated cells were visualized using a Diff-Quick Staining Set (Dade) and counted.

**Anoikis Assays.** Anoikis resistance was evaluated by seeding 7.5 x 10⁴ cells in ultra-low attachment plates (Corning). After 24 hours, cells were resuspended in 0.4% trypan blue (Sigma) and the proportion of viable cells was quantified using a hemocytometer.

**Measurements of *in vitro* Cell Proliferation.** Unless otherwise denoted, proliferative kinetics were assayed by seeding 1.0 x 10⁵ cells per well in 6-well plates. Total cell number was assessed every two to three days by trypsinization and manual counting with a hemocytometer. Alternatively, cell proliferation was measured by seeding 5.0 x 10² or 1.0 x 10³ cells per well in 96-well plates and utilizing a CellTiter96 AQᵤₑₒᵤₛ One Solution MTS Cell Proliferation Assay (Promega); cells were incubated with the MTS reagent for 1.5 hours, then total cell number was quantitated by measuring absorbance at 492 nm on a 96-well plate reader.

### REFERENCE LIST 2

Ambros V. 2004. The functions of animal microRNAs. Nature 431: 350-355.
Baek D, Villén J, Shin C, Camargo FD, Gygi SP, Bartel DP. 2008. The impact of microRNAs on protein output. Nature 455: 64-71.
Bartel DP. 2009. MicroRNAs: target recognition and regulatory functions. Cell 136: 215-233.
Elenbaas B, Spirio L, Koerner F, Fleming MD, Zimonjic DB, Donaher JL, Popescu NC, Hahn WC, Weinberg RA. 2001. Human breast cancer cells generated by oncogenic transformation of primary mammary epithelial cells. Genes Dev. 15: 50-65.
Fidler IJ. 2003. The pathogenesis of cancer metastasis: the 'seed and soil' hypothesis revisited. Nat Rev Cancer 3: 453-458.
Grimson A, Farh KK, Johnston WK, Garrett-Engele P, Lim LP, Bartel DP. 2007. MicroRNA targeting specificity in mammals: determinants beyond seed pairing. Mol Cell 27: 91-105.
Gupta GP, Massagué J. 2006. Cancer metastasis: building a framework. Cell 127: 679-695.
Johnnidis JB, Harris MH, Wheeler RT, Stehling-Sun S, Lam MH, Kirak O, Brummelkamp TR, Fleming MD, Camargo FD. 2008. Regulation of progenitor cell proliferation and granulocyte function by microRNA-223. Nature 451: 1125-1129.
Krek A, Grün D, Poy MN, Wolf R, Rosenberg L, Epstein EJ, MacMenamin P, da Piedade I, Gunsalus KC, Stoffel M, et al. 2005. Combinatorial microRNA target predictions. Nat Genet. 37: 495-500.
Kumar MS, Erkeland SJ, Pester RE, Chen CY, Ebert MS, Sharp PA, Jacks T. 2008. Suppression of non-small cell lung tumor development by the let-7 microRNA family. PNAS 105: 3903-3908.
Ma L, Teruya-Feldstein J, Weinberg RA. 2007. Tumour invasion and metastasis initiated by microRNA-10b in breast cancer. Nature 449: 682-688.
Pillé JY, Denoyelle C, Varet J, Bertrand JR, Soria J, Opolon P, Lu H, Pritchard LL, Vannier JP, Malvy C, et al. 2005. Anti-RhoA and anti-RhoC siRNAs inhibit the proliferation and invasiveness of MDA-MB-231 breast cancer cells in vitro and in vivo. Mol Ther. 11: 267-274.
Rodriguez A, Vigorito E, Clare S, Warren MV, Couttet P, Soond DR, van Dongen S, Grocock RJ, Das PP, Miska EA, et al. 2007. Requirement of bic/microRNA-155 for normal immune function. Science 316: 608-611.
Selbach M, Schwanhäusser B, Thierfelder N, Fang Z, Khanin R, Rajewsky N. 2008. Widespread changes in protein synthesis induced by microRNAs. Nature 455: 58-63.
Thai TH, Calado DP, Casola S, Ansel KM, Xiao C, Xue Y, Murphy A, Frendewey D, Valenzuela D, Kutok JL, et al. 2007. Regulation of germinal center response by microRNA-155. Science 316: 604-608.
Valastyan S, Reinhardt F, Benaich N, Calogrias D, Szász AM, Wang ZC, Brock JE, Richardson AL, Weinberg RA. 2009. A pleiotropically acting microRNA, miR-31, inhibits breast cancer metastasis. Cell 137: 1032-1046.
van Rooij E, Sutherland LB, Qi X, Richardson JA, Hill J, Olson EN. 2007. Control of stress-dependent cardiac growth and gene expression by a microRNA. Science 316: 575-579.
Ventura A, Jacks T. 2009. MicroRNAs and cancer: short RNAs go a long way. Cell 136: 586-591.
Ventura A, Young AG, Winslow MM, Lintault L, Meissner A, Erkeland SJ, Newman J, Bronson, RT, Crowley D, Stone JR, et al. 2008. Targeted deletion reveals essential and overlapping functions of the miR-17 through 92 family of miRNA clusters. Cell 132: 875-886.
Xiao C, Calado DP, Galler G, Thai TH, Patterson HC, Wang J, Rajewsky N, Bender TP, Rajewsky K. 2007. MiR-150 controls B cell differentiation by targeting the transcription factor c-Myb. Cell 131: 146-159.
Yu F, Yao H, Zhu P, Zhang X, Pan Q, Gong C, Huang Y, Hu X, Su F, Lieberman J, et al. 2007. Let-7 regulates self renewal and tumorigenicity of breast cancer cells. Cell 131: 1109-1123.
Zhao Y, Ransom JF, Li A, Vedantham V, von Drehle M, Muth AN, Tsuchihashi T, McManus MT, Schwartz RJ, Srivastava D. 2007. Dysregulation of cardiogenesis, cardiac conduction, and cell cycle in mice lacking miRNA-1-2. Cell 129: 303-317.
Batchelor CL, Woodward AM, Crouch DH. 2004. Nuclear ERM (ezrin, radixin, moesin) proteins: regulation by cell density and nuclear import. Exp Cell Res. 296: 208-222.
Deardorff MA, Tan C, Saint-Jeannet JP, Klein PS. 2001. A role for frizzled 3 in neural crest development. Development 128: 3655-3663.
Elenbaas B, Spirio L, Koerner F, Fleming MD, Zimonjic DB, Donaher JL, Popescu NC, Hahn WC, Weinberg RA. 2001. Human breast cancer cells generated by oncogenic transformation of primary mammary epithelial cells. Genes Dev. 15: 50-65.
Hotary K, Li XY, Allen E, Stevens SL, Weiss SJ. 2006. A cancer cell metalloprotease triad regulates the basement membrane transmigration program. Genes Dev. 20: 2673-2686.
Kuwada SK, Li X. 2000. Integrin alpha5/beta1 mediates fibronectin-dependent epithelial cell proliferation through epidermal growth factor receptor activation. Mol Biol Cell 11: 2485-2496.
Subauste MC, Von Herrath M, Benard V, Chamberlain CE, Chuang TH, Chu K, Bokoch GM, Hahn KM. 2000. Rho family proteins modulate rapid apoptosis induced by cytotoxic T lymphocytes and Fas. J Biol Chem. 275: 9725-9733.
Surks HK, Richards CT, Mendelsohn ME. 2003. Myosin phosphatase-Rho interacting protein. A new member of the myosin phosphatase complex that directly binds RhoA. J Biol Chem. 27: 51484-51493.
Valastyan S, Reinhardt F, Benaich N, Calogrias D, Szász AM, Wang ZC, Brock JE, Richardson AL, Weinberg RA. 2009. A pleiotropically acting microRNA, miR-31, inhibits breast cancer metastasis. Cell 137: 1032-1046.

Those skilled in the art will recognize, or be able to ascertain using no more than routine experimentation, many equivalents to the specific embodiments of the invention described herein.

Articles such as "a" and "an", and the like, may mean one or more than one unless indicated to the contrary or otherwise evident from the context.

The phrase "and/or" as used herein in the specification and in the claims, should be understood to mean "either or both" of the elements so conjoined. Multiple elements listed with "and/or" should be construed in the same fashion, i.e., "one or more" of the elements so conjoined. Other elements may optionally be present other than the elements specifically identified by the "and/or" clause. As used herein in the specification and in the claims, "or" should be understood to have the same meaning as "and/or" as defined above. For example, when used in a list of elements, "or" or "and/or" shall be interpreted as being inclusive, i.e., the inclusion of at least one, but optionally more than one, of list of elements, and, optionally, additional unlisted elements. Only terms clearly indicative to the contrary, such as "only one of "or "exactly one of" will refer to the inclusion of exactly one element of a number or list of elements. Thus claims that include "or" between one or more members of a group are considered satisfied if one, more than one, or all of the group members are present, employed in, or otherwise relevant to a given product or process unless indicated to the contrary. The invention provides embodiments in which exactly one member of the group is present, employed in, or otherwise relevant to a given product or process. The invention also provides embodiments in which more than one, or all of the group members are present, employed in, or otherwise relevant to a given product or process. It is to be understood that the invention encompasses embodiments in which one or more limitations, elements, clauses, descriptive terms, etc., of a claim is introduced into another claim. For example, a claim that is dependent on another claim can be modified to include one or more elements or limitations found in any other claim that is dependent on the same base claim.

Where the claims recite a composition, it is understood that methods of using the composition as disclosed herein are provided, and methods of making the composition according to any of the methods of making disclosed herein are provided. Where the claims recite a method, it is understood that a composition for performing the method is provided. Where elements are presented as lists or groups, each subgroup is also disclosed. It should also be understood that, in general, where the invention, or aspects of the invention, is/are referred to as comprising particular elements, features, etc., certain embodiments of the invention or aspects of the invention consist of, or consist essentially of, such elements, features, etc.

It should also be understood that, unless clearly indicated to the contrary, in any methods claimed herein that include more than one step or act, the order of the steps or acts of the method is not necessarily limited to the order in which the steps or acts of the method are recited.

Where ranges are given herein, the invention provides embodiments in which the endpoints are included, embodiments in which both endpoints are excluded, and embodiments in which one endpoint is included and the other is excluded. It should be assumed that both endpoints are included unless indicated otherwise. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise. "About" in reference to a numerical value generally refers to a range of values that fall within ±10%, in some embodiments ±5%, in some embodiments ±1%, in some embodiments ±0.5% of the value unless otherwise stated or otherwise evident from the context. In any embodiment of the invention in which a numerical value is prefaced by "about", the invention provides an embodiment in which the exact value is recited. In any embodiment of the invention in which a numerical value is not prefaced by "about", the invention provides an embodiment in which the value is prefaced by "about". Where the phrase "at least" precedes a series of numbers, it is to be understood that the phrase applies to each number in the list (it being understood that, depending on the context, such as in describing percent identity, 100% of a value may be an upper limit). It is also understood that any particular embodiment, feature, or aspect of the present invention may be explicitly excluded from any one or more of the claims.

## Claims

1. An inhibitor of expression or activity of at least one target of miR-31 selected from miR-31, a nucleic acid construct encoding a precursor to miR-31, mature miR-31, or a precursor to miR-31 for use in treating tumor metastasis in a subject or metastasis of a tumor cell in a subject.

2. The inhibitor for use of claim 1, wherein the use comprises expressing miR31 in the tumor cell.

3. A composition for treating tumor metastasis comprising miR-31, a nucleic acid construct encoding a precursor to miR-31, mature miR-31, or a precursor to miR-31 .

4. A method for assessing the likelihood that a tumor in a subject will metastasize, the method comprising:
(a) determining the level of a miR-31 gene product, preferably mature miR-31 RNA or a miR-31 precursor RNA, in a biological sample obtained from the tumor, preferably comprising tumor tissue; and
(b) comparing the level with a control level, wherein if the level determined in (a) is less than the control level, the tumor is considered to have increased likelihood of metastasizing, preferably wherein determining the level of the miR-31 gene product in the biological sample comprises performing a hybridization based assay, polymerase chain reaction assay, or sequencing.

5. A method of assessing the likelihood of development of a tumor metastasis in a subject, comprising the steps of:
(a) determining the level of a miR-31 gene product, preferably a mature miR-31 RNA or a miR-31 precursor RNA, in a biological sample obtained from a subject, preferably comprising tumor tissue; and
(b) comparing the level determined in (a) with an appropriate control, wherein if the level determined in (a) is less than the control level of the miR-31 gene product, then the subject has an increased likelihood of developing a metastasis, preferably wherein determining the level of the miR-31 gene product in the biological sample comprises performing a hybridization based assay, polymerase chain reaction assay, or sequencing.

6. A method for providing prognostic information relating to the likelihood that a tumor in a subject will metastasize, the method comprising:
(a) determining the level of a miR-31 gene product, preferably miR-31 RNA or a miR-31 precursor RNA, in a biological sample obtained from the tumor, preferably comprising tumor tissue; and
(b) comparing the level with a control level, wherein (i) if the level determined in (a) is less than the control level, the tumor is considered to have a poor prognosis; or (ii) if the level determined in (a) is greater than the control level, the tumor is considered to have a good prognosis, preferably wherein determining the level of the miR-31 gene product in the biological sample comprises performing a hybridization based assay, polymerase chain reaction assay, or sequencing.

7. The method of any one of claims 4-6, wherein the tumor is a breast cancer.

8. A method for testing the ability of a compound to inhibit the survival and/or proliferation of a cell, preferably a cancer cell, in particular a breast cancer cell, comprising (a) contacting one or more test cells with a compound, wherein the one or more test cells has reduced or absent miR-31 expression or activity as compared to a control cell, preferably wherein the test cells have a mutation or deletion in the miR-31 gene or express a miRNA sponge that suppresses miR-31 activity, and (b) detecting the level of inhibition of the survival and/or proliferation of the one or more test cells by the compound, preferably wherein the test cells and the control cells are genetically matched.

9. The method of claim 8, comprising: (a) contacting one or more test cells and one or more control cells with the compound, wherein the one or more test cells has reduced or absent miR-31 expression or activity as compared with the one or more control cells; and (b) detecting the level of inhibition of the survival and/or proliferation of the one or more test cells and control cells by the compound; and (c) identifying the compound as a candidate anti-tumor agent if the compound has a greater inhibitory effect on the survival and/or proliferation of the test cells than the control cells.

10. The method of claim 8, further comprising administering the compound to a subject, preferably wherein the subject has cancer, wherein the subject is a non-human animal and the method optionally further comprises introducing one or more cancer cells into the subject or inducing cancer in the subject.

11. The method of claim 10, further comprising assessing the ability of the compound to inhibit tumor development or metastasis in the subject.

## Patentansprüche

1. Inhibitor der Expression oder Aktivität von mindestens einem Ziel von miR-31, ausgewählt aus miR-31, einem Nukleinsäurekonstrukt kodierend einen Vorläufer von miR-31, reifem miR-31 oder einem Vorläufer von miR-31 zur Verwendung bei der Behandlung von Tumormetastase in einem Patienten oder Metastase einer Tumorzelle in einem Patienten.

2. Inhibitor zur Verwendung nach Anspruch 1, wobei die Verwendung Exprimieren von miR31 in der Tumorzelle umfasst.

3. Zusammensetzung zur Behandlung von Tumormetastase, umfassend miR-31, ein Nukleinsäurekonstrukt kodierend einen Vorläufer von miR-31, reifes miR-31 oder einen Vorläufer von miR-31.

4. Verfahren zur Bewertung der Wahrscheinlichkeit, dass ein Tumor in einem Patienten metastasieren wird, das Verfahren umfassend:
(a) Bestimmen der Höhe eines miR-31-Genprodukts, bevorzugt reife miR-31-RNA oder eine miR-31-Vorläufer-RNA, in einer biologischen Probe, erhalten von dem Tumor, bevorzugt umfassend Tumorgewebe; und
(b) Vergleichen der Höhe mit einer Kontrollhöhe, wobei, falls die in (a) bestimmte Höhe geringer als die Kontrollhöhe ist, der Tumor erachtet wird, eine erhöhte Wahrscheinlichkeit der Metastasierung zu haben, bevorzugt wobei Bestimmen der Höhe des miR-31-Genprodukts in der biologischen Probe Durchführen eines Hybridisierung-basierten Assays, Polymerase-Kettenreaktion-Assay oder Sequenzieren umfasst.

5. Verfahren zur Bewertung der Wahrscheinlichkeit der Entwicklung einer Tumormetastase in einem Patienten, umfassend die Schritte von:
(a) Bestimmen der Höhe eines miR-31-Genprodukts, bevorzugt einer reifen miR-31-RNA oder einer miR-31-Vorläufer-RNA in einer biologischen Probe, erhalten von einem Patienten, bevorzugt umfassend Tumorgewebe; und
(b) Vergleichen der in (a) bestimmten Höhe mit einer geeigneten Kontrolle, wobei, falls die in (a) bestimmte Höhe geringer ist als die Kontrollhöhe des miR-31-Genprodukts, der Patient dann eine erhöhte Wahrscheinlichkeit hat, eine Metastase zu entwickeln, bevorzugt wobei Bestimmen der Höhe des miR-31-Genprodukts in der biologischen Probe Durchführen eines Hybridisierung-basierten Assays, Polymerase-Kettenreaktion-Assay oder Sequenzieren umfasst.

6. Verfahren zur Bereitstellung von prognostischer Information bezogen auf die Wahrscheinlichkeit, dass ein Tumor in einem Patienten metastasieren wird, das Verfahren umfassend:
(a) Bestimmen der Höhe eines miR-31-Genprodukts, bevorzugt miR-31-RNA oder einer miR-31-Vorläufer-RNA, in einer biologischen Probe, erhalten von dem Tumor, bevorzugt umfassend Tumorgewebe; und
(b) Vergleichen der Höhe mit einer Kontrollhöhe, wobei, (i) falls die in (a) bestimmte Höhe geringer als die Kontrollhöhe ist, der Tumor erachtet wird, eine schlechte Prognose zu haben; oder (ii) falls die in (a) bestimmte Höhe größer als die Kontrollhöhe ist, der Tumor erachtet wird, eine gute Prognose zu haben, bevorzugt wobei Bestimmen der Höhe des miR-31-Genprodukts in der biologischen Probe Durchführen eines Hybridisierung-basierten Assays, Polymerase-Kettenreaktion-Assay oder Sequenzieren umfasst.

7. Verfahren nach einem der Ansprüche 4-6, wobei der Tumor ein Brustkrebs ist.

8. Verfahren zum Testen der Fähigkeit einer Verbindung, das Überleben und/oder Proliferation einer Zelle, bevorzugt einer Krebszelle, insbesondere einer Brustkrebszelle, zu verhindern, umfassend (a) Kontaktieren einer oder mehrerer Testzelle(n) mit einer Verbindung, wobei, die eine oder mehreren Testzelle(n) reduzierte oder fehlende miR-31-Expression oder -Aktivität hat/haben verglichen mit einer Kontrollzelle, bevorzugt wobei die Testzellen eine Mutation oder Deletion in dem miR-31-Gen haben oder einen miRNA-Schwamm exprimieren, der miR-31-Aktivität unterdrückt, und (b) Detektieren der Höhe der Inhibition des Überlebens und/oder Proliferation der einen oder mehreren Testzelle(n) durch die Verbindung, bevorzugt wobei die Testzellen und die Kontrollzellen genetisch passend sind.

9. Verfahren nach Anspruch 8, umfassend: (a) Kontaktieren einer oder mehrerer Testzelle(n) und einer oder mehrerer Kontrollzelle(n) mit der Verbindung, wobei die eine oder mehreren Testzelle(n) reduzierte oder fehlende miR-31-Expression oder -Aktivität hat/haben verglichen mit der einen oder den mehreren Kontrollzelle(n); und (b) Detektieren der Höhe der Inhibition des Überlebens und/oder Proliferation der einen oder den mehreren Testzelle(n) und Kontrollzelle(n) durch die Verbindung; und (c) Identifizieren der Verbindung als einen Antitumormittel-Kandidaten, falls die Verbindung einen größeren inhibitorischen Effekt auf das Überleben und/oder Proliferation der Testzellen als die Kontrollzellen hat.

10. Verfahren nach Anspruch 8, ferner umfassend Verabreichen der Verbindung an einen Patienten, bevorzugt wobei der Patient Krebs hat, wobei der Patient ein nichtmenschliches Tier ist und das Verfahren optional ferner Einführen einer oder mehrerer Krebszelle(n) in den Patienten oder Induzieren von Krebs in dem Patienten umfasst.

11. Verfahren nach Anspruch 10, ferner umfassend Bewerten der Fähigkeit der Verbindung, Tumorentwicklung oder -metastase in dem Patienten zu inhibieren.

## Revendications

1. Inhibiteur de l'expression ou de l'activité d'au moins une cible de miR-31 sélectionné parmi miR-31, un produit de recombinaison d'acide nucléique codant pour un précurseur de miR-31, un miR-31 mature, ou un précurseur de miR-31, destiné à être utilisé pour traiter une métastase tumorale chez un sujet ou la métastase d'une cellule tumorale chez un sujet.

2. Inhibiteur destiné à être utilisé selon la revendication 1, dans lequel l'utilisation comprend l'expression de miR-31 dans la cellule tumorale.

3. Composition pour traiter la métastase tumorale comprenant miR-31, un produit de recombinaison d'acide nucléique codant pour un précurseur de miR-31, un miR-31 mature, ou un précurseur de miR-31.

4. Procédé pour évaluer la probabilité de métastase d'une tumeur chez un sujet, le procédé comprenant :
(a) la détermination du taux d'un produit génique de miR-31, de préférence de l'ARN de miR-31 mature ou d'un ARN précurseur de miR-31, dans un échantillon biologique obtenu à partir de la tumeur, comprenant de préférence un tissu tumoral ; et
(b) la comparaison du taux avec un taux de contrôle, dans laquelle si le taux déterminé en (a) est inférieur au taux de contrôle, la tumeur est considérée comme présentant une probabilité accrue de métastase, de préférence où la détermination du taux du produit génique de miR-31 dans l'échantillon biologique comprend la réalisation d'un essai basé sur une hybridation, d'un essai de réaction en chaîne par polymérase, ou d'un séquençage.

5. Procédé pour évaluer la probabilité de développement d'une métastase tumorale chez un sujet, comprenant les étapes consistant à :
(a) déterminer le taux d'un produit génique de miR-31, de préférence d'un ARN de miR-31 mature ou d'un ARN précurseur de miR-31, dans un échantillon biologique obtenu à partir d'un sujet, comprenant de préférence un tissu tumoral ; et
(b) comparer le taux déterminé en (a) avec un contrôle approprié, dans lequel si le taux déterminé en (a) est inférieur au taux de contrôle du produit génique de miR-31, alors le sujet présente une probabilité accrue de développer une métastase, de préférence où la détermination du taux du produit génique de miR-31 dans l'échantillon biologique comprend la réalisation d'un essai basé sur une hybridation, d'un essai de réaction en chaîne par polymérase, ou d'un séquençage.

6. Procédé pour obtenir des informations pronostiques concernant la probabilité de métastase d'une tumeur chez un sujet, le procédé comprenant :
(a) la détermination du taux d'un produit génique de miR-31, de préférence de l'ARN de miR-31 ou d'un ARN précurseur de miR-31, dans un échantillon biologique obtenu à partir de la tumeur, comprenant de préférence un tissu tumoral ; et
(b) la comparaison du taux avec un taux de contrôle, dans laquelle (i) si le taux déterminé en (a) est inférieur au taux de contrôle, la tumeur est considérée comme présentant un mauvais pronostic ; ou (ii) si le taux déterminé en (a) est supérieur au taux de contrôle, la tumeur est considérée comme présentant un bon pronostic, de préférence où la détermination du taux du produit génique de miR-31 dans l'échantillon biologique comprend la réalisation d'un essai basé sur une hybridation, d'un essai de réaction en chaîne par polymérase, ou d'un séquençage.

7. Procédé selon l'une quelconque des revendications 4-6, dans lequel la tumeur est un cancer du sein.

8. Procédé pour tester l'aptitude d'un composé à inhiber la survie et/ou la prolifération d'une cellule, de préférence une cellule cancéreuse, en particulier une cellule du cancer du sein, comprenant (a) la mise en contact d'une ou de plusieurs cellules de test avec un composé, où la une ou plusieurs cellules de test présentent une expression ou une activité de miR-31 réduite ou absente par rapport à une cellule de contrôle, de préférence où les cellules de test comportent une mutation ou une délétion dans le gène miR-31 ou expriment une éponge à ARNmi qui supprime l'activité de miR-31, et (b) la détection du taux d'inhibition de la survie et/ou de la prolifération de la une ou plusieurs cellules de test par le composé, de préférence où les cellules de test et les cellules de contrôle sont génétiquement compatibles.

9. Procédé selon la revendication 8, comprenant : (a) la mise en contact d'une ou de plusieurs cellules de test et d'une ou de plusieurs cellules de contrôle avec le composé, où la une ou plusieurs cellules de test présentent une expression ou une activité de miR-31 réduite ou absente par rapport à la une ou plusieurs cellules de contrôle ; et (b) la détection du taux d'inhibition de la survie et/ou de la prolifération de la une ou plusieurs cellules de test et cellules de contrôle par le composé ; et (c) l'identification du composé en tant qu'agent antitumoral candidat si le composé présente un plus grand effet inhibiteur sur la survie et/ou la prolifération des cellules de test que les cellules de contrôle.

10. Procédé selon la revendication 8, comprenant en outre l'administration du composé à un sujet, de préférence dans lequel le sujet est atteint d'un cancer, où le sujet est un animal non humain et le procédé comprend en outre de manière facultative l'introduction d'une ou de plusieurs cellules cancéreuses chez le sujet ou l'induction du cancer chez le sujet.

11. Procédé selon la revendication 10, comprenant en outre l'évaluation de l'aptitude du composé à inhiber le développement ou la métastase d'une tumeur chez le sujet.
